# EUROPEAN PATENT SPECIFICATION

(11) **EP 2 903 650 B1**
(45) Date of publication and mention of the grant of the patent: **04.04.2018**
(21) Application number: 13771153.7
(22) Date of filing: 02.10.2013
(51) Int. Cl.: A61K 47/50, A61K 39/095, A61P 31/04, A61P 37/04, A61K 39/00, A61K 39/385, A61K 47/64

(54) **NONLINEAR SACCHARIDE CONJUGATES**
NICHTLINEARE SACCHARIDKONJUGATE
CONJUGUÉS SACCHARIDIQUES NON LINÉAIRES

(30) Priority: 02.10.2012 US 201261709093 P
(43) Date of publication of application: 12.08.2015
(73) Proprietor: GlaxoSmithKline Biologicals SA, 1330 Rixensart (BE)
(72) Inventor: BERTI, Francesco, I-53100 Siena (IT); BROGIONI, Giulia, I-53100 Siena (IT); COSTANTINO, Paolo, I-53100 Siena (IT); DEL GIUDICE, Giuseppe, I-53100 Siena (IT); ROMANO, Maria, I-53100 Siena (IT)
(74) Representative: Thornley, Rachel Mary
(86) International application number: PCT/EP2013/070496
(87) International publication number: WO 2014/053521

(56) References cited:
- WO-A1-2004/041310
- WO-A1-2004/041310
- WO-A2-99/55730
- WO-A2-99/55730
- WO-A2-2006/067632
- DE-A1- 19 821 859
- US-A1- 2009 043 077
- DE VELASCO E A ET AL: "Synthetic peptides representing T-cell epitopes act as carriers in pneumococcal polysaccharide conjugate vaccines", INFECTION AND IMMUNITY, AMERICAN SOCIETY FOR MICROBIOLOGY, US, vol. 63, no. 3, March 1995 (1995-03), pages 961-968, XP002128493, ISSN: 0019-9567
- FALUGI F ET AL: "Rationally designed strings of promiscuous CD4(+) T cell epitopes provide help to Haemophilus influenzae type b oligosaccharide: a model for new conjugate vaccines", EUROPEAN JOURNAL OF IMMUNOLOGY, WILEY - V C H VERLAG GMBH & CO. KGAA, DE, vol. 31, no. 12, December 2001 (2001-12), pages 3816-3824, XP002401049, ISSN: 0014-2980, DOI: 10.1002/1521-4141(200112)31:12<3816::AID-I MMU3816>3.0.CO;2-K
- PARADISO P R ET AL: "NOVEL APPROACHES TO THE DEVELOPMENT OF GLYCOCONJUGATE VACCINES WITHSYNTHETIC PEPTIDES AS CARRIERS", VACCINE RESEARCH, MARY ANN LIEBERT, INC., PUBLISHERS, US, vol. 2, no. 4, January 1993 (1993-01), pages 239-248, XP000870111,
- LECLERC C ET AL: "Identification of a T-cell epitope adjacent to neutralization antigenic site 1 of poliovirus type 1.", JOURNAL OF VIROLOGY FEB 1991, vol. 65, no. 2, February 1991 (1991-02), pages 711-718, XP55287334, ISSN: 0022-538X
- DE VELASCO E A ET AL: "Synthetic peptides representing T-cell epitopes act as carriers in pneumococcal polysaccharide conjugate vaccines", INFECTION AND IMMUNITY, AMERICAN SOCIETY FOR MICROBIOLOGY, US, vol. 63, no. 3, March 1995 (1995-03), pages 961-968, XP002128493, ISSN: 0019-9567
- FALUGI F ET AL: "Rationally designed strings of promiscuous CD4(+) T cell epitopes provide help to Haemophilus influenzae type b oligosaccharide: a model for new conjugate vaccines", EUROPEAN JOURNAL OF IMMUNOLOGY, WILEY - V C H VERLAG GMBH & CO. KGAA, DE, vol. 31, no. 12, December 2001 (2001-12), pages 3816-3824, XP002401049, ISSN: 0014-2980, DOI: 10.1002/1521-4141(200112)31:12<3816::AID-I MMU3816>3.0.CO;2-K
- PARADISO P R ET AL: "NOVEL APPROACHES TO THE DEVELOPMENT OF GLYCOCONJUGATE VACCINES WITHSYNTHETIC PEPTIDES AS CARRIERS", VACCINE RESEARCH, MARY ANN LIEBERT, INC., PUBLISHERS, US, vol. 2, no. 4, January 1993 (1993-01), pages 239-248, XP000870111,
- LECLERC C ET AL: "Identification of a T-cell epitope adjacent to neutralization antigenic site 1 of poliovirus type 1.", JOURNAL OF VIROLOGY FEB 1991, vol. 65, no. 2, February 1991 (1991-02), pages 711-718, XP55287334, ISSN: 0022-538X

## Description

### RELATED APPLICATION

This application claims the benefit of U.S. Provisional Application No. 61/709,093, filed October 2, 2012.

### TECHNICAL FIELD

This invention relates to immunization using nonlinear saccharide conjugates that comprise polysaccharides or oligosaccharides that are linked to at least two carrier peptides that comprise T-cell epitopes and have no conformational B-cell epitopes where one of the carrier peptides is linked to an internal saccharide. Of particular interest is use of such compositions as vaccines against bacterial and fungal infections and diseases. This invention also relates to immunization using linear saccharide conjugates that comprise polysaccharides or oligosaccharides that are linked to at least one carrier peptide that comprises at least two PV1 T-cell epitopes and has no conformational B-cell epitopes.

### BACKGROUND ART

Carrier proteins are used to improve the immune response to polysaccharide immunogens. Such carrier proteins can be particularly advantageous in the induction of an immune response in the very young and are therefore found in a number of pediatric vaccines. The recommended pediatric immunization schedule includes a significant number of vaccines including hepatitis B vaccine at birth; starting at six weeks, all of diphtheria/tetanus/pertussis (DTaP), rotavirus, *H. influenzae* type b (Hib) conjugate, inactivated poliovirus and pneumococcal conjugates; starting at six months, inactivated influenza vaccines; starting at 12 months, measles/mumps/rubella (MMR), varicella, and hepatitis A; and after two years, meningococcal conjugate. Among this list, the following are polysaccharide conjugates: Hib conjugate (e.g., VaxemHib - a diphtheria CRM₁₉₇ conjugate); pneumococcal conjugates (e.g., Prevnar - a diphtheria CRM₁₉₇ conjugate) ; and meningococcal conjugate (e.g., Menveo - a diphtheria CRM₁₉₇ conjugate).
Adding new vaccines to the current pediatric immunization schedule can encounter two potential problems that must be addressed. First, the issue of carrier-induced epitopic suppression (or "carrier suppression", as it is generally known) must be addressed, particularly suppression arising from carrier priming. "Carrier suppression" is the phenomenon whereby pre-immunization of an animal with a carrier protein prevents it from later eliciting an immune response against a new antigenic epitope that is presented on that carrier (Herzenberg et al. (1980) Nature 285: 664-667).
As reported in Schutze et al. (1985) J Immunol 135:2319-2322, where several vaccine antigens contain the same protein component (being used as an immunogen and/or as a carrier protein in a conjugate) then there is the potential for interference between those antigens. Schutze *et al.* observed that the immune response against an antigen that was conjugated to a tetanus toxoid (Tt) carrier was suppressed by pre-existing immunity against Tt.

Dagan *et al.* observed that a combination of DTP vaccines with a Hib conjugate vaccine was adversely affected where the carrier for the Hib conjugate was the same as the tetanus antigen from the DTP vaccine ((1998) Infect Immun 66:2093-2098). Dagan *et al.* concluded that this "carrier suppression" phenomenon, arising from interference by a common protein carrier, should be taken into account when introducing vaccines that include multiple conjugates.

In contrast to Schutze *et al.* and Dagan *et al.,* Barington *et al.* reported that priming with tetanus toxoid had no negative impact on the immune response against a subsequently-administered Hib-Tt conjugate, but suppression was seen in patients with maternally acquired anti-Tt antibodies ((1994) Infect Immun 62:9-14). Di John *et al.,* however, observed an "epitopic suppression" effect for a Tt-based peptide conjugate in patients having existing anti-Tt antibodies resulting from tetanus vaccination ((1989) Lancet 2(8677):1415-8).

Granoff *et al.* suggested that a conjugate having CRM₁₉₇ (a detoxified mutant of diphtheria toxin) as the carrier may be ineffective in children that had not previously received diphtheria toxin as part of a vaccine *(e.g.,* as part of a DTP or DT vaccine) ((1993) Vaccine Suppl 1: S46-51). This work was further developed in Granoff et al. (1994) JAMA 272:1116-1121, where a carrier priming effect by D-T immunization was seen to persist for subsequent immunization with Hib conjugates.

In Barington et al. (1993) Infect Immun 61:432-438, the authors found that pre-immunization with a diphtheria or tetanus toxoid carrier protein reduced the increase in anti-Hib antibody levels after a subsequent immunization with the Hib capsular saccharide conjugated to those carriers, with IgG1 and IgG2 being equally affected. Responses to the carrier portions of the conjugates were also suppressed. Furthermore, a more general non-epitope-specific suppression was seen, as pre-immunization with one conjugate was seen to affect immune responses against both the carrier and saccharide portions of a second conjugate that was administered four weeks later.

Thus, given the confusion over the impact of "carrier suppression," having additional carrier proteins available for conjugation will be beneficial to reduce such adverse interactions. Ideally, a carrier protein should induce strong helper effect to a conjugated B-cell epitope (e.g. polysaccharide) without inducing an antibody response against itself. As an alternative to carrier proteins, the use of universal peptide epitopes, which are immunogenic in the context of most major histocompatibility complex class II molecules, is one approach towards this goal [see, e.g., Alexander et al. (2000) J Immunol 164:1625-1633]. Such peptide epitopes have been identified within TT and other proteins. However, using single epitopes was typically insufficient to induce a strong T-cell dependent immune response. To address this, it was demonstrated that that polyepitope carrier peptides comprising multiple T-cell epitopes, but lacking in conformational B-cell epitopes, are particularly useful as carriers for both individual saccharides as well as combinations of saccharides from distinct pathogens. Furthermore, it has been discovered that only a low immunogenic response is seen against these polyepitope carrier peptide even though they comprise a number of known pathogenic epitopes, whereas it would have been expected that the immunogenic response would increase proportionally to the number of pathogenic epitopes [See, e.g., U.S. Patent Publ. 2008/0260773].
Second, given the already crowded immunization schedule, addition of new vaccines to the immunization schedule will become increasingly difficult due to possible adverse interactions, but also due simply to the number of separate injections required. Thus, being able to combine vaccines into a single injection such as the DTaP or MMR vaccines is advantageous. Having additional carrier peptides that can enhance an immune response to a polysaccharide immunogen without inducing an immune response to itself will be beneficial as it can simplify addition of new saccharide based vaccines with existing vaccines into a single injectable composition. WO2004/041310 discloses a conjugate comprising a polysaccharide/oligosaccharide (E) such as a bacterial capsular polysaccharide linked via a dendrimer to an immunomodulating substance. It is an object of the invention to provide further and/or better carrier peptides for conjugation to polysaccharide immunogens and better modes of conjugation to distribute the peptide epitopes along the length of the polysaccharide.

### SUMMARY OF THE INVENTION

The invention is as defined in the claims. In one embodiment, the disclosure provides compositions that includes a nonlinear saccharide conjugate that with a saccharide selected from a polysaccharide and an oligosaccharide that is linked to at least two carrier peptides, where the at least two carrier peptides each have at least one T-cell epitope, but no conformational B-cell epitopes and at least one of the at least two peptides is internally linked to the saccharide. In certain instances, the at least two peptides include a linear B-cell epitope. In other instances, the at least two peptides do not include a linear B-cell epitope. At least one of the at least two peptides has a PV1 T-cell epitope. In certain instances that have a PV1 T-cell epitope, the amino acid sequence comprises SEQ ID NO: 9, which optionally has 1, 2, 3, 4, or 5 single amino acid alterations. In some instances that may be combined with the preceeding instances, the at least two peptides have the same amino acid sequence or different amino acid sequences. In some instances that may be combined with the preceeding instances, the at least two peptides are linked directly to the saccharide or the at least two peptides are linked to the saccharide via a linker. In some instances that may be combined with the preceeding instances with a linker, the linkers for the at least two peptides are the same or are different. In some instances that may be combined with the preceeding instances with a linker, the linker is linear (e.g., straight chain alkyls with 1 to 10 carbon atoms (e.g., C₁, C₂, C₃, C₄, C₅, C₆, C₇, C₈, C₉, C₁₀)). In some instances that may be combined with the preceeding instances with a linker, the linker is N-kappa-Maleimidoundecanoic acid hydrazide-TFA (KMUH) or N-β-Maleimidopropionic acid hydrazide-TFA (BMPH). In certain instances that may be combined with the preceeding instances, the saccharide is not linked to a carrier protein. In some instances that may be combined with the preceeding instances, the saccharide is linked to at least one peptide per five to thirty-five saccharides, at least one peptide per five to twenty-five saccharides, at least one peptide per five to twenty saccharides, or at least one peptide per seven to fifteen saccharides. In certain instances that may be combined with the preceeding instances, the saccharide is linked to at least three peptides, at least four peptides, at least five peptides, at least six peptides, at least seven peptides, at least eight peptides, at least nine peptides, or at least ten peptides. In some instances that may be combined with the preceeding instances, the saccharide is a capsular saccharide such as from *N. meningitidis, S. pneumoniae, S. pyogenes, S. agalactiae, H. influenzae, P. aeruginosa, S. aureus*, *E. faecalis, E. faecium, Y. enteracolitica*, *V*. *cholerae* or *S. typhi.* In other instances that may be combined with the preceeding instances, the saccharide is a glucan such as from *C. albicans, Coccidioides immitis, Trichophyton verrucosum, Blastomyces dermatidis, Cryptococcus neoformans, Histoplasma capsulatum, Saccharomyces cerevisiae, Paracoccidioides brasiliensis,* or *Pythiumn insidiosum.* In some instances that may be combined with the preceeding instances, the saccharide comprises at least ten saccharides, at least fifteen saccharides, at least twenty saccharides, at least twenty-five saccharides, at least thirty saccharides, at least thirty-five saccharides, at least forty saccharides, at least fifty saccharides, at least seventy-five saccharides, or at least one hundres saccharides. In some instances that may be combined with the preceeding instances, the composition also includes a pharmaceutically acceptable carrier. In certain instances that may be combined with the preceeding instances, the compositon also includes an adjuvant. In some instances that may be combined with the preceeding instances, the composition also includes an additional component selected from: a *Neisseria meningitidis* antigen, a *Streptococcus pneumoniae* antigen, a *Streptococcus pyogenes* antigen, a *Moraxella catarrhalis* antigen, a *Bordetella pertussis* antigen, a *Staphylococcus aureus* antigen, a *Staphylococcus epidermis* antigen, a *Clostridium tetani* antigen, a *Cornynebacterium diphtheriae* antigen, a *Haemophilus influenzae* type B (Hib) antigen, a *Pseudomonas aeruginosa* antigen, a *Legionella pneumophila* antigen, a *Streptococcus agalactiae* antigen, a *Neiserria gonorrhoeae* antigen, a *Chlamydia trachomatis* antigen, a *Treponema pallidum* antigen, a *Haemophilus ducreyi* antigen, an *Enterococcus faecalis* antigen, an *Enterococcus faecium* antigen, a *Helicobacter pylori* antigen, a *Staphylococcus saprophyticus* antigen, a *Yersinia enterocolitica* antigen, an *E. coli* antigen, a *Bacillus anthracis* antigen, a *Yersinia pestis* antigen, a *Mycobacterium tuberculosis* antigen, a Rickettsia antigen, a *Listeria monocytogenes* antigen, a *Chlamydia pneumoniae* antigen, a *Vibrio cholerae* antigen, a *Salmonella typhi* antigen, a *Borrelia burgdorferi* antigen, a *Porphyromonas gingivalis* antigen, a Shigella antigen and a Klebsiella antigen.

Another aspect of the disclosure includes methods of inducing an immune response comprising administration of the composition of the preceding aspect any any of its various instances to a subject. In certain instances, the subject is human. In some instances which may be combined with the preceding instance, the immune response recognizes the saccharide. In some instances which may be combined with the preceding instance, the immune response to the saccharide is more T-cell dependent that an immune response induced by the saccharide unlinked to carrier proteins or other T-cell epitopes.

Another aspect of the disclosure includes use of the composition composition of the preceding aspect any any of its various instances to induce an enhanced immune response in a mammalian subject to the saccharide. In certain instances, the mammalian subject is human. In some instances which may be combined with the preceding instance, the enhanced immune response recognizes the saccharide. In some instances which may be combined with the preceding instance, the enhanced immune response to the saccharide is more T-cell dependent that an immune response induced by the saccharide unlinked to carrier proteins or other T-cell epitopes.

Yet another aspect of the present disclosure includes compositions that include a saccharide conjugate that comprises a saccharide selected from a polysaccharide and an oligosaccharide, wherein the saccharide linked to a peptide comprising at least two T-cell epitopes having no conformational B-cell epitopes and wherein at least one of the T-cell epitopes is the PV1 epitope. In certain instances, the peptide includes a linear B-cell epitope. In other instances, the peptide does not include a linear B-cell epitope. In some insances that may be combined with either prior instances, the amino acid sequence of the PV1 epitope comprises SEQ ID NO: 9, which optionally has 1, 2, 3, 4, or 5 single amino acid alterations. In some instances that may be combined with the preceeding instances, the peptide is linked directly to the saccharide or the peptide is linked to the saccharide via a linker. In some instances that may be combined with the preceeding instances with a linker, the linker is the same or are different. In some instances that may be combined with the preceeding instances with a linker, the linker is linear (e.g., straight chain alkyls with 1 to 10 carbon atoms *(e.g.,* C₁, C₂, C₃, C₄, C₅, C₆, C₇, C₈, C₉, C₁₀)). In some instances that may be combined with the preceeding instances with a linker, the linker is N-kappa-Maleimidoundecanoic acid hydrazide-TFA (KMUH) or N-β-Maleimidopropionic acid hydrazide-TFA (BMPH). In certain instances that may be combined with the preceeding instances, the saccharide is not linked to a carrier protein. In certain instances that may be combined with the preceeding instances, the peptide has at least three, at least four, at least five, at least six, at least seven, at least eight, at least nine, or at least ten T-cell epitopes. In some instances that may be combined with the preceeding instances, the saccharide is a capsular saccharide such as from *N. meningitidis, S. pneumoniae, S. pyogenes, S. agalactiae, H. influenzae, P. aeruginosa, S. aureus*, *E. faecalis, E. faecium, Y. enteracolitica, V*. *cholerae* or *S. typhi.* In other instances that may be combined with the preceeding instances, the saccharide is a glucan such as from *C. albicans, Coccidioides immitis, Trichophyton verrucosum, Blastomyces dermatidis, Cryptococcus neoformans, Histoplasma capsulatum, Saccharomyces cerevisiae, Paracoccidioides brasiliensis,* or *Pythiumn insidiosum.* In some instances that may be combined with the preceeding instances, the saccharide comprises at least ten saccharides, at least fifteen saccharides, at least twenty saccharides, at least twenty-five saccharides, at least thirty saccharides, at least thirty-five saccharides, or at least forty saccharides. In some instances that may be combined with the preceeding instances, the composition also includes a pharmaceutically acceptable carrier. In certain instances that may be combined with the preceeding instances, the compositon also includes an adjuvant. In some instances that may be combined with the preceeding instances, the composition also includes an additional component selected from: a *Neisseria meningitidis* antigen, a *Streptococcus pneumoniae* antigen, a *Streptococcus pyogenes* antigen, a *Moraxella catarrhalis* antigen, a *Bordetella pertussis* antigen, a *Staphylococcus aureus* antigen, a *Staphylococcus epidermis* antigen, a *Clostridium tetani* antigen, a *Cornynebacterium diphtheriae* antigen, a *Haemophilus influenzae* type B (Hib) antigen, a *Pseudomonas aeruginosa* antigen, a *Legionella pneumophila* antigen, a *Streptococcus agalactiae* antigen, a *Neiserria gonorrhoeae* antigen, a *Chlamydia trachomatis* antigen, a *Treponema pallidum* antigen, a *Haemophilus ducreyi* antigen, an *Enterococcus faecalis* antigen, an *Enterococcus faecium* antigen, a *Helicobacter pylori* antigen, a *Staphylococcus saprophyticus* antigen, a *Yersinia enterocolitica* antigen, an *E. coli* antigen, a *Bacillus anthracis* antigen, a *Yersinia pestis* antigen, a *Mycobacterium tuberculosis* antigen, a Rickettsia antigen, a *Listeria monocytogenes* antigen, a *Chlamydia pneumoniae* antigen, a *Vibrio cholerae* antigen, a *Salmonella typhi* antigen, a *Borrelia burgdorferi* antigen, a *Porphyromonas gingivalis* antigen, a Shigella antigen and a Klebsiella antigen.

Another aspect of the disclosure includes methods of inducing an immune response comprising administration of the composition of the immediately preceding aspect any any of its various instances to a subject. In certain instances, the subject is human. In some instances which may be combined with the preceding instance, the immune response recognizes the saccharide. In some instances which may be combined with the preceding instance, the immune response to the saccharide is more T-cell dependent that an immune response induced by the saccharide unlinked to carrier proteins or other T-cell epitopes.

Another aspect of the disclosure includes use of the composition composition of the preceding aspect any any of its various instances to induce an enhanced immune response in a mammalian subject to the saccharide. In certain instances, the mammalian subject is human. In some instances which may be combined with the preceding instance, the enhanced immune response recognizes the saccharide. In some instances which may be combined with the preceding instance, the enhanced immune response to the saccharide is more T-cell dependent that an immune response induced by the saccharide unlinked to carrier proteins or other T-cell epitopes.

### DETAILED DESCRIPTION

Pure polysaccharides and oligosaccharides are often poor immunogens and therefore often need to be conjugated to a carrier peptide. Even where a polysaccharide has sufficient immunogenicity, conjugation to a carrier protein can enhance the immunogenicity so that less polysaccharide need be delivered. Furthermore, for protective efficacy in the very young, conjugation to a carrier peptide is often required. The use of conjugation to carrier proteins in order to enhance the immunogenicity of carbohydrate antigens is well known (see, *e.g.,* Lindberg (1999) Vaccine 17 Suppl 2:S28-36, Buttery & Moxon (2000) J R Coll Physicians Lond 34: 163-8, Ahmad & Chapnick (1999) Infect Dis Clin North Am 13: 113-33, vii, Goldblatt (1998) J. Med. Microbiol. 47:563-567, EP-B-0477508, US Pat. No. 5,306,492, WO98/42721, Dick et al. in Conjugate Vaccines (eds. Cruse et al.) Karger, Basel, 1989, Vol. 10, 48-1 14, Hermanson Bioconjugate Techniques, Academic Press, San Diego CA (1996), *etc*.); and is used in particular for pediatric vaccines (Ramsay et al. (2001) Lancet 357(9251):195-6).

As an alternative to carrier proteins, the use of universal peptide epitopes, which are immunogenic in the context of most major histocompatibility complex class II molecules, has been explored [see, e.g., Alexander et al. (2000) J Immunol 164:1625-1633]. Such peptide epitopes have been identified within TT and other proteins. However, using single epitopes was typically insufficient to induce a strong T-cell dependent immune response. To address this, it was demonstrated that that polyepitope carrier peptides comprising multiple T-cell epitopes, but lacking in conformational B-cell epitopes, are particularly useful as carriers for both individual saccharides as well as combinations of saccharides from distinct pathogens. Furthermore, it has been discovered that these polyepitope carrier peptides only induce at most a low immunogenic response against themselves even though they comprise a number of known pathogenic epitopes, whereas it would have been expected that the immunogenic response would increase proportionally to the number of pathogenic epitopes [see, e.g., U.S. Patent Publ. 2008/0260773]. Thus, even with multiple T-cell epitopes in a linear arrangement, the polyepitope carrier peptides induce only an immune response against the saccharide to which they are conjugated without inducing an immune response against themselves.

The inventors have surprisingly found that single peptide epitopes can be as efficacious in inducing a strong T-cell dependent immune response against saccharide antigens when the saccharide antigen has at least two peptide epitopes conjugated to the saccharide such that at least one epitope is linked an internal saccharide forming a nonlinear conjugate.

An aspect of the invention therefore provides a saccharide conjugate comprising a polysaccharide or oligosaccharide conjugated to at least two carrier peptide each comprising at least one T-cell epitope and optionally, an adjuvant (as defined below). The invention therefore provides a saccharide conjugate comprising a polysaccharide or oligosaccharide conjugated to at least one carrier peptide comprising at least two T-cell epitopes one of which is the PV1 epitope and optionally, an adjuvant (as defined below).
The carrier peptides may be covalently conjugated to the saccharide directly or via a linker. Any suitable conjugation reaction can be used, with any suitable linker where necessary.
Attachment of the saccharide to the carrier peptides is preferably through an -SH group, preferably at or near the N- or C-terminus, *e.g.,* through the side chain(s) of a cysteine residue(s). The attachment may alternatively be through an -NH₂ group, *e.g.,* through the side chain(s) of a lysine residue(s) or arginine residue(s) in the carrier peptide. Where the polysaccharide has a free aldehyde group, this group can react with an amine in the carrier peptide to form a conjugate by reductive amination. Alternatively the polysaccharide may be attached to the carrier protein via a linker molecule.
The saccharide will typically be activated or functionalized prior to conjugation. Activation may involve, for example, cyanylating reagents such as CDAP (1-cyano-4-dimethylamino pyridinium tetrafluoroborate). Other suitable techniques use carbodiimides, hydrazides, active esters, norborane, p-nitrobenzoic acid, N-hydroxysuccinimide, S-NHS, EDC, TSTU (see, *e.g.,* the introduction to WO98/42721).
Direct linkages to the carrier peptide may comprise oxidation of the saccharide followed by reductive amination with the carrier peptide, as described in, for example, U.S. Pat No. 4,761,283 and U.S. Pat No. 4,356,170.
Linkages via a linker group may be made using any known procedure, for example, the procedures described in U.S. Pat No. 4,882,317 and U.S. Pat No. 4,695,624. Typically, the linker is attached via an anomeric carbon of the saccharide. A preferred type of linkage is an adipic acid linker, which may be formed by coupling a free -NH2 group (*e.g.,* introduced to a polysaccharide by amination) with adipic acid (using, for example, diimide activation), and then coupling a protein to the resulting saccharide-adipic acid intermediate (see, *e.g.,* EP-B-0477508, Mol. Immunol, (1985) 22, 907-919, and EP-A-0208375). A similar preferred type of linkage is a glutaric acid linker, which may be formed by coupling a free -NH group with glutaric acid in the same way. Adipic and glutaric acid linkers may also be formed by direct coupling to the polysaccharide, *i.e.,* without prior introduction of a free group, *e.g.,* a free -NH group, to the polysaccharide, followed by coupling a protein to the resulting saccharide-adipic/glutaric acid intermediate. Another preferred type of linkage is a carbonyl linker, which may be formed by reaction of a free hydroxyl group of a modified polysaccharide with CDI (Bethell G.S. et al. (1979) J Biol Chem 254, 2572-4 and Hearn M.T.W. (1981) J. Chromatogr 218, 509-18); followed by reaction with a protein to form a carbamate linkage. Other linkers include β-propionamido (WO00/10599), nitrophenyl-ethylamine (Gever et al. (1979) Med Microbiol Immunol 165, 171-288), haloacyl halides (U.S. Pat. No. 4,057,685), glycosidic linkages (U.S. Pat. Nos. 4,673,574; 4,761,283; and 4,808,700), 6-aminocaproic acid (U.S. Pat. No. 4,459,286), N-succinimidyl-3-(2-pyridyldithio)-propionate (SPDP) (U.S. Pat. No. 5,204,098), adipic acid dihydrazide (ADH) (U.S. Pat. No. 4,965,338), C4 to C12 moieties (U.S. Pat. No. 4,663, 160), *etc.* Carbodiimide condensation can also be used (WO2007/000343).

A bifunctional linker may be used to provide a first group for coupling to an amine group in the saccharide *(e.g.,* introduced to the polysaccharide by amination) and a second group for coupling to the carrier (typically for coupling to an amine in the carrier). Alternatively, the first group is capable of direct coupling to the polysaccharide, *i.e.,* without prior introduction of a group, *e.g.,* an amine group, to the polysaccharide.

In some embodiments, the first group in the bifunctional linker is thus able to react with an amine group (-NH2) on the polysaccharide. This reaction will typically involve an electrophilic substitution of the amine's hydrogen. In other embodiments, the first group in the bifunctional linker is able to react directly with the polysaccharide. In both sets of embodiments, the second group in the bifunctional linker is typically able to react with an amine group on the carrier peptide. This reaction will again typically involve an electrophilic substitution of the amine.

Where the reactions with both the polysaccharide and the carrier protein involve amines then it is preferred to use a bifunctional linker. For example, a homobifunctional linker of the formula X-L-X, may be used where: the two X groups are the same as each other and can react with the amines; and where L is a linking moiety in the linker. Similarly, a heterobifunctional linker of the formula X-L-X may be used, where: the two X groups are different and can react with the amines; and where L is a linking moiety in the linker. A preferred X group is N-oxysuccinimide. L preferably has formula L'-L²-L', where L' is carbonyl. Preferred L² groups are straight chain alkyls with 1 to 10 carbon atoms (*e.g.,* C₁, C₂, C₃, C₄, C₅, C₆, C₇, C₈, C₉, C₁₀) *e.g.* -(CH₂)₄- or -(CH₂)₃-.

Other X groups for use in the bifunctional linkers described in the preceding paragraph are those which form esters when combined with HO-L-OH, such as norborane, p-nitrobenzoic acid, and sulfo-N- hydroxysuccinimide.

Further bifunctional linkers for use with the invention include acryloyl halides (*e.g.,* chloride) and haloacylhalides.

The linker will generally be added in molar excess to polysaccharide during coupling to the polysaccharide.

After conjugation, free and conjugated saccharides can be separated. There are many suitable methods, e.g., ammonium sulfate precipitation, hydrophobic chromatography, tangential ultrafiltration, diafiltration, *etc.* (see also Lei et al. (2000) Dev Biol (Basel) 103:259-264 and WO00/38711). Tangential flow ultrafiltration is preferred.

The saccharide moiety in the conjugate is may be high molecular weight, low molecular weight, or intermediate molecular weight polysaccharides, as defined below (see section on Saccharide Immunogens). Oligosaccharides will typically be sized prior to conjugation.

The carrier peptide-saccharide conjugate is preferably soluble in water and/or in a physiological buffer.

For some saccharides, the immunogenicity may be improved if there is a spacer between the polysaccharide and the carrier protein. In this context, a "spacer" is a moiety that is longer than a single covalent bond. This spacer may be a linker (*e.g.,* a KMUH linker), as described above. Alternatively, it may be a moiety covalently bonded between the polysaccharide and a linker. Typically, the moiety will be covalently bonded to the polysaccharide prior to coupling to the linker or carrier. For example, the spacer may be moiety Y, wherein Y comprises a straight chain alkyl with 1 to 15 carbon atoms (*e.g.,* C₁, C₂, C₃, C₄, C₅, C₆, C₇, C₈, C₉, C₁₀, C₁₁, C₁₂, C₁₃, C₁₄, C₁₅), typically 6 to 12 carbon atoms (*e.g.,* C₆, C₇, C₈, C₉, C₁₀, C₁₁, C₁₂). The inventors have found that a straight chain alkyl with 10 carbon atoms (*i.e.,* -(CH₂)₁₀) is particularly suitable, and may provide greater immunogenicity than shorter chains (e.g., -(CH₂)₂). Typically, Y is attached to the anomeric carbon of the polysaccharide, usually via an -O- linkage. However, Y may be linked to other parts of the polysaccharide and/or via other linkages. The other end of Y is bonded to the linker by any suitable linkage. Typically, Y terminates with an amine group to facilitate linkage to a bifunctional linker as described above. In these embodiments, Y is therefore bonded to the linker by an -NH- linkage. Accordingly, a conjugate with the following structure is specifically envisaged for use in the present invention: wherein n+2 is in the range of 2-60, *e.g.,* between 10-50 or between 2-40. Preferably, n+2 is in the range of 25-30 or 11-19, *e.g.,* 13-17. The inventors have found that n+2 = 15 is suitable. Y is as described above.

In one aspect, the invention provides a method for making a saccharide conjugated to a carrier peptide(s), wherein the step of conjugation is carried out in a phosphate buffer with >10mM phosphate; and to a conjugate obtained by this method. The inventors have found that sodium phosphate is a suitable form of phosphate for the buffer. The pH of the buffer may be adjusted to between 7.0-7.5, particularly 7.2. The step of conjugation is typically carried out in a phosphate buffer with between 20-200 mM phosphate, *e.g.,* 50-150 mM. In particular, the inventors have found that a phosphate buffer with 90-110 mM, *e.g.,* about 100 mM, phosphate is suitable. The step of conjugation is usually carried out at room temperature. Similarly, the step of conjugation is usually carried out at room pressure. Typically, the saccharide is attached to a linker as described above prior to the step of conjugation. In particular, the saccharide may be attached to one or more bifunctional linkers as described above. The free end of the linker(s) may comprise a group to facilitate conjugation to the carrier peptide(s). For example, the inventors have found that the free end of the linker may comprise an ester group, *e.g.,* an N-hydroxysuccinimide ester group.

The saccharide conjugates disclosed herein can further include a pharmaceutically acceptable carrier.

The saccharide conjugates disclosed herein can further include an adjuvant. The adjuvant can comprise one or more of the adjuvants described below.

The saccharide conjugates may also be used in methods for raising an immune response in a mammal (or avian), comprising administering to the mammal (or avian) a composition of the invention.

### Saccharide Immunogens

Any polysaccharide or oligosaccharide capable of inducing an immune response in a mammal or avian may be used in the nonlinear saccharide conjugates and linear saccharide conjugates as disclosed herein (*i.e.,* a saccharide immunogen). Preferably, the saccharide is capably of inducing an immune response against a pathogen of interest. The saccharide may be branched or linear.

Low molecular weight saccharides may be used, particularly those with a molecular weight of less than 100 kDa (*e.g.,* less than 80, 70, 60, 50, 40, 30, 25, 20, or 15 kDa). It is also possible to use oligosaccharides containing, for example, 60 or fewer (*e.g.,* 59, 58, 57, 56, 55, 54, 53, 52, 51, 50, 49, 48, 47, 46, 45, 44, 43, 42, 41, 40, 39, 38, 37, 36, 35, 34, 33, 32, 31, 30, 29, 28, 27, 26, 25, 24, 23, 22, 21, 20, 19, 18, 17, 16, 15, 14, 13, 12, 11, 10, 9, 8, 7, 6, 5, or 4) monosaccharide units. Within this range, oligosaccharides with between 10 and 50 or between 20 and 40 monosaccharide units are preferred.

High molecular weight or even native polysaccharides may be used, particularly those with a molecular weight of greater than 50 kDa (*e.g.,* greater than 60, 70, 80, 90, 100, 125, 150, 175, 200, 225, 250, 270, 300, 350, or 400 kDa). It is also possible to use saccharides containing, for example, 20 or more(e.g., 21, 22, 23, 24, 25, 26, 27, 28, 29, 30, 32, 34, 36, 38, 40, 42, 44, 46, 48, 50, 55, 60, 65, 70, 75, 80, 85, 90, 95, or 100) monosaccharide units. Within this range, saccharides with greater than fifty or even greater than one hundred monosaccharide units are preferred.

Exemplary saccharide immunogens are detailed below.

Preferably, the saccharide immunogen in the conjugates in a composition of the invention is a bacterial saccharide and in particular a bacterial capsular saccharide.

Examples of bacterial capsular saccharides which may be included in the compositions of the invention include capsular saccharides from *Neisseria meningitidis* (serogroups A, B, C, W135 and/or Y), *Streptococcus pneumoniae* (serotypes 1, 2, 3, 4, 5, 6B, 7F, 8, 9N, 9V, 10A, 11A, 12F, 14, 15B, 17F, 18C, 19A, 19F, 20, 22F, 23F and 33F, particularly 4, 6B, 9V, 14, 18C, 19F and/or 23F), *Streptococcus agalactiae* (types 1a, 1b, II, III, IV, V, VI, VII, and/or VIII, such as the saccharide antigens disclosed in references 20-23), *Haemophilus influenzae* (typeable strains: a, b, c, d, e and/or f), *Pseudomonas aeruginosa* (for example LPS isolated from PA01, O5 serotype), *Staphylococcus aureus* (from, for example, serotypes 5 and 8), *Enterococcus faecalis* or *E. faecium* (trisaccharide repeats), *Yersinia enterocolitica, Vibrio cholerae*, *Salmonella typhi, Klebsiella spp., etc.* Other saccharides which may be included in the compositions of the invention include glucans (*e.g.* fungal glucans, such as those in *Candida albicans*), and fungal capsular saccharides *e.g.* from the capsule of *Cryptococcus neoformans.*

***N. meningitidis*:** In certain embodiments, the conjugate compositions may include capsular saccharides from at least two of serogroups A, C, W135 and Y of *Neisseria meningitidis.* In other embodiments, such compositions further comprise an antigen from one or more of the following: (a) *N. meningitidis*; (b) *Haemophilus influenzae* type B; *Staphylococcus aureus*, groups A and B streptococcus, pathogenic *E. coli*, and/or (c) *Streptococcus pneumoniae.*

The natural *N. meningitidis* serogroup A (MenA) capsule is a homopolymer of (a1→6)-linked N-acetyl-D-mannosamine-1-phosphate, with partial O-acetylation in the C3 and C4 positions. The N. meningitidis serogroup B (MenB) capsule is a homopolymer of (a2→8)-linked sialic acid. The N. meningitidis serogroup C (MenC) capsular saccharide is a homopolymer of (a2→9) linked sialic acid, with variable O-acetylation at positions 7 and/or 8. The N. meningitidis serogroup W135 saccharide is a polymer having sialic acid-galactose disaccharide units [→4)-D-Neup5Ac(7/9OAc)-α-(2→6)-D-Gal-α-(1→). It has variable O-acetylation at the 7 and 9 positions of the sialic acid. The N. meningitidis serogroup Y saccharide is similar to the serogroup W135 saccharide, except that the disaccharide repeating unit includes glucose instead of galactose [→4)-D-Neup5Ac(7/9OAc)-α-(2→6)-D-Glc-α-(1→). It also has variable O-acetylation at positions 7 and 9 of the sialic acid.

In certain embodiments the conjugate compositions include capsular saccharides from serogroups C, W135 & Y of *N. meningitidis.* In certain embodiments the conjugate compositions include capsular saccharides from serogroups A, C, W135 & Y of *N. meningitidis.* In certain embodiments the conjugate compositions include capsular saccharides from *H. influenzae* type B and serogroups C, W135 & Y of *N. meningitidis.* In certain embodiments the conjugate compositions include capsular saccharides from *H. influenzae* type B and serogroups A, C, W135 & Y of *N. meningitidis.* In certain embodiments the conjugate compositions include capsular saccharides from *S. pneumoniae* and serogroups C, W135 & Y of *N. meningitidis.* In certain embodiments the conjugate compositions include capsular saccharides from *S. pneumoniae* and serogroups A, C, W135 & Y of *N. meningitidis.* In certain embodiments the conjugate compositions include capsular saccharides from *H. influenzae* type B, *S. pneumoniae* and serogroups C, W135 & Y of *N. meningitidis.* In certain embodiments the conjugate compositions include capsular saccharides from *H. influenzae* type B, *S. pneumoniae* and serogroups A, C, W135 & Y of *N. meningitidis.*

***Streptococcus pneumoniae*:** *Streptococcus pneumoniae* saccharide conjugates may include a saccharide (including a polysaccharide or an oligosaccharide) and optionally one or more proteins from *Streptococcus pneumoniae.* Saccharide antigens maybe selected from serotypes 1, 2, 3, 4, 5, 6B, 7F, 8, 9N, 9V, 1OA, HA, 12F, 14, 15B, 17F, 18C, 19A, 19F, 20, 22F, 23F, and 33F. Optional protein antigens may be selected from a protein identified in WO98/18931, WO98/18930, U.S. Pat. No. 6,699,703, U.S. Pat. No. 6,800,744, WO97/43303, and WO97/37026. *Streptococcus pneumoniae* proteins may be selected from the Poly Histidine Triad family (PhtX), the Choline Binding Protein family (CbpX), CbpX truncates, LytX family, LytX truncates, CbpX truncate-LytX truncate chimeric proteins, pneumolysin (Ply), PspA, PsaA, Spl28, SpIOl, Spl30, Spl25 or Spl33. ***Staphylococcus aureus*:** *Staphylococcus aureus* saccharide conjugates may include *S. aureus* type 5, 8 and 336 capsular saccharides and fragments thereof and optionally protein antigens derived from surface proteins, invasins (leukocidin, kinases, hyaluronidase), surface factors that inhibit phagocytic engulfment (capsule, Protein A), carotenoids, catalase production, Protein A, coagulase, clotting factor, and/or membrane-damaging toxins (optionally detoxified) that lyse eukaryotic cell membranes (hemolysins, leukotoxin, leukocidin). Exemplary depolymerization methods of generating fragments of *S. aureus* capsular saccharides may be found in U.S. Ser. No. 61/247,518, titled "Conjugation of Staphylococcus Aureus Type 5 and Type 8 Capsular Polysaccharides," filed Sept. 30, 2009, from page 5, line 6 through page 6, line 23, which is hereby referenced for its teaching of such depolymerization techniques. ***Haemophilus influenzae* B (Hib):** Hib saccharide conjugates may include Hib saccharide antigens. ***Streptococcus agalactiae* (Group B Streptococcus):** Group B Streptococcus saccharide conjugates may include saccharide antigens derived from serotypes Ia, Ib, Ia/c, II, III, IV, V, VI, VII and VIII as identified in WO04/041157 and optionally one or more protein antigens including, without limitation as identified in WO02/34771, WO03/093306, WO04/041157, or WO05/002619 (including by way of example proteins GBS 80, GBS 104, GBS 276 and GBS 322).
***Vibrio cholerae*:** *V*. *cholerae* saccharide conjugates may include LPS, particularly lipopolysaccharides of Vibrio cholerae II, O1 Inaba O-specific polysaccharides.
***Salmonella typhi*** (typhoid fever): Saccharide conjugates may include capsular polysaccharides such as Vi.
***Glucan saccharides*:** The saccharides for conjugates include glucans. Glucans are glucose-containing polysaccharides found in, among other pathogens, fungal cell walls. The β-glucans include one or more α-linkages between glucose subunits, whereas β-glucans include one or more β-linkages between glucose subunits. The glucan used in accordance with the invention includes β linkages, and may contain only β linkages (*i.e.,* no α linkages).
The glucan may comprise one or more β-1,3-linkages and/or one or more β-1,6-linkages. It may also comprise one or more β-1,2-linkages and/or β-1,4-linkages, but normally its only β linkages will be β-1,3-linkages and /or β-1,6-linkages.

The glucan may be branched or linear.

Full-length native β-glucans are insoluble and have a molecular weight in the megadalton range. It is preferred to use soluble glucans in immunogenic compositions of the invention. Solubilization may be achieved by fragmenting long insoluble glucans. This may be achieved by hydrolysis or, more conveniently, by digestion with a glucanase (*e.g.,* with a β-1,3-glucanase or a β-1,6-glucanase). As an alternative, short glucans can be prepared synthetically by joining monosaccharide building blocks.

Low molecular weight glucans may be used, particularly those with a molecular weight of less than 100 kDa (*e.g.,* less than 80, 70, 60, 50, 40, 30, 25, 20, or 15 kDa). It is also possible to use oligosaccharides containing, for example, 60 or fewer (*e.g.,* 59, 58, 57, 56, 55, 54, 53, 52, 51, 50, 49, 48, 47, 46, 45, 44, 43, 42, 41, 40, 39, 38, 37, 36, 35, 34, 33, 32, 31, 30, 29, 28, 27, 26, 25, 24, 23, 22, 21,20, 19, 18, 17, 16, 15, 14, 13, 12, 11, 10, 9, 8, 7, 6, 5, or 4) glucose monosaccharide units. Within this range, oligosaccharides with between 10 and 50 or between 20 and 40 monosaccharide units are preferred.

High molecular weight or even glucans may also be used, particularly those with a molecular weight of greater than 50 kDa (*e.g.,* greater than 60, 70, 80, 90, 100, 125, 150, 175, 200, 225, 250, 270, 300, 350, or 400 kDa). It is also possible to use saccharides containing, for example, 20 or more(*e.g*., 21, 22, 23, 24, 25, 26, 27, 28, 29, 30, 32, 34, 36, 38, 40, 42, 44, 46, 48, 50, 55, 60, 65, 70, 75, 80, 85, 90, 95, or 100) monosaccharide units. Within this range, saccharides with greater than 50 or greater than 100 monosaccharide units are preferred.

The glucan may be a fungal glucan. A "fungal glucan" will generally be obtained from a fungus but, where a particular glucan structure is found in both fungi and non-fungi (*e.g.,* in bacteria, lower plants or algae) then the non-fungal organism may be used as an alternative source. Thus the glucan may be derived from the cell wall of a Candida, such as *C. albicans,* or from *Coccidioides immitis, Trichophyton verrucosum, Blastomyces dermatidis, Cryptococcus neoformans, Histoplasma capsulatum, Saccharomyces cerevisiae, Paracoccidioides brasiliensis,* or *Pythiumn insidiosum.* Exemplary sources of fungal β-glucans may be found in WO2009/077854.

In some embodiments, the glucan is a β-1,3 glucan with some β-1,6 branching, as seen in, for example, laminarins. Laminarins are found in brown algae and seaweeds. The β(1-3):β(1-6) ratios of laminarins vary between different sources, for example, the ratio is as low as 3:2 in *Eisenia bicyclis* laminarin, but as high as 7:1 in *Laminaria digititata* laminarin (Pang et al. (2005) Biosci Biotechnol Biochem 69:553-8). Thus the glucan used with the invention may have a β(1-3): β(1-6) ratio of between 1.5:1 and 7.5:1 (*e.g*., about 2:1, 3:1, 4:1, 5:1, 6:1 or 7:1). Optionally, the glucan may have a terminal mannitol subunit, e.g., a 1,1-α-linked mannitol residue (Read et al. (1996) Carbohydr Res. 281:187-201). The glucan may also comprise mannose subunits.

In other embodiments, the glucan has exclusively or mainly β-1,3 linkages, as seen in curdlan. The inventors have found that these glucans may be more immunogenic than glucans comprising other linkages, particularly glucans comprising β-1,3 linkages and a greater proportion of β-1,6 linkages. Thus the glucan may be made solely of β-1,3-linked glucose residues (*e.g.,* linear β-D-glucopyranoses with exclusively 1,3 linkages). Optionally, though, the glucan may include monosaccharide residues that are not β-1,3-linked glucose residues, *e.g.,* it may include β-1,6-linked glucose residues. The ratio of β-1,3-linked glucose residues to these other residues should be at least 8:1 (e.g. >9:1, >10:1, > 11:1, >12:1, >13:1, >14:1, >15:1, >16:1, >17:1, >18:1, >19:1, >20:1, >25:1, >30:1, >35:1, >40:1, >45:1, >50:1, >75:1, >100:1, *etc*.) and/or there are one or more (e.g. >1, >2, >3, >4, >5, >6, >7, >8, >9, >10, > 11, >12, etc.) sequences of at least five (e.g. >5, >6, >7, >8, >9, >10, >11, >12, >13, >14, >15, >16, >17, >18, >19, >20, >30, >40, >50, >60, etc.) adjacent non-terminal residues linked to other residues only by β-1,3 linkages. By "non-terminal" it is meant that the residue is not present at a free end of the glucan. In some embodiments, the adjacent non-terminal residues may not include any residues conjugated to a carrier molecule, linker or other spacer as described below. It has been found that the presence of five adjacent non-terminal residues linked to other residues only by β-1,3 linkages may provide a protective antibody response, *e.g.,* against *C. albicans*.

In further embodiments, a composition may include two different glucans, *e.g.,* a first glucan having a β(1-3): β(1-6) ratio of between 1.5:1 and 7.5:1, and a second glucan having exclusively or mainly β-1,3 linkages. For instance a composition may include both a laminarin glucan and a curdlan glucan.

Exemplary methods of preparing β-glucans may be found in WO2009/077854.

Laminarin and curdlan are typically found in nature as high molecular weight polymers e.g. with a molecular weight of at least 100kDa. They are often insoluble in aqueous media. In their natural forms, therefore, they are not well suited to immunization. Nonlinear polyconjugation with more hydrophilic peptides can overcome this solubility issue rendering the high molecular weight polymers suitable for immunization as conjugates. In particular, glucans with the following structures (A) or (B) are specifically envisaged for use in the present invention: 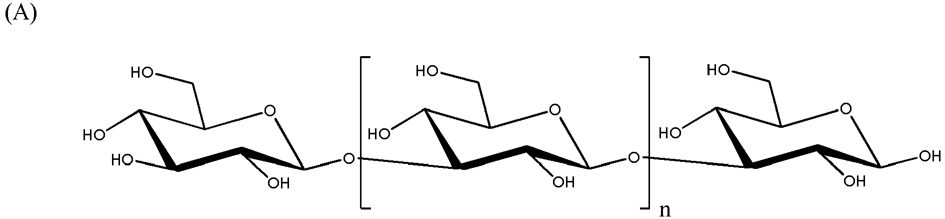 wherein n+2 is in the range of 2-60, *e.g.,* between 10-50 or between 2-40. Preferably, n+2 is in the range of 25-30 or 11-19, *e.g.,* 13-17. The inventors have found that n+2 = 15 is suitable. 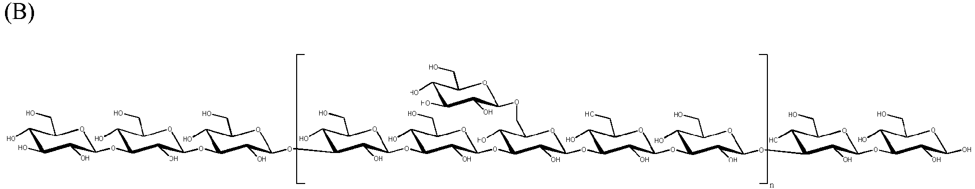 wherein n is in the range of 0-9, *e.g.,* between 1-7 or between 2-6. Preferably, n is in the range of 3-4 or 1-3. The inventors have found that n = 2 is suitable.

The glucan (as defined above) is preferably a single molecular species. In this embodiment, all of the glucan molecules are identical in terms of sequence. Accordingly, all of the glucan molecules are identical in terms of their structural properties, including molecular weight, *etc.* Typically, this form of glucan is obtained by chemical synthesis, *e.g.,* using the methods described above. For example, Jamois et al. (2005) Glycobiology 15(4):393-407, describes the synthesis of a single β-1,3 linked species. Alternatively, in other embodiments, the glucan may be obtained from a natural glucan, *e.g.,* a glucan from *L. digitata*, Agrobacterium or Euglena as described above, with the glucan being purified until the required single molecular species is obtained. Natural glucans that have been purified in this way are commercially available. A glucan that is a single molecular species may be identified by measuring the polydispersity (Mw/Mn) of the glucan sample. This parameter can conveniently be measured by SEC-MALLS, for example as described in Bardotti et al. (2008) Vaccine 26:2284-96. Suitable glucans for use in this embodiment of the invention have a polydispersity of about 1, *e.g.,* 1.01 or less.

The solubility of natural glucans, such as curdlan, can be increased by introducing ionic groups (*e.g.,* by sulfation, particularly at O-6 in curdlan). Such modifications may be used with the invention, but are ideally avoided as they may alter the glucan's antigenicity.

When glucans are isolated from natural sources, they may be isolated in combination with contaminants. For example, the inventors have found that glucans may be contaminated with phlorotannin, which is identifiable by ultraviolet-visible (UV/VIS) spectroscopy. This problem is particularly common when the glucan is isolated from a brown alga or seaweed. For example, the UV spectrum of a commercially-available laminarin extracted from *Laminaria digitata* includes an absorption peak resulting from the presence of phlorotannin contamination. Similarly, glucans extracted from *Artie laminarialis*, *Saccorhiza dermatodea* and *Alaria esculenta* have UV spectra that include an absorption peak resulting from phlorotannin contamination.

The presence of phlorotannin in a sample of glucan may affect the biological properties of the glucan. Accordingly, it may be desirable to remove phlorotannin from the sample, especially when the glucan is for medical use numerous aspects of the present invention. Exemplary methods of removing phlorotannins from β-glucans may be found in WO2009/077854.

### Carrier peptides

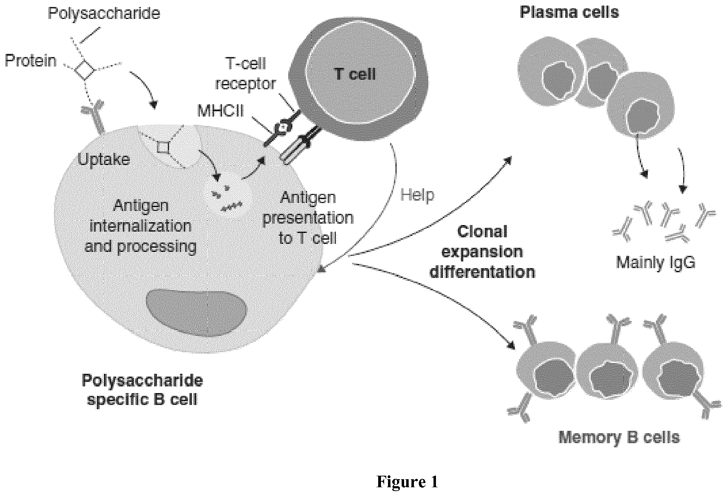

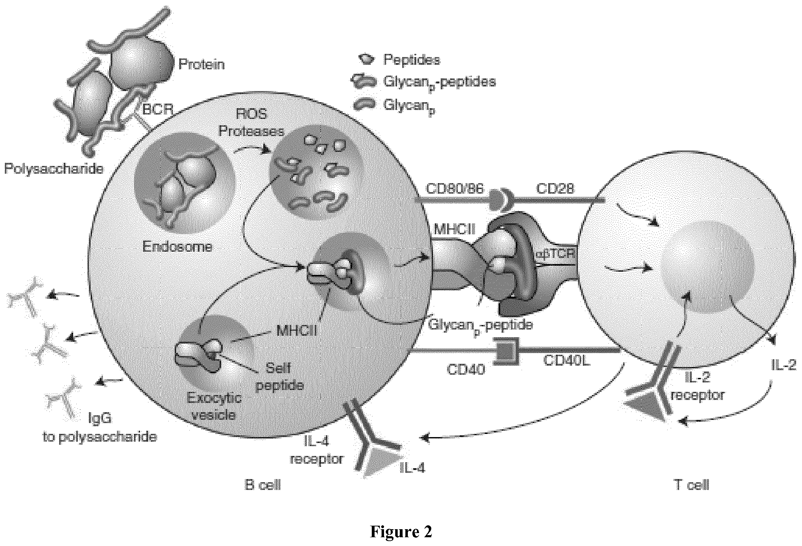

As discussed above, polysaccharides are typically weak T-independent antigens. This immunogenicity can be increased in quantity and quality by conjugation to protein carriers, which shift the immune response to a more T-cell dependent response. As shown in Figure 1, the current model is that the conjugates are processed inside the antigen presenting cells and broken down in peptides or glycopeptides which are presented by the major histocompatibility complex (MHC) class II proteins; further interaction with carrier-specific T cells then induces polysaccharide-specific B-cell differentiation providing the stimuli for antibody production and memory. As a consequence, a conjugate vaccine induces a T-cell dependent (TD) response already early in life which leads to immunological memory and boosting of the response by further doses of the vaccine [Costantino, P. et al. Expert Opin. Drug Discov. 2011, 6(10), 1045-1066].
In search of alternative carriers, others have replaced carrier proteins with synthetic peptides which contain selected CD4+ T cell epitopes [Falugi, F. et al. Eur. J. Immunol, 2001, 31, 3816-3824]. In literature some papers reported the preparation of glycopeptides where the whole carrier protein has been substituted with T-cell epitope peptides inducing immune response comparable or higher respect to a conventional glycoconjugate vaccine. Regarding the development of vaccines against *Haemophilus influenzae* type b (Hib), synthetic fragments of the capsular polysaccharide, poly-3-β-D-ribose-(1, 1)-D-ribitol-5-phosphate (sPRP) has been conjugated to synthetic peptides derived from Hib outer membrane proteins P1, P2 and P6. These synthetic glycopeptides were immunogenic, eliciting anti-PRP antibody responses, and moreover they induced protective level of anti-PRP antibody [Kandil, A.A. et al. Glycoconjugate Journal, 1997, 14, 13-17].
For the vaccine against *Streptococcus pneumoniae* type 17F, in order to substitute the carrier protein, some peptides were selected on basis of their demonstrated or predicted Th-cell-stimulating properties: HSP, a dominant Th epitope from mycobacterial hsp65; HAG, from influenza virus hemagglutinin; Pneumolysin (or its toxoids). As saccharide antigen, pneumococcal polysaccharide type 17F (PS) has been selected and the resulting glycopeptides consistently have elicited in mice anti-PS IgM and IgG responses [De Velasco, E.A. et al. Infection and Immunity, 1995, 63, 961-968]. As shown in Figure 2, Avcil *et al.* proposed a new working model for glycoconjugate vaccine, where the processing of both the protein and the carbohydrate portions of glycoconjugates generated glycanₚ-peptides. MHCII binding of the peptide portion of the glycanₚ-peptide allowed the presentation by MHCII of the more hydrophilic carbohydrate to the αβ receptor of CD4+ T cells (αβTCR). The αβ receptor of CD4+ T helper cells recognized and responded to glycanₚ epitopes presented in the context of MHCII. Activation of the T cell by the carbohydrate-MHCII, along with co-stimulation, resulted in T cell production of cytokines such as IL-4 and IL-2, which in turn induced maturation of the cognate B cell to become a memory B cell, with consequent production of carbohydrate-specific IgG antibodies as reported in the picture below. In summary, following this working model, T cells could recognize polysaccharides and possibly other structures if they are covalently linked to a peptide anchor, as demonstrated by the T cell clone recognition of a pure carbohydrate epitope regardless of the peptide to which they are linked on a prototypical GBS type III-TT conjugate. Thus, there is a fair amount of literature available to guide the skilled artisan in selection of T-cell epitopes for use in the conjugates disclosed herein.

The carrier peptides may comprise one or more T-cell epitopes (e.g. 1, 2, 3, 4, 5, 6, 7, 8, 9, 10, 11, 12, 13, 14, 15, 16, 17, 18, 19, 20, 22, 24, 26, 28, 30, 32, 34, 36, 38, 40, 45, 50, 55, 60, 65, 70, 75, 80, 85, 90, 95, 100 or more). For the conjugates, the carrier peptide preferably has six or fewer T-cell epitopes, five or fewer T-cell epitopes, four or fewer T-cell epitopes, three or fewer T-cell epitopes, six or fewer T-cell epitopes, or only one T-cell epitope. For the polyepitope carrier peptides, the carrier peptide preferably comprises 6 or more T-cell, epitopes or 10 or more T-cell epitopes, where at least one epitope is the PV1 epitope. More preferably the polyepitope carrier peptide comprises nineteen or more T-cell epitopes, where at least one epitope is the PV1 epitope. The carrier peptide preferably comprises at least one PV1 T-cell epitope, at least two PV1 T-cell epitopes, at least three PV1 T-cell epitopes, at least four PV1 T-cell epitopes, at least five PV 1 T-cell epitopes, at least one PV1 T-cell epitopes, or all of the T-cell epitopes maybe PV1 T-cell epitopes. Each carrier peptide may only have one copy of a particular epitope or may have more than one copy of a particular epitope. The epitopes are CD4⁺ T-cell epitopes. Preferably the polyepitope carrier peptide comprises at least one bacterial epitope in addition to the PV1 viral epitope. Preferably the epitopes are derived from antigens to which the human population is frequently exposed either by natural infection or vaccination, for example, epitopes may be derived from Hepatitis A virus, Hepatitis B virus, Measles virus, Influenza Virus, Varicella-zoster virus, poliovirus, heat shock proteins from Mycobacterium bovis and M. leprae and/or Streptococcus strains etc. Preferably the epitopes are selected from Tetanus toxin (TT), Plasmodium falciparum CSP (PfCs), Hepatitis B virus nuclear capsid (HBVnc), Influenza haemagglutinin (HA), HBV surface antigen (HBsAg) and Influenza matrix (MT). The epitopes used in the carrier peptides are preferably selected the following table 1. Most preferably the epitope is the PV1 epitope.

| Peptide name | Source | Amino acid sequence [SEQ ID NO] |
|---|---|---|
| P32TT | Tetanus toxin | LKFIKRYTPNNEIDS- [1] |
| P30TT | Tetanus toxin | FNNFTVSFWLRVPKVSAHLE- [2] |
| Hpw | *Candida albicans* | QGETEEALIQKRSY- [3] |
| Eno1 | *Candida albicans* | DSRGNPTVEVDFTT- [4] |
| Gap1 | *Candida albicans* | NRSPSTGEQKSSGI- [5] |
| Fba | *Candida albicans* | YGKDVKDLFDYAQE- [6] |
| Met6 | *Candida albicans* | PRIGGQRELKKITE- [7] |
| P2TT | Tetanus toxin | QYIKANSKFIGITE [8] |
| PV1 | Poliovirus | KLFAVWKITYKDT [9] |
| P23TT | Tetanus toxin | VSIDKFRIFCKANPK [10] |
| P32TT | Tetanus toxin | LKFIIKRYTPNNEIDS [11] |
| P21TT | Tetanus toxin | IREDNNITLKLDRCNN [12] |
| PfCs | P. falciparum | EKKIAKMEKASSVFNVVN [13] |
| HBVnc | Hepatitis B | PHHTALRQAILCWGELMTLA [14] |
| HA | Influenza virus | PKYVKQNTLKLAT [15] |
| HBsAg | Hepatitis B virus | FFLLTRILTIPQSLD [16] |
| MT | Influenza virus (matrix protein) | YSGPLKAEIAQRLEDV [17] |
| OvaP | Ovalbumin | ISQAVHAAHAEINEAGR [18] |

Any of the epitope sequences may have 1, 2, 3, 4, 5, 6, 7, 8, 9 or 10 (or more) single amino acid alterations (deletions, insertions, substitutions), which may be at separate locations or may be contiguous, as compared to SEQ ID NOs 1-18.

T cell epitopes are well known and characterized antigenic peptide fragments typically derived from a pathogen that, when presented by a major histocompatibility complex (MHC) molecule, interact with T cell receptors after transport to the surface of an antigen-presenting cell. Experimentally determined affinity data have been used to develop a variety of MHC binding prediction algorithms, which can distinguish binders from non-binders based on the peptide sequence [Davies, m.N.; Flower, D.R. Harnessing bioinformatics to discover new vaccines Drug Discovery Today, 2007, 12 ( 9/10), 389-395;]. These algorithms can be used to select additional T-cell epitopes for use in the conjugates disclosed herein.

Where there are multiple epitopes in a carrier peptide, the epitopes preferably are joined by spacers. Preferably the spacer is a short (e.g. 1, 2, 3, 4 or 5) amino acid sequence which is not an epitope. A preferred spacer comprises one or more glycine residues, e.g. -KG-. Preferably the longer carrier peptides comprise a N- or C-terminal region comprising a six-His tail, an immunoaffinity tag useful for screening or purifying the carrier peptide (for example the sequence "MDYKDDDD" [SEQ ID NO: 18] may be used), and/or a protease cleavage sequence. Preferably the proteolytic sequence is the factor Xa recognition site.

Preferably the carrier peptide comprises no suppressor T cell epitopes.

In addition to T-cell epitopes, carrier peptides may comprise other peptides or protein fragments, such as epitopes from immunomodulating cytokines such as interleukin-2 (IL-2) or granulocyte-macrophage colony stimulating factor (GM-CSF).

Carrier peptides used with the invention can be prepared by various means (*e.g.* recombinant expression, purification from cell culture, chemical synthesis, *etc*.)*.* Chemically synthesized peptides are preferred, especially where the carrier peptide has only one or a few epitopes.

Carrier peptides used with the invention are preferably provided in purified or substantially purified form, *i.e.,* substantially free from other peptides, and are generally at least about 50% pure (by weight), and usually at least about 90% pure, *i.e*., less than about 50%, and more preferably less than about 10% (*e.g.,* 5%) of a composition is made up of other expressed peptides. For the avoidance of doubt, when a purified or substantially carrier peptide is conjugated to a saccharide is used as a component of a vaccine, the carrier peptide is still "purified" or "substantially purified" despite the presence of other protein antigens and cellular components (*e.g.,* other polysaccharides, outer membrane vesicles, *etc.*).

The term "peptide" refers to amino acid polymers of any length. The polymer may be linear or branched, it may comprise modified amino acids, and it may be interrupted by non-amino acids. The terms also encompass an amino acid polymer that has been modified naturally or by intervention; for example, disulfide bond formation, glycosylation, lipidation, acetylation, phosphorylation, or any other manipulation or modification, such as conjugation with a labeling component. Also included are, for example, peptides containing one or more analogs of an amino acid (including, for example, unnatural amino acids, *etc.),* as well as other modifications known in the art. Peptides can occur as single chains or associated chains.

Comparison of the immune response raised in a subject by the conjugate with the immune response raised by the saccharide along may be carried out use by any means available to one of skill in the art. One simple method as used in the examples below involves immunization of a model subject such as mouse and then challenge with a lethal dose of the pathogen of interest. For many compositions such as meningococcal conjugates, immunization of a mouse and then demonstration that the mouse sera comprises bactericidal antibodies is typically sufficient to demonstrate that the composition can induce a protective immune response. For proper comparison, one of skill in the art would naturally select the same adjuvant such as an aluminum adjuvant.

The invention provides a process for producing a carrier peptide of the invention, comprising the step of synthesizing at least part of the peptide by chemical means.

The invention also provides a process for producing a peptide of the invention, comprising the step of culturing a host cell transformed with nucleic acid of the invention under conditions which induce peptide expression. The peptide may then be purified, *e.g.,* from culture supernatants.

Although expression of the carrier peptides of the invention may take place in a homologous host, the invention will usually use a heterologous host for expression. The heterologous host may be prokaryotic (*e.g.,* a bacterium) or eukaryotic. Suitable hosts include, but are not limited to, *Bacillus subtilis, Vibrio cholerae, Salmonella typhi, Salmonella typhimurium, Neisseria lactamica*, *Neisseria cinerea*, *Mycobacteria* (*e.g. M. tuberculosis*), yeasts, *etc.*

### Immunogenic compositions and medicaments

Saccharide conjugates of the invention are useful as active ingredients (immunogens) in immunogenic compositions, and such compositions may be useful as vaccines. Vaccines according to the invention may either be prophylactic (*i.e.* to prevent infection) or therapeutic (*i.e.* to treat infection), but will typically be prophylactic.

Immunogenic compositions will be pharmaceutically acceptable. They will usually include components in addition to the antigens e.g. they typically include one or more pharmaceutical carrier(s), excipient(s) and/or adjuvant(s). A thorough discussion of carriers and excipients is available in ref. 96. Thorough discussions of vaccine adjuvants are available in refs. 1 and 2.

Compositions will generally be administered to a mammal in aqueous form. Prior to administration, however, the composition may have been in a non-aqueous form. For instance, although some vaccines are manufactured in aqueous form, then filled and distributed and administered also in aqueous form, other vaccines are lyophilized during manufacture and are reconstituted into an aqueous form at the time of use. Thus a composition of the invention may be dried, such as a lyophilized formulation.

The composition may include preservatives such as thiomersal or 2-phenoxyethanol. It is preferred, however, that the vaccine should be substantially free from (*i.e.* less than 5µg/ml) mercurial material *e.g.* thiomersal-free. Vaccines containing no mercury are more preferred. Preservative-free vaccines are particularly preferred.

To improve thermal stability, a composition may include a temperature protective agent.

To control tonicity, it is preferred to include a physiological salt, such as a sodium salt. Sodium chloride (NaCl) is preferred, which may be present at between 1 and 20 mg/ml e.g. about 10±2mg/ml NaCl. Other salts that may be present include potassium chloride, potassium dihydrogen phosphate, disodium phosphate dehydrate, magnesium chloride, calcium chloride, *etc.*

Compositions will generally have an osmolality of between 200 mOsm/kg and 400 mOsm/kg, preferably between 240-360 mOsm/kg, and will more preferably fall within the range of 290-310 mOsm/kg.

Compositions may include one or more buffers. Typical buffers include: a phosphate buffer; a Tris buffer; a borate buffer; a succinate buffer; a histidine buffer (particularly with an aluminum hydroxide adjuvant); or a citrate buffer. Buffers will typically be included in the 5-20mM range.

The pH of a composition will generally be between 5.0 and 8.1, and more typically between 6.0 and 8.0 *e.g.* 6.5 and 7.5, or between 7.0 and 7.8.

The composition is preferably sterile. The composition is preferably non-pyrogenic *e.g.* containing <1 EU (endotoxin unit, a standard measure) per dose, and preferably <0.1 EU per dose. The composition is preferably gluten free.

The composition may include material for a single immunization, or may include material for multiple immunizations (*i.e.* a 'multidose' kit). The inclusion of a preservative is preferred in multidose arrangements. As an alternative (or in addition) to including a preservative in multidose compositions, the compositions may be contained in a container having an aseptic adaptor for removal of material.

Human vaccines are typically administered in a dosage volume of about 0.5ml, although a half dose (*i.e.* about 0.25ml) may be administered to children.

Immunogenic compositions of the invention may also comprise one or more immunoregulatory agents. Preferably, one or more of the immunoregulatory agents include one or more adjuvants. The adjuvants may include a TH1 adjuvant and/or a TH2 adjuvant, further discussed below.

Adjuvants which may be used in compositions of the invention include, but are not limited to:

### A. Mineral-containing compositions

Mineral containing compositions suitable for use as adjuvants in the invention include mineral salts, such as aluminum salts and calcium salts (or mixtures thereof). Calcium salts include calcium phosphate (*e.g.* the "CAP" particles disclosed in ref. 3). Aluminum salts include hydroxides, phosphates, sulfates, *etc.,* with the salts taking any suitable form (*e.g.* gel, crystalline, amorphous, *etc*.). Adsorption to these salts is preferred. The mineral containing compositions may also be formulated as a particle of metal salt (4).

The adjuvants known as aluminum hydroxide and aluminum phosphate may be used. These names are conventional, but are used for convenience only, as neither is a precise description of the actual chemical compound which is present (*e.g.* see chapter 9 of reference 1). The invention can use any of the "hydroxide" or "phosphate" adjuvants that are in general use as adjuvants. The adjuvants known as "aluminum hydroxide" are typically aluminum oxyhydroxide salts, which are usually at least partially crystalline. The adjuvants known as "aluminum phosphate" are typically aluminum hydroxyphosphates, often also containing a small amount of sulfate (*i.e.* aluminum hydroxyphosphate sulfate). They may be obtained by precipitation, and the reaction conditions and concentrations during precipitation influence the degree of substitution of phosphate for hydroxyl in the salt.

A fibrous morphology (*e.g.* as seen in transmission electron micrographs) is typical for aluminum hydroxide adjuvants. The pI of aluminum hydroxide adjuvants is typically about 11 *i.e.* the adjuvant itself has a positive surface charge at physiological pH. Adsorptive capacities of between 1.8-2.6 mg protein per mg Al⁺⁺⁺ at pH 7.4 have been reported for aluminum hydroxide adjuvants.

Aluminum phosphate adjuvants generally have a PO₄/Al molar ratio between 0.3 and 1.2, preferably between 0.8 and 1.2, and more preferably 0.95±0.1. The aluminum phosphate will generally be amorphous, particularly for hydroxyphosphate salts. A typical adjuvant is amorphous aluminum hydroxyphosphate with PO₄/Al molar ratio between 0.84 and 0.92, included at 0.6mg Al³⁺/ml. The aluminum phosphate will generally be particulate (*e.g.* plate-like morphology as seen in transmission electron micrographs). Typical diameters of the particles are in the range 0.5-20µm (*e.g.* about 5-10µm) after any antigen adsorption. Adsorptive capacities of between 0.7-1.5 mg protein per mg Al⁺⁺⁺ at pH 7.4 have been reported for aluminum phosphate adjuvants.

The point of zero charge (PZC) of aluminum phosphate is inversely related to the degree of substitution of phosphate for hydroxyl, and this degree of substitution can vary depending on reaction conditions and concentration of reactants used for preparing the salt by precipitation. PZC is also altered by changing the concentration of free phosphate ions in solution (more phosphate = more acidic PZC) or by adding a buffer such as a histidine buffer (makes PZC more basic). Aluminum phosphates used according to the invention will generally have a PZC of between 4.0 and 7.0, more preferably between 5.0 and 6.5 *e.g.* about 5.7.

Suspensions of aluminum salts used to prepare compositions of the invention may contain a buffer (*e.g.* a phosphate or a histidine or a Tris buffer), but this is not always necessary. The suspensions are preferably sterile and pyrogen-free. A suspension may include free aqueous phosphate ions *e.g.* present at a concentration between 1.0 and 20 mM, preferably between 5 and 15 mM, and more preferably about 10 mM. The suspensions may also comprise sodium chloride.

The invention can use a mixture of both an aluminum hydroxide and an aluminum phosphate. In this case there may be more aluminum phosphate than hydroxide *e.g.* a weight ratio of at least 2:1 *e.g.* ≥5:1, ≥6:1, ≥7:1, ≥8:1, ≥9:1, *etc.*

The concentration of Al⁺⁺⁺ in a composition for administration to a patient is preferably less than 10mg/ml *e.g.* ≤5 mg/ml, ≤4 mg/ml, ≤3 mg/ml, ≤2 mg/ml, ≤1 mg/ml, *etc.* A preferred range is between 0.3 and 1mg/ml. A maximum of 0.85mg/dose is preferred.

### B. Oil Emulsions

Oil emulsion compositions suitable for use as adjuvants in the invention include squalene-water emulsions, such as MF59(TM) (Chapter 10 of ref. 1; see also ref. 5) (5% Squalene, 0.5% TWEEN 80(TM), and 0.5% SPAN 85(TM), formulated into submicron particles using a microfluidizer). Complete Freund's adjuvant (CFA) and incomplete Freund's adjuvant (IFA) may also be used.

Various oil-in-water emulsion adjuvants are known, and they typically include at least one oil and at least one surfactant, with the oil(s) and surfactant(s) being biodegradable (metabolisable) and biocompatible. The oil droplets in the emulsion are generally less than 5µm in diameter, and ideally have a sub-micron diameter, with these small sizes being achieved with a microfluidizer to provide stable emulsions. Droplets with a size less than 220nm are preferred as they can be subjected to filter sterilization.

The emulsion can comprise oils such as those from an animal (such as fish) or vegetable source. Sources for vegetable oils include nuts, seeds and grains. Peanut oil, soybean oil, coconut oil, and olive oil, the most commonly available, exemplify the nut oils. Jojoba oil can be used *e.g.* obtained from the jojoba bean. Seed oils include safflower oil, cottonseed oil, sunflower seed oil, sesame seed oil and the like. In the grain group, corn oil is the most readily available, but the oil of other cereal grains such as wheat, oats, rye, rice, teff, triticale and the like may also be used. 6-10 carbon fatty acid esters of glycerol and 1,2-propanediol, while not occurring naturally in seed oils, may be prepared by hydrolysis, separation and esterification of the appropriate materials starting from the nut and seed oils. Fats and oils from mammalian milk are metabolizable and may therefore be used in the practice of this invention. The procedures for separation, purification, saponification and other means necessary for obtaining pure oils from animal sources are well known in the art. Most fish contain metabolizable oils which may be readily recovered. For example, cod liver oil, shark liver oils, and whale oil such as spermaceti exemplify several of the fish oils which may be used herein. A number of branched chain oils are synthesized biochemically in 5-carbon isoprene units and are generally referred to as terpenoids. Shark liver oil contains a branched, unsaturated terpenoids known as squalene, 2,6,10,15,19,23-hexamethyl-2,6,10,14,18,22-tetracosahexaene, which is particularly preferred herein. Squalane, the saturated analog to squalene, is also a preferred oil. Fish oils, including squalene and squalane, are readily available from commercial sources or may be obtained by methods known in the art. Other preferred oils are the tocopherols (see below). Mixtures of oils can be used.

Surfactants can be classified by their 'HLB' (hydrophile/lipophile balance). Preferred surfactants of the invention have a HLB of at least 10, preferably at least 15, and more preferably at least 16. The invention can be used with surfactants including, but not limited to: the polyoxyethylene sorbitan esters surfactants (commonly referred to as the Tweens), especially polysorbate 20 and polysorbate 80; copolymers of ethylene oxide (EO), propylene oxide (PO), and/or butylene oxide (BO), sold under the DOWFAX™ tradename, such as linear EO/PO block copolymers; octoxynols, which can vary in the number of repeating ethoxy (oxy-1,2-ethanediyl) groups, with octoxynol-9 (Triton X-100, or t-octylphenoxypolyethoxyethanol) being of particular interest; (octylphenoxy)polyethoxyethanol (IGEPAL CA-630/NP-40); phospholipids such as phosphatidylcholine (lecithin); nonylphenol ethoxylates, such as the Tergitol™ NP series; polyoxyethylene fatty ethers derived from lauryl, cetyl, stearyl and oleyl alcohols (known as Brij surfactants), such as triethyleneglycol monolauryl ether (Brij 30); and sorbitan esters (commonly known as the SPANs), such as sorbitan trioleate (SPAN 85(TM)) and sorbitan monolaurate. Non-ionic surfactants are preferred. Preferred surfactants for including in the emulsion are TWEEN 80(TM) (polyoxyethylene sorbitan monooleate), SPAN 85(TM) (sorbitan trioleate), lecithin and Triton X-100.

Mixtures of surfactants can be used e.g. TWEEN 80(TM)/SPAN 85(TM) mixtures. A combination of a polyoxyethylene sorbitan ester such as polyoxyethylene sorbitan monooleate (TWEEN 80(TM)) and an octoxynol such as t-octylphenoxypolyethoxyethanol (Triton X-100) is also suitable. Another useful combination comprises laureth 9 plus a polyoxyethylene sorbitan ester and/or an octoxynol.

Preferred amounts of surfactants (% by weight) are: polyoxyethylene sorbitan esters (such as TWEEN 80(TM)) 0.01 to 1%, in particular about 0.1 %; octyl- or nonylphenoxy polyoxyethanols (such as Triton X-100, or other detergents in the Triton series) 0.001 to 0.1 %, in particular 0.005 to 0.02%; polyoxyethylene ethers (such as laureth 9) 0.1 to 20 %, preferably 0.1 to 10 % and in particular 0.1 to 1 % or about 0.5%.

Preferred emulsion adjuvants have an average droplets size of <1µm *e.g.* ≤750nm, ≤500nm, ≤400nm, ≤300nm, ≤250nm, ≤220nm, ≤200nm, or smaller. These droplet sizes can conveniently be achieved by techniques such as microfluidisation.

Specific oil-in-water emulsion adjuvants useful with the invention include, but are not limited to:
- A submicron emulsion of squalene, TWEEN 80(TM), and SPAN 85(TM). The composition of the emulsion by volume can be about 5% squalene, about 0.5% polysorbate 80 and about 0.5% SPAN 85(TM). In weight terms, these ratios become 4.3% squalene, 0.5% polysorbate 80 and 0.48% SPAN 85(TM). This adjuvant is known as 'MF59(TM)' (6-7), as described in more detail in Chapter 10 of ref. 8 and chapter 12 of ref. 9. The MF59(TM) emulsion advantageously includes citrate ions *e.g.* 10mM sodium citrate buffer.
- An emulsion of squalene, a tocopherol, and TWEEN 80(TM). The emulsion may include phosphate buffered saline. It may also include SPAN 85(TM) (*e.g.* at 1%) and/or lecithin. These emulsions may have from 2 to 10% squalene, from 2 to 10% tocopherol and from 0.3 to 3% TWEEN 80(TM), and the weight ratio of squalene:tocopherol is preferably ≤1 as this provides a more stable emulsion. Squalene and TWEEN 80(TM) may be present volume ratio of about 5:2. One such emulsion can be made by dissolving TWEEN 80(TM) in PBS to give a 2% solution, then mixing 90ml of this solution with a mixture of (5g of DL-α-tocopherol and 5ml squalene), then microfluidising the mixture. The resulting emulsion may have submicron oil droplets *e.g.* with an average diameter of between 100 and 250nm, preferably about 180nm.
- An emulsion of squalene, a tocopherol, and a Triton detergent (*e.g.* Triton X-100). The emulsion may also include a 3d-MPL (see below). The emulsion may contain a phosphate buffer.
- An emulsion comprising a polysorbate (*e.g.* polysorbate 80), a Triton detergent (*e.g.* Triton X-100) and a tocopherol (*e.g.* an α-tocopherol succinate). The emulsion may include these three components at a mass ratio of about 75:11:10 (*e.g.* 750µg/ml polysorbate 80, 110µg/ml Triton X-100 and 100µg/ml α-tocopherol succinate), and these concentrations should include any contribution of these components from antigens. The emulsion may also include squalene. The emulsion may also include a 3d-MPL (see below). The aqueous phase may contain a phosphate buffer.
- An emulsion of squalane, polysorbate 80 and poloxamer 401 ("PLURONIC(TM) L121"). The emulsion can be formulated in phosphate buffered saline, pH 7.4. This emulsion is a useful delivery vehicle for muramyl dipeptides, and has been used with threonyl-MDP in the "SAF-1" adjuvant (10) (0.05-1% Thr-MDP, 5% squalane, 2.5% PLURONIC(TM) L121 and 0.2% polysorbate 80). It can also be used without the Thr-MDP, as in the "AF" adjuvant (11) (5% squalane, 1.25% PLURONIC(TM) L121 and 0.2% polysorbate 80). Microfluidisation is preferred.
- An emulsion comprising squalene, an aqueous solvent, a polyoxyethylene alkyl ether hydrophilic nonionic surfactant (*e.g.* polyoxyethylene (12) cetostearyl ether) and a hydrophobic nonionic surfactant (*e.g.* a sorbitan ester or mannide ester, such as sorbitan monoleate or 'SPAN 80(TM)'). The emulsion is preferably thermoreversible and/or has at least 90% of the oil droplets (by volume) with a size less than 200 nm (12). The emulsion may also include one or more of: alditol; a cryoprotective agent (*e.g.* a sugar, such as dodecylmaltoside and/or sucrose); and/or an alkylpolyglycoside. Such emulsions may be lyophilized.
- An emulsion of squalene, poloxamer 105 and Abil-Care (13). The final concentration (weight) of these components in adjuvanted vaccines are 5% squalene, 4% poloxamer 105 (pluronic polyol) and 2% Abil-Care 85 (Bis-PEG/PPG-16/16 PEG/PPG-16/16 dimethicone; caprylic/capric triglyceride).
- An emulsion having from 0.5-50% of an oil, 0.1-10% of a phospholipid, and 0.05-5% of a non-ionic surfactant. As described in reference 14, preferred phospholipid components are phosphatidylcholine, phosphatidylethanolamine, phosphatidylserine, phosphatidylinositol, phosphatidylglycerol, phosphatidic acid, sphingomyelin and cardiolipin. Submicron droplet sizes are advantageous.
- A submicron oil-in-water emulsion of a non-metabolisable oil (such as light mineral oil) and at least one surfactant (such as lecithin, TWEEN 80(TM) or SPAN 80(TM)). Additives may be included, such as QuilA saponin, cholesterol, a saponin-lipophile conjugate (such as GPI-0100, described in reference 15, produced by addition of aliphatic amine to desacylsaponin via the carboxyl group of glucuronic acid), dimethyidioctadecylammonium bromide and/or N,N-dioctadecyl-N,N-bis (2-hydroxyethyl)propanediamine.
- An emulsion in which a saponin (*e.g.* QuilA or QS21) and a sterol (*e.g.* a cholesterol) are associated as helical micelles (16).
- An emulsion comprising a mineral oil, a non-ionic lipophilic ethoxylated fatty alcohol, and a non-ionic hydrophilic surfactant (*e.g.* an ethoxylated fatty alcohol and/or polyoxyethylene-polyoxypropylene block copolymer) (17).
- An emulsion comprising a mineral oil, a non-ionic hydrophilic ethoxylated fatty alcohol, and a non-ionic lipophilic surfactant (*e.g.* an ethoxylated fatty alcohol and/or polyoxyethylene-polyoxypropylene block copolymer) (17).

In some embodiments an emulsion may be mixed with antigen extemporaneously, at the time of delivery, and thus the adjuvant and antigen may be kept separately in a packaged or distributed vaccine, ready for final formulation at the time of use. In other embodiments an emulsion is mixed with antigen during manufacture, and thus the composition is packaged in a liquid adjuvanted form,. The antigen will generally be in an aqueous form, such that the vaccine is finally prepared by mixing two liquids. The volume ratio of the two liquids for mixing can vary (*e.g.* between 5:1 and 1:5) but is generally about 1:1. Where concentrations of components are given in the above descriptions of specific emulsions, these concentrations are typically for an undiluted composition, and the concentration after mixing with an antigen solution will thus decrease.

Where a composition includes a tocopherol, any of the α, β, γ, δ, ε or ξ tocopherols can be used, but α-tocopherols are preferred. The tocopherol can take several forms *e.g.* different salts and/or isomers. Salts include organic salts, such as succinate, acetate, nicotinate, *etc.* D-α-tocopherol and DL-α-tocopherol can both be used. Tocopherols are advantageously included in vaccines for use in elderly patients (*e.g.* aged 60 years or older) because vitamin E has been reported to have a positive effect on the immune response in this patient group (18). They also have antioxidant properties that may help to stabilize the emulsions (19). A preferred α-tocopherol is DL-α-tocopherol, and the preferred salt of this tocopherol is the succinate. The succinate salt has been found to cooperate with TNF-related ligands *in vivo.*

### C. Saponin formulations (chapter 22 of ref. 1)

Saponin formulations may also be used as adjuvants in the invention. Saponins are a heterogeneous group of sterol glycosides and triterpenoid glycosides that are found in the bark, leaves, stems, roots and even flowers of a wide range of plant species. Saponin from the bark of the *Quillaia saponaria* Molina tree have been widely studied as adjuvants. Saponin can also be commercially obtained from *Smilax ornata* (sarsaprilla), *Gypsophilla paniculata* (brides veil), and *Saponaria officianalis* (soap root). Saponin adjuvant formulations include purified formulations, such as QS21, as well as lipid formulations, such as ISCOMs. QS21 is marketed as Stimulon™.

Saponin compositions have been purified using HPLC and RP-HPLC. Specific purified fractions using these techniques have been identified, including QS7, QS17, QS18, QS21, QH-A, QH-B and QH-C. Preferably, the saponin is QS21. A method of production of QS21 is disclosed in ref. 20. Saponin formulations may also comprise a sterol, such as cholesterol (21).

Combinations of saponins and cholesterols can be used to form unique particles called immunostimulating complexs (ISCOMs) (chapter 23 of ref. 1). ISCOMs typically also include a phospholipid such as phosphatidylethanolamine or phosphatidylcholine. Any known saponin can be used in ISCOMs. Preferably, the ISCOM includes one or more of QuilA, QHA & QHC. ISCOMs are further described in refs. 21-22. Optionally, the ISCOMS may be devoid of additional detergent (23).

A review of the development of saponin based adjuvants can be found in refs. 24 & 25.

### D. Virosomes and virus-like particles

Virosomes and virus-like particles (VLPs) can also be used as adjuvants in the invention. These structures generally contain one or more proteins from a virus optionally combined or formulated with a phospholipid. They are generally non-pathogenic, non-replicating and generally do not contain any of the native viral genome. The viral proteins may be recombinantly produced or isolated from whole viruses. These viral proteins suitable for use in virosomes or VLPs include proteins derived from influenza virus (such as HA or NA), Hepatitis B virus (such as core or capsid proteins), Hepatitis E virus, measles virus, Sindbis virus, Rotavirus, Foot-and-Mouth Disease virus, Retrovirus, Norwalk virus, human Papilloma virus, HIV, RNA-phages, Qβ-phage (such as coat proteins), GA-phage, fr-phage, AP205 phage, and Ty (such as retrotransposon Ty protein p1). VLPs are discussed further in refs. 26-27. Virosomes are discussed further in, for example, ref. 28

### E. Bacterial or microbial derivatives

Adjuvants suitable for use in the invention include bacterial or microbial derivatives such as non-toxic derivatives of enterobacterial lipopolysaccharide (LPS), Lipid A derivatives, immunostimulatory oligonucleotides and ADP-ribosylating toxins and detoxified derivatives thereof.

Non-toxic derivatives of LPS include monophosphoryl lipid A (MPL) and 3-O-deacylated MPL (3dMPL). 3dMPL is a mixture of 3 de-O-acylated monophosphoryl lipid A with 4, 5 or 6 acylated chains. A preferred "small particle" form of 3 De-O-acylated monophosphoryl lipid A is disclosed in ref. 29. Such "small particles" of 3dMPL are small enough to be sterile filtered through a 0.22µm membrane (29). Other non-toxic LPS derivatives include monophosphoryl lipid A mimics, such as aminoalkyl glucosaminide phosphate derivatives *e.g.* RC-529 (30,31).

Lipid A derivatives include derivatives of lipid A from *Escherichia coli* such as OM-174. OM-174 is described for example in refs. 32 & 33.

Immunostimulatory oligonucleotides suitable for use as adjuvants in the invention include nucleotide sequences containing a CpG motif (a dinucleotide sequence containing an unmethylated cytosine linked by a phosphate bond to a guanosine). Double-stranded RNAs and oligonucleotides containing palindromic or poly(dG) sequences have also been shown to be immunostimulatory.

The CpG's can include nucleotide modifications/analogs such as phosphorothioate modifications and can be double-stranded or single-stranded. References 34, 35 and 36 disclose possible analog substitutions *e.g.* replacement of guanosine with 2'-deoxy-7-deazaguanosine. The adjuvant effect of CpG oligonucleotides is further discussed in refs. 37-38.

The CpG sequence may be directed to TLR9, such as the motif GTCGTT or TTCGTT (39). The CpG sequence may be specific for inducing a Th1 immune response, such as a CpG-A ODN, or it may be more specific for inducing a B cell response, such a CpG-B ODN. CpG-A and CpG-B ODNs are discussed in refs. 40-41. Preferably, the CpG is a CpG-A ODN.

Preferably, the CpG oligonucleotide is constructed so that the 5' end is accessible for receptor recognition. Optionally, two CpG oligonucleotide sequences may be attached at their 3' ends to form "immunomers". See, for example, refs. 39 & 42-43.

A useful CpG adjuvant is CpG7909, also known as ProMune™ (Coley Pharmaceutical Group, Inc.). Another is CpG1826. As an alternative, or in addition, to using CpG sequences, TpG sequences can be used (44), and these oligonucleotides may be free from unmethylated CpG motifs. The immunostimulatory oligonucleotide may be pyrimidine-rich. For example, it may comprise more than one consecutive thymidine nucleotide (*e.g.* TTTT, as disclosed in ref. 44), and/or it may have a nucleotide composition with >25% thymidine (*e.g.* >35%, >40%, >50%, >60%, >80%, *etc*.). For example, it may comprise more than one consecutive cytosine nucleotide (*e.g.* CCCC, as disclosed in ref. 44), and/or it may have a nucleotide composition with >25% cytosine (*e.g.* >35%, >40%, >50%, >60%, >80%, *etc*.). These oligonucleotides may be free from unmethylated CpG motifs. Immunostimulatory oligonucleotides will typically comprise at least 20 nucleotides. They may comprise fewer than 100 nucleotides.

A particularly useful adjuvant based around immunostimulatory oligonucleotides is known as IC-31™ (45). Thus an adjuvant used with the invention may comprise a mixture of (i) an oligonucleotide *(e.g.* between 15-40 nucleotides) including at least one (and preferably multiple) CpI motifs (*i.e.* a cytosine linked to an inosine to form a dinucleotide), and (ii) a polycationic polymer, such as an oligopeptide (*e.g.* between 5-20 amino acids) including at least one (and preferably multiple) Lys-Arg-Lys tripeptide sequence(s). The oligonucleotide may be a deoxynucleotide comprising 26-mer sequence 5'-(IC)₁₃-3' (SEQ ID NO: 51). The polycationic polymer may be a peptide comprising 11-mer amino acid sequence KLKLLLLLKLK (SEQ ID NO: 52).

Bacterial ADP-ribosylating toxins and detoxified derivatives thereof may be used as adjuvants in the invention. Preferably, the protein is derived from *E. coli* (*E. coli* heat labile enterotoxin "LT"), cholera ("CT"), or pertussis ("PT"). The use of detoxified ADP-ribosylating toxins as mucosal adjuvants is described in ref. 46 and as parenteral adjuvants in ref. 47. The toxin or toxoid is preferably in the form of a holotoxin, comprising both A and B subunits. Preferably, the A subunit contains a detoxifying mutation; preferably the B subunit is not mutated. Preferably, the adjuvant is a detoxified LT mutant such as LT-K63, LT-R72, and LT-G192. The use of ADP-ribosylating toxins and detoxified derivatives thereof, particularly LT-K63 and LT-R72, as adjuvants can be found in refs. 48-49. A useful CT mutant is or CT-E29H (50). Numerical reference for amino acid substitutions is preferably based on the alignments of the A and B subunits of ADP-ribosylating toxins set forth in ref. 51, specifically referred to solely for the purpose of the alignment and amino acid numbering therein.

### F. Human immunomodulators

Human immunomodulators suitable for use as adjuvants in the invention include cytokines, such as interleukins (*e.g.* IL-1, IL-2, IL-4, IL-5, IL-6, IL-7, IL-12 (52), *etc.*) (53), interferons (*e.g.* interferon-γ), macrophage colony stimulating factor, and tumor necrosis factor. A preferred immunomodulator is IL-12.

### G. Bioadhesives and Mucoadhesives

Bioadhesives and mucoadhesives may also be used as adjuvants in the invention. Suitable bioadhesives include esterified hyaluronic acid microspheres (54) or mucoadhesives such as cross-linked derivatives of poly(acrylic acid), polyvinyl alcohol, polyvinyl pyrollidone, polysaccharides and carboxymethylcellulose. Chitosan and derivatives thereof may also be used as adjuvants in the invention (55).

### H. Microparticles

Microparticles may also be used as adjuvants in the invention. Microparticles (*i.e.* a particle of ∼100nm to ∼150µm in diameter, more preferably ∼200nm to ∼30µm in diameter, and most preferably ∼500nm to ∼10µm in diameter) formed from materials that are biodegradable and non-toxic (*e.g.* a poly(α-hydroxy acid), a polyhydroxybutyric acid, a polyorthoester, a polyanhydride, a polycaprolactone, *etc*.), with poly(lactide-co-glycolide) are preferred, optionally treated to have a negatively-charged surface (*e.g.* with SDS) or a positively-charged surface (*e.g.* with a cationic detergent, such as CTAB).

### I. Liposomes (Chapters 13 & 14 of ref. 1)

Examples of liposome formulations suitable for use as adjuvants are described in refs. 56-57.

### J. Polyoxyethylene ether and polyoxyethylene ester formulations

Adjuvants suitable for use in the invention include polyoxyethylene ethers and polyoxyethylene esters (58). Such formulations further include polyoxyethylene sorbitan ester surfactants in combination with an octoxynol (59) as well as polyoxyethylene alkyl ethers or ester surfactants in combination with at least one additional non-ionic surfactant such as an octoxynol (60). Preferred polyoxyethylene ethers are selected from the following group: polyoxyethylene-9-lauryl ether (laureth 9), polyoxyethylene-9-steoryl ether, polyoxytheylene-8-steoryl ether, polyoxyethylene-4-lauryl ether, polyoxyethylene-35-lauryl ether, and polyoxyethylene-23-lauryl ether.

### K. Phosphazenes

A phosphazene, such as poly(di(carboxylatophenoxy)phosphazene) ("PCPP") as described, for example, in references 61 and 62, may be used.

### L. Muramyl peptides

Examples of muramyl peptides suitable for use as adjuvants in the invention include N-acetyl-muramyl-L-threonyl-D-isoglutamine (thr-MDP), N-acetyl-normuramyl-L-alanyl-D-isoglutamine (nor-MDP), and N-acetylmuramyl-L-alanyl-D-isoglutaminyl-L-alanine-2-(1'-2'-dipalmitoyl-*sn-*glycero-3-hydroxyphosphoryloxy)-ethylamine MTP-PE).

### M. Imidazoquinolone Compounds.

Examples of imidazoquinolone compounds suitable for use adjuvants in the invention include Imiquimod ("R-837") (63,64), Resiquimod ("R-848") (65), and their analogs; and salts thereof (*e.g.* the hydrochloride salts). Further details about immunostimulatory imidazoquinolines can be found in references 66 to67.

### N. Substituted ureas

Substituted ureas useful as adjuvants include compounds of formula I, II or III, or salts thereof: 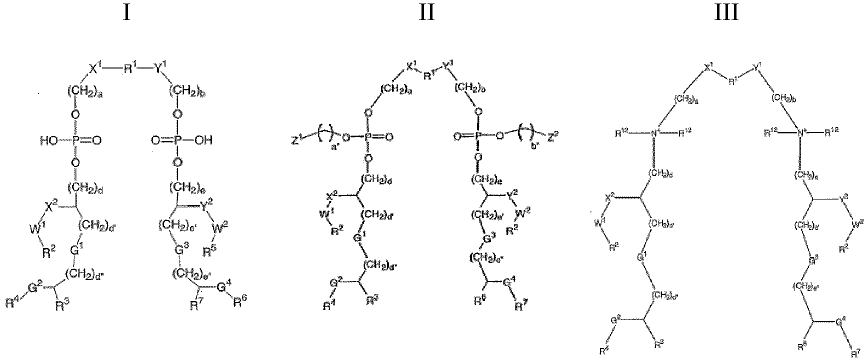 as defined in reference 68, such as 'ER 803058', 'ER 803732', 'ER 804053', ER 804058', 'ER 804059', 'ER 804442', 'ER 804680', 'ER 804764', ER 803022 or 'ER 804057' e.g.:

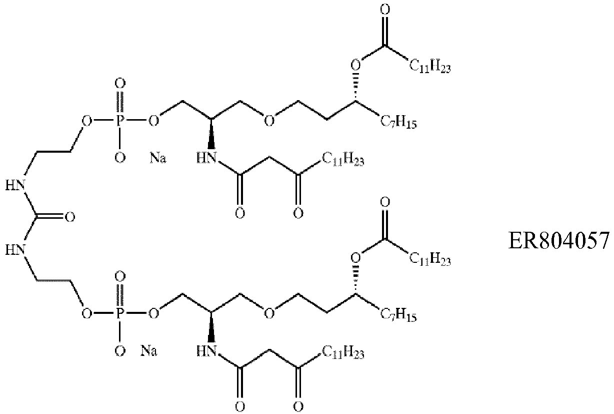

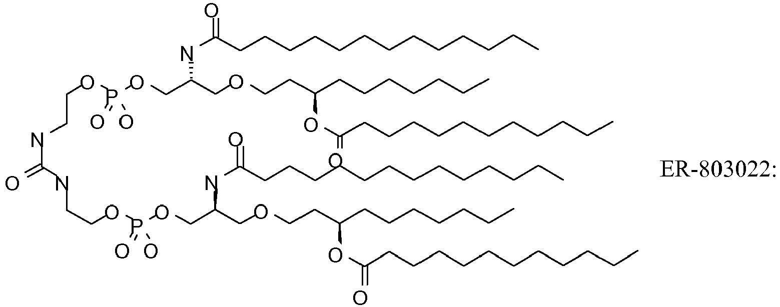

### O. Further adjuvants

Further adjuvants that may be used with the invention include:
- An aminoalkyl glucosaminide phosphate derivative, such as RC-529 (69,70).
- A thiosemicarbazone compound, such as those disclosed in reference 71. Methods of formulating, manufacturing, and screening for active compounds are also described in reference 71. The thiosemicarbazones are particularly effective in the stimulation of human peripheral blood mononuclear cells for the production of cytokines, such as TNF-α.
- A tryptanthrin compound, such as those disclosed in reference 72. Methods of formulating, manufacturing, and screening for active compounds are also described in reference 72. The thiosemicarbazones are particularly effective in the stimulation of human peripheral blood mononuclear cells for the production of cytokines, such as TNF-α.
- A nucleoside analog, such as: (a) Isatorabine (ANA-245; 7-thia-8-oxoguanosine): 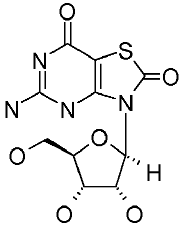 and prodrugs thereof; (b) ANA975; (c) ANA-025-1; (d) ANA380; (e) the compounds disclosed in references 73 to74Loxoribine (7-allyl-8-oxoguanosine) (75).
- Compounds disclosed in reference 76, including: Acylpiperazine compounds, Indoledione compounds, Tetrahydraisoquinoline (THIQ) compounds, Benzocyclodione compounds, Aminoazavinyl compounds, Aminobenzimidazole quinolinone (ABIQ) compounds (77,78), Hydrapthalamide compounds, Benzophenone compounds, Isoxazole compounds, Sterol compounds, Quinazilinone compounds, Pyrrole compounds (79), Anthraquinone compounds, Quinoxaline compounds, Triazine compounds, Pyrazalopyrimidine compounds, and Benzazole compounds (80).
- Compounds containing lipids linked to a phosphate-containing acyclic backbone, such as the TLR4 antagonist E5564 (81,82):
- A polyoxidonium polymer (83,84) or other N-oxidized polyethylene-piperazine derivative.
- Methyl inosine 5'-monophosphate ("MIMP") (85).
- A polyhydroxlated pyrrolizidine compound (86), such as one having formula: 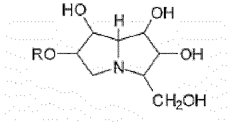 where R is selected from the group comprising hydrogen, straight or branched, unsubstituted or substituted, saturated or unsaturated acyl, alkyl (*e.g.* cycloalkyl), alkenyl, alkynyl and aryl groups, or a pharmaceutically acceptable salt or derivative thereof. Examples include, but are not limited to: casuarine, casuarine-6-α-D-glucopyranose, 3-*epi*-casuarine, 7-*epi*-casuarine, 3,7-di*epi*-casuarine, *etc.*
- A CD1d ligand, such as an α-glycosylceramide (87-88) (*e.g.* α-galactosylceramide), phytosphingosine-containing α-glycosylceramides, OCH, KRN7000 ((2S,3S,4R)-1-O-(α-D-galactopyranosyl)-2-(N-hexacosanoylamino)-1,3,4-octadecanetriol), CRONY-101, 3"-O-sulfo-galactosylceramide, *etc.*
- A gamma inulin (89) or derivative thereof, such as algammulin.

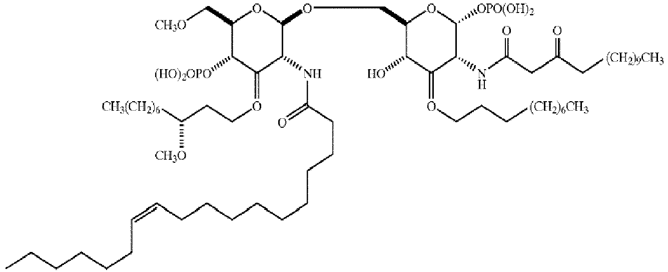

### Adjuvant combinations

The invention may also comprise combinations of aspects of one or more of the adjuvants identified above. For example, the following adjuvant compositions may be used in the invention: (1) a saponin and an oil-in-water emulsion (90); (2) a saponin (*e.g.* QS21) + a non-toxic LPS derivative (*e.g.* 3dMPL) (91); (3) a saponin (*e.g.* QS21) + a non-toxic LPS derivative (*e.g.* 3dMPL) + a cholesterol; (4) a saponin (*e.g.* QS21) + 3dMPL + IL-12 (optionally + a sterol) (92); (5) combinations of 3dMPL with, for example, QS21 and/or oil-in-water emulsions (93); (6) SAF, containing 10% squalane, 0.4% TWEEN 80™, 5% PLURONIC(TM)-block polymer L121, and thr-MDP, either microfluidized into a submicron emulsion or vortexed to generate a larger particle size emulsion. (7) Ribi™ adjuvant system (RAS), (Ribi Immunochem) containing 2% squalene, 0.2% TWEEN 80(TM), and one or more bacterial cell wall components from the group consisting of monophosphorylipid A (MPL), trehalose dimycolate (TDM), and cell wall skeleton (CWS), preferably MPL + CWS (Detox™); and (8) one or more mineral salts (such as an aluminum salt) + a non-toxic derivative of LPS (such as 3dMPL).

Other substances that act as immunostimulating agents are disclosed in chapter 7 of ref. 1.

The use of an aluminum hydroxide and/or aluminum phosphate adjuvant is particularly preferred, and antigens are generally adsorbed to these salts. Calcium phosphate is another preferred adjuvant. Other preferred adjuvant combinations include combinations of Th1 and Th2 adjuvants such as CpG & alum or resiquimod & alum. A combination of aluminum phosphate and 3dMPL may be used.

The compositions of the invention may elicit both a cell mediated immune response as well as a humoral immune response. This immune response will preferably induce long lasting (*e.g.* neutralizing) antibodies and a cell mediated immunity that can quickly respond upon exposure to the pathogen immunized against.

Two types of T cells, CD4 and CD8 cells, are generally thought necessary to initiate and/or enhance cell mediated immunity and humoral immunity. CD8 T cells can express a CD8 co-receptor and are commonly referred to as Cytotoxic T lymphocytes (CTLs). CD8 T cells are able to recognized or interact with antigens displayed on MHC Class I molecules.

CD4 T cells can express a CD4 co-receptor and are commonly referred to as T helper cells. CD4 T cells are able to recognize antigenic peptides bound to MHC class II molecules. Upon interaction with a MHC class II molecule, the CD4 cells can secrete factors such as cytokines. These secreted cytokines can activate B cells, cytotoxic T cells, macrophages, and other cells that participate in an immune response. Helper T cells or CD4+ cells can be further divided into two functionally distinct subsets: TH1 phenotype and TH2 phenotypes which differ in their cytokine and effector function.

Activated TH1 cells enhance cellular immunity (including an increase in antigen-specific CTL production) and are therefore of particular value in responding to intracellular infections. Activated TH1 cells may secrete one or more of IL-2, IFN-γ, and TNF-β. A TH1 immune response may result in local inflammatory reactions by activating macrophages, NK (natural killer) cells, and CD8 cytotoxic T cells (CTLs). A TH1 immune response may also act to expand the immune response by stimulating growth of B and T cells with IL-12. TH1 stimulated B cells may secrete IgG2a.

Activated TH2 cells enhance antibody production and are therefore of value in responding to extracellular infections. Activated TH2 cells may secrete one or more of IL-4, IL-5, IL-6, and IL-10. A TH2 immune response may result in the production of IgG1, IgE, IgA and memory B cells for future protection.

An enhanced immune response may include one or more of an enhanced TH1 immune response and a TH2 immune response.

A TH1 immune response may include one or more of an increase in CTLs, an increase in one or more of the cytokines associated with a TH1 immune response (such as IL-2, IFN-γ, and TNF-β), an increase in activated macrophages, an increase in NK activity, or an increase in the production of IgG2a. Preferably, the enhanced TH1 immune response will include an increase in IgG2a production.

A TH1 immune response may be elicited using a TH1 adjuvant. A TH1 adjuvant will generally elicit increased levels of IgG2a production relative to immunization of the antigen without adjuvant. TH1 adjuvants suitable for use in the invention may include for example saponin formulations, virosomes and virus like particles, non-toxic derivatives of enterobacterial lipopolysaccharide (LPS), immunostimulatory oligonucleotides. Immunostimulatory oligonucleotides, such as oligonucleotides containing a CpG motif, are preferred TH1 adjuvants for use in the invention.

A TH2 immune response may include one or more of an increase in one or more of the cytokines associated with a TH2 immune response (such as IL-4, IL-5, IL-6 and IL-10), or an increase in the production of IgG1, IgE, IgA and memory B cells. Preferably, the enhanced TH2 immune response will include an increase in IgG1 production.

A TH2 immune response may be elicited using a TH2 adjuvant. A TH2 adjuvant will generally elicit increased levels of IgG1 production relative to immunization of the antigen without adjuvant. TH2 adjuvants suitable for use in the invention include, for example, mineral containing compositions, oil-emulsions, and ADP-ribosylating toxins and detoxified derivatives thereof. Mineral containing compositions, such as aluminum salts are preferred TH2 adjuvants for use in the invention.

Preferably, the invention includes a composition comprising a combination of a TH1 adjuvant and a TH2 adjuvant. Preferably, such a composition elicits an enhanced TH1 and an enhanced TH2 response, i.e., an increase in the production of both IgG1 and IgG2a production relative to immunization without an adjuvant. Still more preferably, the composition comprising a combination of a TH1 and a TH2 adjuvant elicits an increased TH1 and/or an increased TH2 immune response relative to immunization with a single adjuvant (*i.e.,* relative to immunization with a TH1 adjuvant alone or immunization with a TH2 adjuvant alone).

The immune response may be one or both of a TH1 immune response and a TH2 response. Preferably, immune response provides for one or both of an enhanced TH1 response and an enhanced TH2 response.

The enhanced immune response may be one or both of a systemic and a mucosal immune response. Preferably, the immune response provides for one or both of an enhanced systemic and an enhanced mucosal immune response. Preferably the mucosal immune response is a TH2 immune response. Preferably, the mucosal immune response includes an increase in the production of IgA.

### Pharmaceutical compositions

One aspect of the invention includes pharmaceutical compositions comprising (a) a saccharide conjugate as disclosed herein, (b) a pharmaceutically acceptable carrier, and optionally (c) an adjuvant as described in the preceding section.

The compositions of the invention may be prepared in various forms. For example, the compositions may be prepared as injectables, either as liquid solutions or suspensions. Solid forms suitable for solution in, or suspension in, liquid vehicles prior to injection can also be prepared (*e.g.* a lyophilized composition or a spray-freeze dried composition). The composition may be prepared for topical administration *e.g.* as an ointment, cream or powder. The composition may be prepared for oral administration *e.g.* as a tablet or capsule, as a spray, or as a syrup (optionally flavored). The composition may be prepared for pulmonary administration *e.g.* as an inhaler, using a fine powder or a spray. The composition may be prepared as a suppository or pessary. The composition may be prepared for nasal, aural or ocular administration *e.g.* as drops. The composition may be in kit form, designed such that a combined composition is reconstituted just prior to administration to a patient. Such kits may comprise one or more antigens in liquid form and one or more lyophilized antigens.

Where a composition is to be prepared extemporaneously prior to use (*e.g.* where a component is presented in lyophilized form) and is presented as a kit, the kit may comprise two vials, or it may comprise one ready-filled syringe and one vial, with the contents of the syringe being used to reactivate the contents of the vial prior to injection.

Immunogenic compositions used as vaccines comprise an immunologically effective amount of antigen(s) (*e.g.,* the polysaccharide and/or the carrier protein), as well as any other components, as needed. By 'immunologically effective amount', it is meant that the administration of that amount to an individual, either in a single dose or as part of a series, is effective for treatment or prevention. This amount varies depending upon the health and physical condition of the individual to be treated, age, the taxonomic group of individual to be treated (*e.g.* non-human primate, primate, *etc*.), the capacity of the individual's immune system to synthesize antibodies, the degree of protection desired, the formulation of the vaccine, the treating doctor's assessment of the medical situation, and other relevant factors. It is expected that the amount will fall in a relatively broad range that can be determined through routine trials.

### Methods of treatment, and administration of the vaccine

The invention also provides a method for raising an immune response in a mammal comprising the step of administering an effective amount of a composition of the invention. The immune response is preferably protective and preferably involves antibodies and/or cell-mediated immunity. The method may raise a booster response.

The invention also provides a peptide of the invention for use as a medicament *e.g.* for use in raising an immune response in a mammal.

The invention also provides the use of a peptide of the invention in the manufacture of a medicament for raising an immune response in a mammal.

The invention also provides a delivery device pre-filled with an immunogenic composition of the invention.

Because glucans (and β-glucans in particular) are an essential and principal polysaccharide constituent of almost all pathogenic fungi, particularly those involved in infections in immunocompromised subjects, and also in bacterial pathogens and protozoa, anti-glucan immunity may have efficacy against a broad range of pathogens and diseases. For example, anti-glucan serum raised after immunization with *S. cerevisiae* is cross-reactive with *C. albicans*. Broad spectrum immunity is particularly useful because, for these human infectious fungal agents, chemotherapy is scanty, antifungal drug resistance is emerging and the need for preventative and therapeutic vaccines is increasingly recognized.

Therefore, where the polysaccharide immunogen of the polysaccharide conjugates disclosed herein are glucans, the uses and methods of the glucan polysaccharide conjugates disclosed herein are particularly useful for treating/protecting against infections of: Candida species, such as *C. albicans*; Cryptococcus species, such as *C. neoformans*; Enterococcus species, such as *E. faecalis;* Streptococcus species, such as *S. pneumoniae, S. mutans, S. agalactiae* and *S. pyogenes*; Leishmania species, such as *L. major*; Acanthamoeba species, such as *A. castellani*; Aspergillus species, such as *A. fumigatus* and *A. flavus*; Pneumocystis species, such as *P. carinii*; Mycobacterium species, such as *M. tuberculosis*; Pseudomonas species, such as *P. aeruginosa*; Staphylococcus species, such as *S. aureus*; Salmonella species, such as *S. typhimurium*; Coccidioides species such as *C. immitis*; Trichophyton species such as *T. verrucosum*; Blastomyces species such as *B. dermatidis*; Histoplasma species such as *H. capsulatum*; Paracoccidioides species such as *P. brasiliensis*; Pythium species such as *P. insidiosum*; and Escherichia species, such as *E. coli.*

The uses and methods are particularly useful for preventing/treating diseases including, but not limited to: candidiasis (including hepatosplenic candidiasis, invasive candidiasis, chronic mucocutaneous candidiasis and disseminated candidiasis); candidemia; aspergillosis, cryptococcosis, dermatomycoses, sporothrychosis and other subcutaneous mycoses, blastomycosis, histoplasmosis, coccidiomycosis, paracoccidiomycosis, pneumocystosis, thrush, tuberculosis, mycobacteriosis, respiratory infections, scarlet fever, pneumonia, impetigo, rheumatic fever, sepsis, septicaemia, cutaneous and visceral leishmaniasis, corneal acanthamoebiasis, cystic fibrosis, typhoid fever, gastroenteritis and hemolytic- uremic syndrome. Anti-*C. albicans* activity is particularly useful for treating infections in AIDS patients.

Efficacy of immunization can be tested by monitoring immune responses against β-glucan (*e.g.,* anti-β-glucan antibodies) after administration of the composition. Efficacy of therapeutic treatment can be tested by monitoring microbial infection after administration of the composition of the invention.

The mammal is preferably a human, but may be, *e.g.,* a cow, a pig, a cat or a dog, as *E. coli* disease is also problematic in these species. While the specification refers to mammals and mammalian subjects, the polysaccharide conjugates disclosed herein are also useful for avian species such as chicken and duck and therefore wherever mammal or mammalian is recited herein, avian can also be included. Where the vaccine is for prophylactic use, the human is preferably a child (*e.g.* a toddler or infant) or a teenager; where the vaccine is for therapeutic use, the human is preferably a teenager or an adult. A vaccine intended for children may also be administered to adults *e.g.* to assess safety, dosage, immunogenicity, *etc.*

One way of checking efficacy of therapeutic treatment involves monitoring *E. coli* infection after administration of the compositions of the invention. One way of checking efficacy of prophylactic treatment involves monitoring immune responses, systemically (such as monitoring the level of IgG1 and IgG2a production) and/or mucosally (such as monitoring the level of IgA production), against the antigens in the compositions of the invention after administration of the composition. Typically, antigen-specific serum antibody responses are determined post-immunization but pre-challenge whereas antigen-specific mucosal antibody responses are determined post-immunization and post-challenge.

Another way of assessing the immunogenicity of the compositions of the present invention is to express the proteins recombinantly for screening patient sera or mucosal secretions by immunoblot and/or microarrays. A positive reaction between the protein and the patient sample indicates that the patient has mounted an immune response to the protein in question. This method may also be used to identify immunodominant antigens and/or epitopes within antigens.

The efficacy of vaccine compositions can also be determined *in vivo* by challenging animal models of *E. coli* infection, *e.g.,* guinea pigs or mice, with the vaccine compositions. A murine model of ExPEC and lethal sepsis is described in reference 94. A cotton rat model is disclosed in ref. 95.

Compositions of the invention will generally be administered directly to a patient. Direct delivery may be accomplished by parenteral injection (*e.g.* subcutaneously, intraperitoneally, intravenously, intramuscularly, or to the interstitial space of a tissue), or mucosally, such as by rectal, oral (*e.g.* tablet, spray), vaginal, topical, transdermal or transcutaneous, intranasal, ocular, aural, pulmonary or other mucosal administration. Novel direct delivery forms can also include transgenic expression of the peptides disclosed herein in foods, e.g., transgenic expression in a potato.

The invention may be used to elicit systemic and/or mucosal immunity, preferably to elicit an enhanced systemic and/or mucosal immunity.

Preferably the enhanced systemic and/or mucosal immunity is reflected in an enhanced TH1 and/or TH2 immune response. Preferably, the enhanced immune response includes an increase in the production of IgG1 and/or IgG2a and/or IgA.

Dosage can be by a single dose schedule or a multiple dose schedule. Multiple doses may be used in a primary immunization schedule and/or in a booster immunization schedule. In a multiple dose schedule the various doses may be given by the same or different routes *e.g.* a parenteral prime and mucosal boost, a mucosal prime and parenteral boost, *etc.* Multiple doses will typically be administered at least 1 week apart (*e.g.* about 2 weeks, about 3 weeks, about 4 weeks, about 6 weeks, about 8 weeks, about 10 weeks, about 12 weeks, about 16 weeks, *etc*.).

Vaccines of the invention may be used to treat both children and adults. Thus a human patient may be less than 1 year old, 1-5 years old, 5-15 years old, 15-55 years old, or at least 55 years old. Preferred patients for receiving the vaccines are the elderly (*e.g.* ≥50 years old, ≥60 years old, and preferably ≥65 years), the young (*e.g.* ≤5 years old), hospitalized patients, healthcare workers, armed service and military personnel, pregnant women, the chronically ill, or immunodeficient patients. The vaccines are not suitable solely for these groups, however, and may be used more generally in a population.

Vaccines of the invention are particularly useful for patients who are expecting a surgical operation, or other hospital in-patients. They are also useful in patients who will be catheterized. They are also useful in adolescent females (*e.g.* aged 11-18) and in patients with chronic urinary tract infections.

Vaccines of the invention may be administered to patients at substantially the same time as (*e.g.* during the same medical consultation or visit to a healthcare professional or vaccination centre) other vaccines *e.g.* at substantially the same time as a measles vaccine, a mumps vaccine, a rubella vaccine, a MMR vaccine, a varicella vaccine, a MMRV vaccine, a diphtheria vaccine, a tetanus vaccine, a pertussis vaccine, a DTP vaccine, a conjugated *H. influenzae* type b vaccine, an inactivated poliovirus vaccine, a hepatitis B virus vaccine, a meningococcal conjugate vaccine (such as a tetravalent A-C-W135-Y vaccine), a respiratory syncytial virus vaccine, *etc.*

### General

The practice of the present invention will employ, unless otherwise indicated, conventional methods of chemistry, biochemistry, molecular biology, immunology and pharmacology, within the skill of the art. Such techniques are explained fully in the literature. See, *e.g.,* references 96-97, *etc.*

The term "comprising" encompasses "including" as well as "consisting" e.g. a composition "comprising" X may consist exclusively of X or may include something additional *e.g.* X + Y.

The term "about" in relation to a numerical value x means, for example, *x*±10%.

"GI" numbering is used herein. A GI number, or "GenInfo Identifier", is a series of digits assigned consecutively to each sequence record processed by NCBI when sequences are added to its databases. The GI number bears no resemblance to the accession number of the sequence record. When a sequence is updated (*e.g.* for correction, or to add more annotation or information) then it receives a new GI number. Thus the sequence associated with a given GI number is never changed.

References to a percentage sequence identity between two amino acid sequences means that, when aligned, that percentage of amino acids are the same in comparing the two sequences. This alignment and the percent homology or sequence identity can be determined using software programs known in the art, for example those described in section 7.7.18 of ref. 98. A preferred alignment is determined by the Smith-Waterman homology search algorithm using an affine gap search with a gap open penalty of 12 and a gap extension penalty of 2, BLOSUM matrix of 62. The Smith-Waterman homology search algorithm is disclosed in ref. 99.

One of skill in the art would understand that "isolated" means altered "by the hand of man" from its natural state, *i.e*., if it occurs in nature, it has been changed or removed from its original environment, or both. For example, a polynucleotide or a peptide naturally present in a living organism is not "isolated" when in such living organism, but the same polynucleotide or peptide separated from the coexisting materials of its natural state is "isolated," as the term is used in this disclosure. Further, a polynucleotide or peptide that is introduced into an organism by transformation, genetic manipulation or by any other recombinant method would be understood to be "isolated" even if it is still present in said organism, which organism may be living or non-living, except where such transformation, genetic manipulation or other recombinant method produces an organism that is otherwise indistinguishable from the naturally occurring organism.

### BRIEF DESCRIPTION OF DRAWINGS

Figure1 shows a proposed mechanism of action for saccharide conjugate vaccines.
Figure2 shows a new proposed mechanism of action for saccharide conjugate vaccines.
Figure 3 shows preferred linkage points for the conjugates and for the linear conjugates that were used in the examples described below.
Figure4 shows two types of glycopeptides: a linear conjugate structure for oligoMenC conjugate and a nonlinear conjugate structure for polyMenC conj.
Figure 5 shows the chemical scheme to prepare the linear oligoMenC-peptide conjugates. (a) ammonium acetate at final concentration of 50mM, sodium cyanoborohydride at final concentration of 10mM in a solution 10% DMSO 90% MeOH of 1 at 5mg/ml, 50°C for 24-72h. (b) 10 eq of sulfo-EMCS linker respect to amino groups, 5 eq. of Et₃N, DMSO:H₂O (9:1). (c) 3 eq. of peptides in PBS 1X, 3mg/ml in terms of peptide.
Figure 6 shows the ¹H NMR spectrum of oligoMenC after introduction of maleimido group.
Figure 7 shows an SDS-PAGE 4-12% Bis-Tris, MES as running buffer of a glycopeptide oligoMenC-peptide (lane 3) as compared to the peptide alone (lane 2); all conjugates described in the examples had a similar SDS-PAGE profile.
Figure 8 shows RP-HPLC elution profiles of oligoMenC-Met6 conj (line pink), in comparison with Peptide Met6 (line blue) and oligoMenC (line red) (Jupiter C18 column, gradient of H₂O - ACN with 0.1%TFA from 95% of H₂O and 5% of ACN up to 45% of ACN; flow 0.1 ml/min. All conjugates described in the examples showed a similar profile.
Figure 9 shows the chemical process used to prepare polyMenC-Peptide conjugates (a) EDAC 20%, KMUH 20% in MES buffer pH4.56, 2mg/ml in terms of saccharide; (b) 3 eq. of peptide respect to mol of linkers introduced in PBS 1X pH 7, 3mg/ml in terms of peptide.
Figure 10 shows a representative ¹H NMR spectrum of a polysaccharide after derivatization with KMUH showing incorporation of about 35 mol of maleimido groups per mol of saccharide chain.
Figure 11 shows a representative SDS-PAGE 4-12% Bis-Tris with MES as running buffer of a polyMenC-peptide conjugate (lane 3) in comparison with peptide alone (lane 2). All conjugates showed a similar trend.
Figure 12 shows a representative set of RP-HPLC profiles of polyMenC-Met6 conjugate (pink line), in comparison with Peptide Met6 alone (blue line) and polyMenC alone (black line). Jupiter C18 column, gradient of H₂O - ACN with 0.1%TFA from 95% of H₂O and 5% of ACN up to 45% of ACN; flow 0.1ml/min. All conjugates showed a similar profile.
Figure 13 shows RP-HPLC profiles of oligoMenC-Met6 conj (pink line) in comparison with polyMenC-Met6 conj (red line), showing a different interaction with C18 column. Jupiter C18 column, gradient of H2O - ACN with 0.1%TFA from 95% of H₂O and 5% of ACN up to 45% of ACN; flow 0.1ml/min. All conjugates showed a similar profile.
Figure 14 shows IgG titers induced by polyMenC conjugates against Met6 and Fba peptide as determined by ELISA assay (coating plates with peptides), reported as GMT (geometrical mean with confidential interval 95%), cut off OD 0.2.
Figure 15 shows IgG titers induced by polyMenC conjugates against the respective glycoconjugates determined by ELISA assay (coating plates with glycopeptides), reported as GMT (geometrical mean with confidential interval 95%), cut off OD 0.2.
Figure 16 shows a representative ¹H NMR spectrum of polysaccharide after derivatization with KMUH linker, showing the introduction of 1 mol of linker per about 16 mol of saccharide.
Figure 17 shows a representative SDS-PAGE 4-12% Bis-Tris with MES as running buffer of a polyMenC-peptide conjugate (lane 3) in comparison with peptide alone (lane 2). All conjugates showed a similar trend.
Figure 18 shows the process used to prepare polyMenC-Peptide conjugates (a) EDAC 20%, 2.3 eq. KMUH linker in MES buffer pH4.56, 2mg/ml in terms of saccharide; (b) 3 eq. of peptide respect to mol of linkers introduced in PBS 1X pH 7, 3mg/ml in terms of peptide.
Figure 19 shows HPLC profiles of polyMenc-PV1 N-terminal conjugate (pink line) compared to free peptide (blue line) and polysaccharide alone (black line); HPLC-SEC, Superdex Peptide column, NaPi 20mM, NaCl 250mM pH7, 0.05ml/min, isocratic condition. All conjugates showed a similar profile.
Figure 20 shows a representative ¹H NMR spectrum of polysaccharide after derivatization with BMPH linker, showing the introduction of 1 mol of linker per about 17 mol of saccharide.
Figure 21 shows a representative SDS-PAGE 4-12% Bis-Tris with MES as running buffer of a polyMenC-peptide conjugate (lane 3) in comparison with peptide alone (lane 2). All conjugates showed a similar trend.
Figure 22 shows the process used to prepare the polyMenC-Peptide conjugates (a) EDAC 20%, 2.3eq. BMPH linker in MES buffer pH4.56, 2mg/ml in terms of saccharide; (b) 3 eq. of peptide respect to mol of linkers introduced in PBS pH 7, 3mg/ml in terms of peptide.
Figure 23 shows a representative SDS-PAGE 4-12% B-T with MES as running buffer of a polyMenC-MIX peptide conjugate (lane 3) in comparison with mixture of peptides (lane 2).
Figure 24 shows the process used to prepare polyMenC-MIX Peptide conjugates (a) EDAC 20%, 2.3eq. BMPH linker in MES buffer pH4.56, 2 mg/ml in terms of saccharide; (b) mixture of 1 eq. of each peptide (3 eq. in total respect to mol of linkers) PBS pH 7.
Figure 25 shows the ¹H NMR spectrum of the Fba peptide.

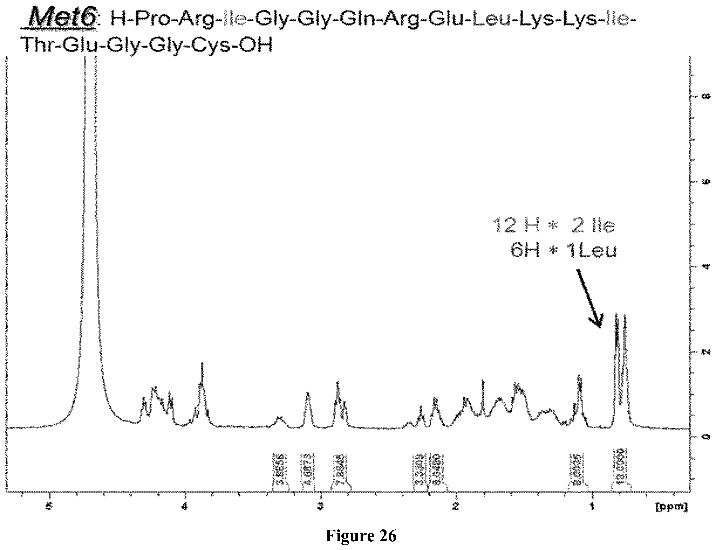

Figure 26 shows the ¹H NMR spectrum of the Met6 peptide.

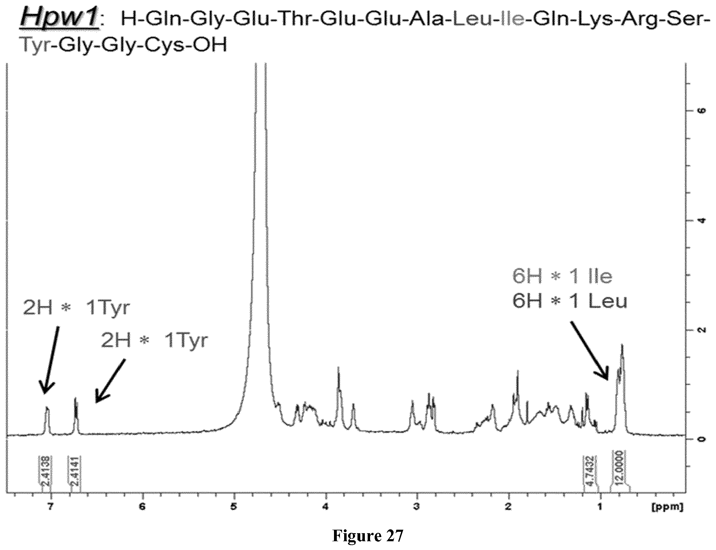

Figure 27 shows the ¹H NMR spectrum of the Hpw1 peptide.
Figure 28 shows the ¹H NMR of conjugate linked to Fba, Hpw1 and Met6 in ratio 1:1:2.

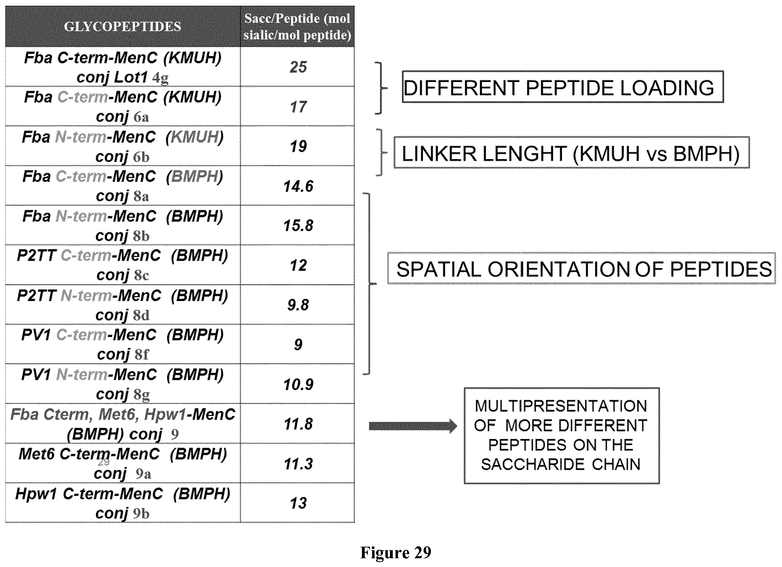

Figure 29 shows the different conjugates compared in the second set of immunogenicity assays.
Figure 30 shows the IgG titers induced by polyMenC conjugates against the CPS meningococcal serogroup C as determined by ELISA assay, reported as GMT (geometrical mean with confidential interval 95%), cut off OD 1.
Figure 31 shows IgG titers induced by polyMenC-PV1 C- and N-terminus conjugates against Meningococcal polysaccharide serogroup C, on post2 (blue) and on post3 (red), determined by ELISA assay, reported as GMT (geometrical mean with confidential interval 95%), cut off OD 1.
Figure 32 shows IgM titers induced by polyMenC-PV1 C- and N-terminus conjugates against Meningococcal polysaccharide serogroup C (red) in comparison with IgG titers (green), determined by ELISA assay, reported as GMT (geometrical mean with confidential interval 95%), cut off OD 1.
Figure 33 shows IgG titers induced by polyMenC-PV1 C- and N-terminus conjugates against the respective peptides, determined by ELISA assay, reported as GMT (geometrical mean with confidential interval 95%), cut off OD 1.

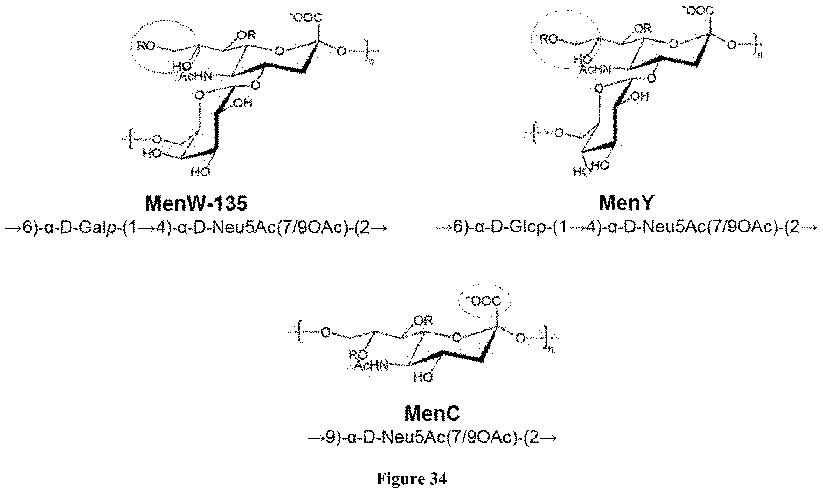

Figure 34 shows the Chemical structure of repeating units of Meningococcal polysaccharide serogroup C, W-135 and Y.
Figure 35 shows the chemical process used to prepare conjugates of Ova peptide with polyMenW and polyMenY (a) NaIO4 0.1M 30% of sialic acid mol in NaPi 10mM pH7 to obtain 10mg/ml in terms of saccharide, 2h, r.t. at dark (b) NaBH3CN (2 eq.), Ova peptide (1.5eq.) in NaPi 50mM NaCl 200mM pH7, 5 days at 37°C.
Figure 36 shows the ¹H NMR spectrum of Meningococcal polysaccharide serogroup W-135 after oxidation of sialic acid (30%).
Figure 37 shows the ¹H NMR spectrum of Meningococcal polysaccharide serogroup Y after oxidation of sialic acid (30%).
Figure 38 show a representative SDS-PAGE 4-12% Bis-Tris, MES as running buffer of conjugates **12** (lane 3) and **13** (lane 4) in comparison with Ova peptide (lane 2).
Figure 39 shows the chemical process used to prepare PolyMenC-Ova peptide conjugates (a) EDAC 20%, 2.3eq. KMUH linker or BMPH linker in MES buffer pH4.56, 2mg/ml in terms of saccharide; (b) 3 eq. of Ova peptide GGC respect to mol of linkers introduced in PBS 1X pH 7, 3mg/ml in terms of peptide.

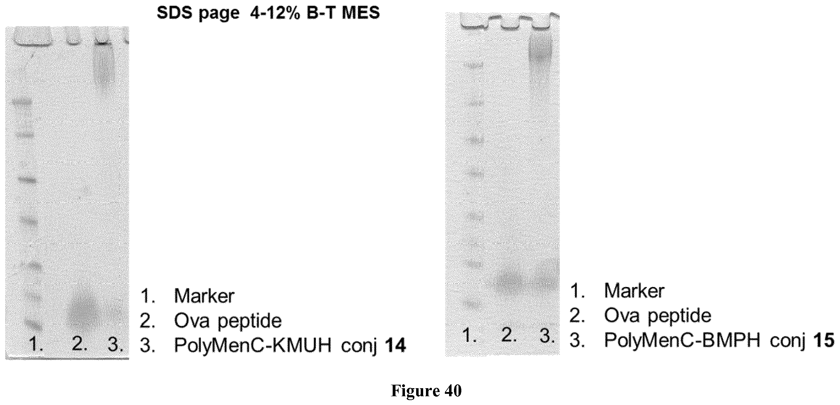

Figure 40 shows SDS-Page 4-12% Bis-Tris, MES as running buffer of conjugates **14** and **15.**

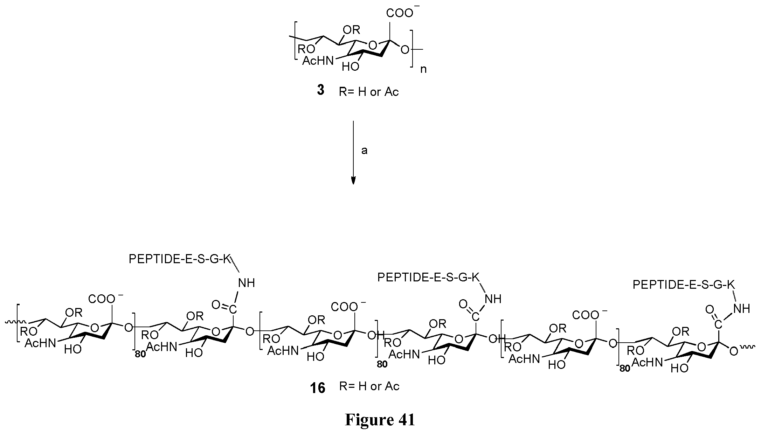

Figure 41 shows the chemical used process to prepare polyMenC-Ova peptide conjugates (a) EDAC 1 eq., sulfo NHS 1 eq., Ova peptide 1 eq, in MES 30mM pH 6,3 mg/ml in terms of peptide.
Figure 42 shows an SDS-Page 4-12% Bis-Tris MES as running buffer of the conjugate polyMenC-Ovap **16.**
Figure 43 shows IgG titers induced by PolyMenC-Ova peptide conjugates against Meningococcal polysaccharide serogroup C, determined by ELISA assay, reported as GMT (geometrical mean with confidential interval 95%), cut off OD 1.
Figure 44 shows IgG titers induced by polyMenC-Ova peptide conjugates against Meningococcal polysaccharide serogroup C on post2 (blue) and on post3 (pink), determined by ELISA assay, reported as GMT (geometrical mean with confidential interval 95%), cut off OD 1.
Figure 45 shows IgG titers induced by polyMenW-Ova peptide and polyMenY-Ova peptide poly conjugates against respectively the Meningococcal polysaccharide serogroup W and Y, determined by ELISA assay, reported as GMT (geometrical mean with confidential interval 95%), cut off OD 1.
Figure 46 shows IgG titers induced by polyMenC- W- Y-Ova peptide conjugates against Ova peptide, determined by ELISA assay, reported as GMT (geometrical mean with confidential interval 95%), cut off OD 1.

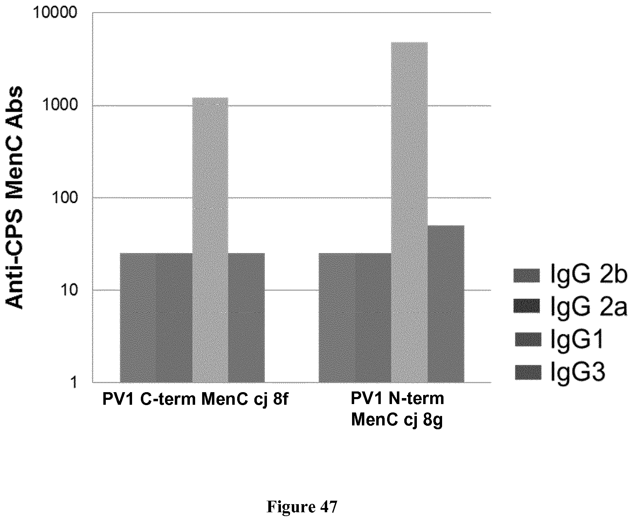

Figure 47 shows titers induced by polyMenC-PV1 C- and N-terminus conjugates against Meningococcal polysaccharide serogroup C fir different IgG subtypes, determined by ELISA assay, reported as GMT (geometrical mean with confidential interval 95%), cut off OD 1.
Figure 48 shows a compeititive ELISA between native Meningococcal polysaccharide from Serogroup C and (A) the polyMenC conjugate (orange line), (B) the PV1 epitope with N-terminal linker alone (blue), and (C) laminarin saccharide negative control (green), determined by ELISA assay, reported as percent inhibition.

### EXAMPLES

### Overview

Meningococcal disease receives prominent public health attention because of its extremely rapid onset and progression; the disease can be fatal in a matter of hours. Surface polysaccharides have long been key antigens for vaccines against major disease-causing meningococcal serogroups A, C, W-135 and Y [Bardotti, A. et al. Vaccine, 2008, 26(18), 2284-2296; Broker, M. et al. Vaccine, 2009, 27(41), 5574-5580; Fusco, P.C. et al. Clin Vaccines Immunol. 2007, 14(5), 577-584.]. The conjugation of polysaccharides to a carrier protein has conferred important public health benefits, improving its immunogenicity and allowing the induction of a T-cell dependent (TD) response [Pollard, A.J. et al. Nat. Rev. Immunol. 2009, 9(3), 213-220; Ada, G. et al. Clin. Microbiol. Infect. 2003, 9(2), 79-85]. Thus, saccharide from meningococcal serogroups provides an exemplary saccharide for illustration of the conjugates disclosed herein. In order to investigate the nonlinear conjugates and PV1 polyepitope conjugates, the inventors studied the immunological properties of saccharide conjugates, where the whole carrier protein was substitutes with synthetic T-cell epitope peptides with either linear or nonlinear conjugation.

As described below, a first panel of peptides was conjugated at their C-terminus to the oligosaccharides from meningococcal serogroup C. The peptides were derived from cell wall protein of *Candida albicans* [Xin, H. et al. PNAS 2008, 105, 13526-13531] and from *Tetanus toxin* epitopes used in the N19 polyepitope carrier peptide [Baraldo, K. et al. Infect. Immun. 2004, 72,4884-4887]. The peptides were conjugated to two different meningococcal serogroup C saccharides: an oligosaccharide (oligoMenC) and a polysaccharide (polyMenC). None of the conjugates (formulated with or without aluminium hydroxide as adjuvant) induced an IgG response against the saccharides. Only the polyMenC-Fba conjugate and the polyMenC-Met6 conjugate induced an IgG response against the peptides (both from cell wall protein of *Candida albicans*).

The immunogenicity of conjugates and the conjugates could be affected by several parameters, such linkage of peptide the peptide at or near its N-terminus versus its C-terminus, the degree of peptide loading on saccharide chain, the length of the linker or spacer, whether all the peptides are the same epitope or there are different epitopes linked to the saccharide [*see, e.g.,* Chong, P. et al. Infection and Immunity, 1997, 65, 4918-4925]. All of these parameters were evaluated in the examples below, but none of the parameters proved relevant (likely due to the selected peptides for the first set of experiments providing only low immunogenicity as T-cell epitope). From testing a second panel of peptides, the inventors discovered that the PV1 peptide, derived from protein poliovirus type1, was a particularly effective substitute for traditional carrier proteins; in particular, the PV1 peptide, linked at its *N*-terminus, induced antibody titers comparable to a conventional carrier protein: a MenC-CRM conjugate.

Furthermore, since a peptide derived from Ovalbumin protein (OVA) has also been shown to be an effective carrier peptide for polysaccharide GBS type III [Avci1, F.Y. et al. Nature Medicine, 2011, 17, 1602-1610], inventors demonstrated in the examples below that the peptide is able to substitute for traditional whole carrier protein-saccharide conjugates such as meningococcal polysaccharides serogroup C, W-135 and Y conjugates. The immunological evaluation below shows that the Ova peptide was a good carrier for meningococcal polysaccharide from serogroup C, but was not as good for meningococcal polysaccharides from serogroup W-135 and Y.

### Introduction

For the initial round of experiments, T-cell epitopes were conjugated to meningococcal oligosaccharides from serogroup C as linear conjugates and to meningococcal polysaccharides from serogroup C as nonlinear conjugates. The inventors used T-cell epitopes selected two tetanus derived peptides (P30TT and P32TT) from the N19 polyepitope carrier peptide, a recombinant peptide designed with multiple CD4+ T cell epitopes derived from different pathogens. The inventors also tested five peptide (Hpw1, Eno1, Gap1, Fba, Met6) derived from the cell wall proteins of *Candida albicans*.

These synthetic peptides were purchased from an external laboratory. The peptides were synthesized with a peptide linker at the C-terminus for attachment to the saccharide.

P30TT and P32TT peptides used in these examples were derived from the Tetanus toxin epitopes found in the N19 peptide. The N19 peptide is a genetically engineered polyepitope carrier peptide expressed in *Escherichia coli* and consisting of several human CD4+ T-cell universal epitopes, which behaves as a strong carrier when conjugated to Hib polysaccharide. Studies with human cells *in vitro* showed that these epitopes in the N19 peptide were correctly processed and recognized and were able to induce an epitope-specific T cell proliferation. Subsequent studies showed that T-cell precursors specific for the epitopes (P32TT, P30TT, P23TT) have been generated *in vivo* in mice following immunization with N19-MenACWY conjugates; other results clearly demonstrated that mouse antigen-presenting cells *in vitro* were able to process the N19 peptide and to generate the correct epitopes which, in turn, were able to reactivate the specific T cells, primed by the in vivo immunization with the single peptide [Baraldo, K. et al. Infect Immun. 2005, 73(9), 5835-5841]. Additional peptides for this first set of examples were selected from *Candida albicans* cell wall proteins on the basis of their known expression during pathogenesis of human disseminated candidiasis which included: hyphal wall protein-1 (Hpw1), enolase (Enol), glyceraldehyde-3-phosphate dehydrogenase (Gap1), fructose-bisphosphate aldolase (Fba) and methyltetrahydropteroyltriglutamate (Met6). After mice immunization, using an antigen-pulsed dendritic cell (DC)-based vaccine strategy, conjugates with synthetic β-1,2-linked mannotriose coupled to each synthetic peptide were immunogenic; moreover the vaccination showed protection against experimental disseminated candidiasis [Xin, H. et al. PNAS, 2008, 105, 13526-13531]. Further studies have shown that, between all these peptides, Fba alone induced an antibody response and protection against candidiasis.

For this first set of experiments, we used oligosaccharides with *av* DP18 (average degree of polymerization) and native polysaccharides (full length) from *Neisseria meningitidis* serogroup C that were extracted and purified from bacteria.

Monovalent meningococcal serogroup C conjugate vaccines have been successfully introduced into many countries around the world, Menjugate® and Meningitec™, where the carrier protein is CRM₁₉₇, or NeisVac-C™ where the carrier is TT or Menitorix® where MenC is conjugated to TT in combination with Hib [Trotter, C.L. et al. Lancet, 2004, 364(9431), 365-7; Balmer, P. et al. J Med Microbiol, 2002, 51(9), 717-22]. Moreover tetravalent conjugate vaccines covering serogroup A, C, W135 and Y have been developed, contributing to strongly reduce the incidence of meningitis worldwide. The tetravalent meningococcal conjugate vaccines currently commercially available, Menactra™, Menveo® and Nimenrix™, differ for saccharide chain length, carrier protein (DT, CRM₁₉₇ and TT respectively) and conjugation chemistry [Gasparini, R. and Panatto, D. Human Vaccines 2011, 7(2), 170-82; Jackson, L.A. et al. Clin Infect Dis 2009, 49(1), 1-10; Reisinger, K.S. et al. Clin Vaccines Immunol 2009, 16(12),1810-5; Halperin, S.A. et al. Vaccines 2010, 28(50), 7865-72]. Thus, there is an abundance of data regarding meningococcal serogroup C saccharides conjugated to traditional carrier protein that can be compared to the conjugates of the present invention.

A second set of experiments was performed evaluating some characteristics that may influence the immunogenicity of glycopeptides, such as peptides linked at the N-terminus versus peptides linked at the C-terminus, density of peptides linked to the saccharide chain, different linker lengths to conjugate peptides to the saccharide, use of peptides with different epitopes linked to the same saccharide chain. There is a fair amount of literature that can provide the skilled artisan additional guidance regarding how these parameters will affect the immunogenicity of the conjugates disclosed herein. For example, resultsobtained with *Haemophilus influenzae* type b glycoconjugates suggested that the orientation of the saccharide moiety relative to the T-cell epitope could influence the host immune response to the saccharide. Saccharide conjugates with the peptide linked to the saccharides at the C-terminus of peptides induced 5-10-fold lower anti-saccharide antibody response than their positional isomers linked through the *N*-terminus of peptides [Chong, P. et al. Infection and Immunity, 1997, 65, 4918-4925]. These results were supported by Alonso De Velasco's work where polysaccharide of *Streptococcus pneumoniae* conjugated to the *N*-terminus of a T-cell epitope elicited an anti-PS antibody response significantly higher than that elicited by the conjugate with PS coupled at the C-terminus of the peptide. The difference in immunogenicity for conjugates with PS at C-terminus was due to the inability of antigen-presenting cells to efficiently cleave the T-cell epitope (C-terminus) from PS and present it in the context of major histocompatibility complex (MHC) class II molecules to helper T cells [De Velasco, E.A. et al. Infection and Immunity, 1995, 63, 961-968]. Studies on working models of a glycopeptide showed that this latter conjugate could bind to class II MHC molecules and elicited specific T cell responses. In fact, it was able to generate specific T cell hybridomas against the glycopeptide but not against the corresponding non-glycosylated peptide. Moreover the carbohydrate moiety had an important conformational influence on the determinant recognized by the T cells; T-cells recognized peptide residues under the carbohydrate influence, the position of the hapten in the peptide was critical for T cell recognition. Also the distance between saccharide hapten and peptide could influence the immunogenicity [Harding, C.V. et al. The Journal of Immunology, 1993, 151, 2419-2425].

With this background in mind, we tested conjugates with linkers of different length, to reduce the distance between B cell (saccharide) and T cell epitope (peptide).

Regarding the peptide loading, in *Haemophilus influenzae* type b conjugates, the authors have described that the presentation of multiple copies of a peptide antigen on a polylysine backbone (MAP) system could enhance its immunogenicity; so multiple T-cell epitopes were incorporated into the construct to overcome MHC class II genetic restriction. These studies showed that the immunogenicity of glycopeptides could be enhanced when a MAP system was used as carrier instead of a linear peptide [Chong, P. et al. Infection and Immunity, 1997, 65, 4918-4925].

Based upon this, we prepared conjugates where peptides are coupled to polysaccharide chain in a nonlinear conjugates form that allows for higher peptide loading and allows different peptides to be coupled on the same saccharide, but on distinct peptide unlike polyepitope carrier peptides such as the N19 peptide.

In this set of experiments, the inventors also tested other synthetic T-cell epitope peptides, including P2TT peptide which is another peptide from the tetanus toxin epitope used in the N19 peptide [Baraldo, K. et al. Infect. Immun. 2004, 72, 4884-4887]⁴ and PV1 peptide identified as a T-cell epitope of the VP1 protein of poliovirus type 1 [Leclerc, C. et al. Journal of Virology, 1991, 65, 711-718.]. In the field of cancer vaccine research, the PV1 peptide has been used in a dendritic multiple antigen glycoprotein (MAG) structure conjugated to a Tn antigen, a glycosidic tumor marker. The MAG:Tn-PV was able to induce anti-Tn IgG antibodies that recognized human tumor cell lines, and therapeutic immunization protocol performed with this fully synthetic immunogen increased the survival of tumor-bearing mice [Bay, S. et al. J. Peptide Res. 1997, 49, 620-625; Lo-Man, R. et al. Cancer Research, 1999, 59, 1520-1524].

Finally, the examples demonstrate the immunological ability of Ova peptide derived from Ovalbumin protein to substitute for the traditional whole carrier protein conjugated to saccharides such as Meningococcal polysaccharide serogroup W-135, Y and C. In fact, Avcil *et al.* showed that this Ova peptide has been a good carrier for polysaccharide GBS type III [Avcil, F.Y. et al. Nature Medicine, 2011, 17, 1602-1610].

Avcil *et al.* compared the immune response of polysaccharide GBS type III-OVAp vaccine with that of polysaccharide GBS type III-OVA glycoconjugate constructed by the technology currently used in the industrial manufacture of several vaccines. The study showed that III-Ovap constructed to maximize the presentation of carbohydrate-specific T cell epitopes was 50-100 times more potent and substantially more protective in a neonatal mouse model of group B Streptococcus infection respect to a vaccine constructed by methods currently used by the vaccine industry [Avci, F.Y. and Kasper, D.L. Annu. Rev. Immunol. 2010, 28, 107-130; Paoletti, L.C. and Kasper, D.L. Expert Opin. Biol. Ther. 2003, 3(6), 975-984].

### Results

### Screening of T-cell epitope peptides with C-terminus orientation: physico-chemically characterization and immunological evaluation in mouse model

To demonstrate efficacy of the conjugates, the inventors tested synthetic peptides containing T-cell epitopes from different sources and combining several features that could affect the immunogenicity such as spatial orientation of peptides, density of peptides on saccharide chain and multipresentation of different types of peptides.

In the first set of experiments, seven peptides were selected: two of them were epitopes derived from Tetanus toxin and used in the N19 peptide and five of them were epitopes derived from cell wall proteins of *Candida albicans.*

**Table 2 shows the amino acid sequences and organism source for each peptide.**

| Peptide name | Source | Amino acid sequence [SEQ ID NO] |
|---|---|---|
| P32TT | Tetanus toxin | LKFIKRYTPNNEIDS- [1] |
| P30TT | Tetanus toxin | FNNFTVSFWLRVPKVSAHLE- [2] |
| Hpw | *Candida albicans* | QGETEEALIQKRSY- [3] |
| Enol | *Candida albicans* | DSRGNPTVEVDFTT- [4] |
| Gap1 | *Candida albicans* | NRSPSTGEQKSSGI- [5] |
| Fba | *Candida albicans* | YGKDVKDLFDYAQE- [6] |
| Met6 | *Candida albicans* | PRIGGQRELKKITE- [7] |

These peptides were purchased from an external laboratory containing a linker equipped with a free thiol group (Gly-Gly-Cys) to introduce saccharide antigen on *C*-terminus. They were provided as pure products at least for 85% after purification by HPLC in reverse phase.

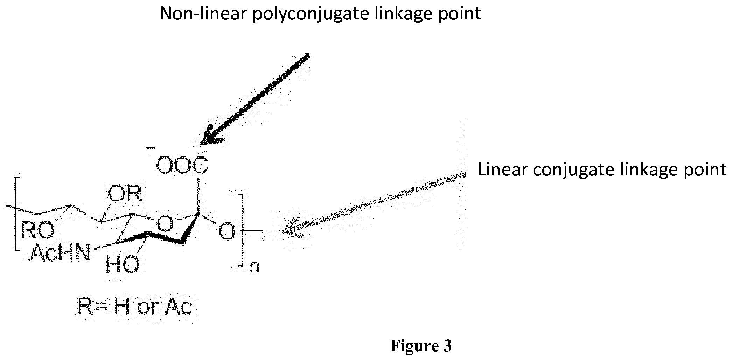

The saccharides were meningococcal oligosaccharide *av*DP 18 from serogroup C (for use in the conjugates) and meningococcal polysaccharide from serogroup C (for use in the conjugates). Their native chains are characterized by the assembly of sialic acid molecules as repeating units (See, e.g., Figure 3).

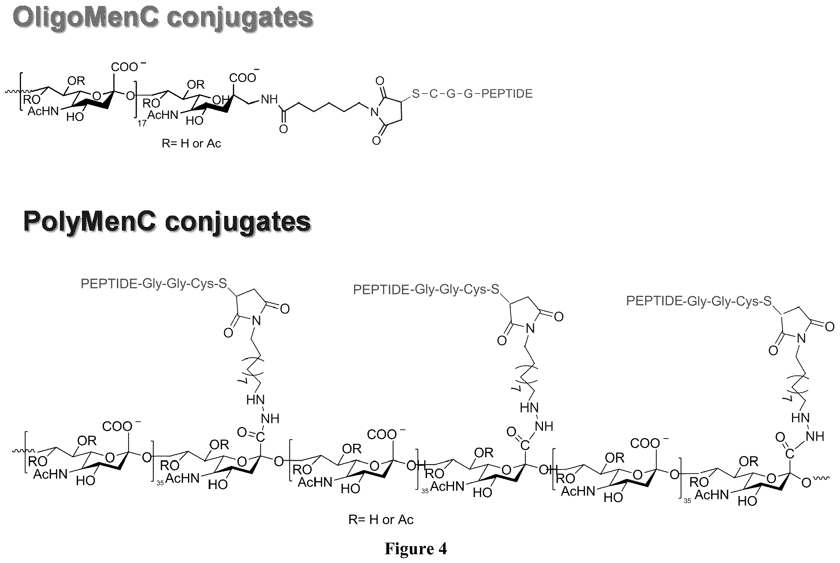

The conjugates were prepared conjugating the different peptides to oligosaccharides for the conjugates ("oligoMenC) and to the polysaccharides for the conjugates ("polyMenC"), obtaining two different structures: linear conjugates or nonlinear conjugates. The oligoMenC-peptide conjugates were synthesized as linear conjugates characterized as having a 1:1 molar ratio between the oligosaccharide and the peptide epitope because the saccharide was only derivatized with a linker at the reducing end. The polyMenC-peptide conjugates were synthesized as nonlinear conjugates characterized by peptide epitopes being linked along the chain of the native polysaccharide due to the introduction of linkers on the carboxyl groups of sialic acid molecules along the saccharide chain (see, e.g., Figure 4).

### OligoMenC Conjugates

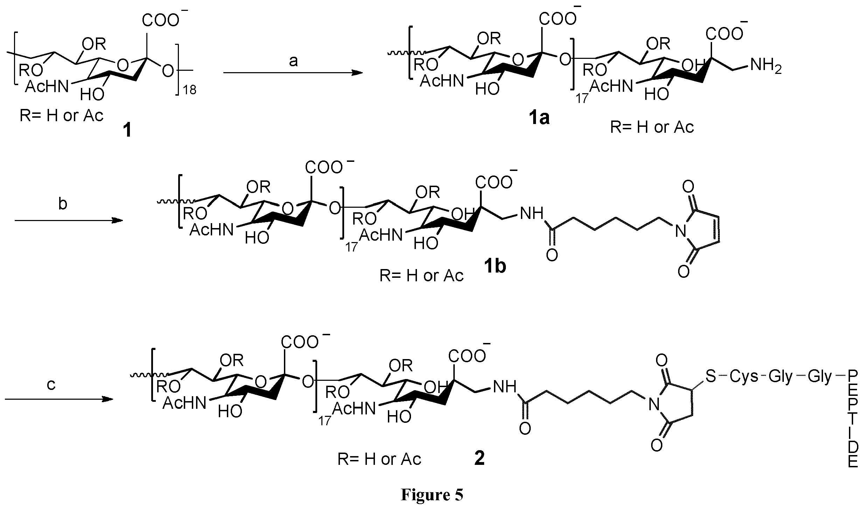

Meningococcal oligosaccharide serogroup C, after reductive ammination, was derivatized with sulfo-EMCS (N-epsilon-Maleimidocaproyl-oxysulfosuccinimide ester) linker. Thus, a maleimido was introduced at the reducing end of saccharide chain able to react with free thiol group of each peptide (Figure 5).

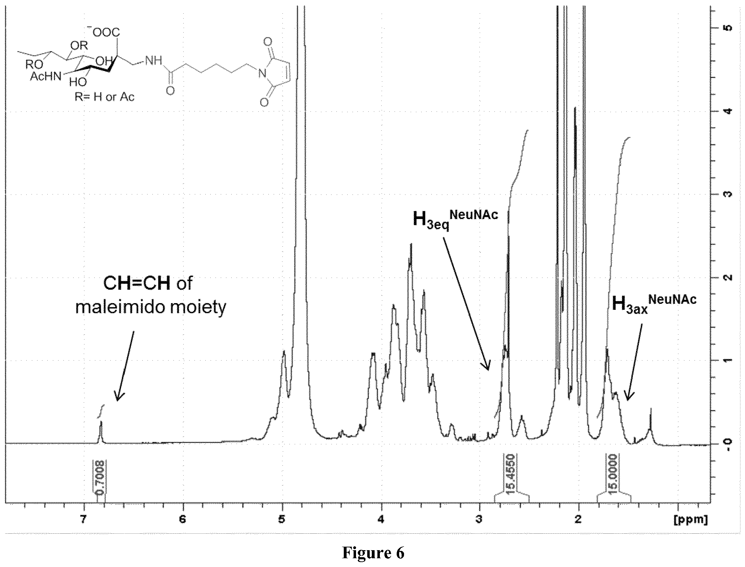

The oligosaccharide after derivatization was purified by precipitation with acetone and then was characterized by ¹H NMR, confirming the introduction of maleimido moiety at the reducing end (Figure 6).

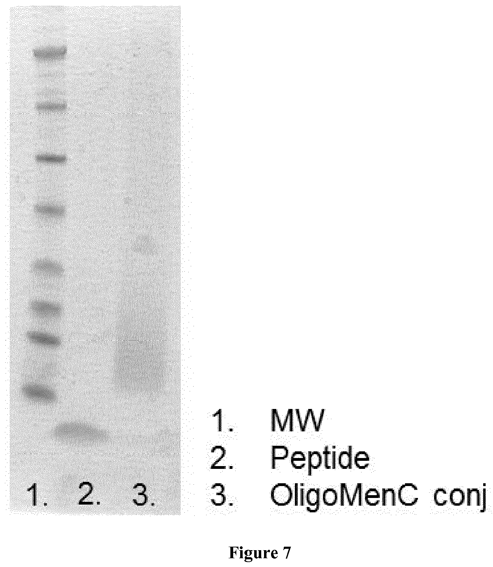

After derivatization and conjugation overnight, the conjugation reaction was confirmed by SDS-Page 4-12% Bis-Tris (see, e.g., Figure 7). All seven oligoMenC conjugates each with a different peptide linked through its C-terminus were obtained.

The conjugates were purified by size exclusion chromatography, using a pre-packed Superdex Peptide column to remove free peptide and followed by precipitation with ammonium sulphate to remove free saccharide.

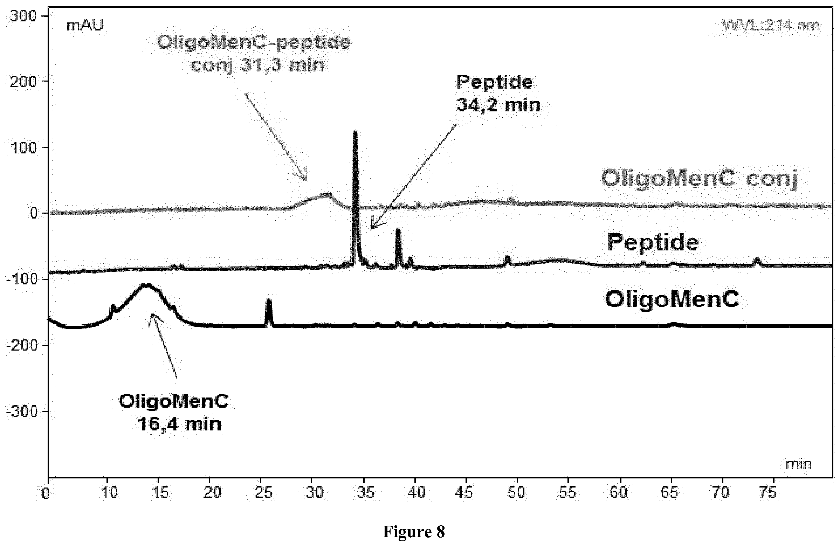

The purified glycopeptides were characterized by RP-HPLC analysis using a Jupiter C18 column (Figure 8). Only one exemplary HPLC profile for the oligoMenC-Met6 conjugate is included; all conjugates showed similar profiles.

As shown in the HPLC profile, the oligoMenC conjugates after conjugation showed an intermediate behavior between peptide and oligoMenC alone, because it was retained on the column for a longer time than oligoMenC but for a shorter time than the peptide (line pink).

After purification, the protein content was estimated by MicroBCA colorimetric assay and the sialic acid content by resorcinol colorimetric assay. The ratio between saccharide and peptide was approximately 1:1 for all glycopeptides in agreement with the preparation of a linear structure and taking in consideration that the oligoMenC moiety is a polydispersion, as reported in table 3.

| Sample | MenC saccharide | Saccharide/peptide (mol/mol) |
|---|---|---|
| P32TT-MenC **2a** | Oligo avDP 18 | 0.7 |
| P30TT-MenC **2b** | Oligo avDP 18 | 0.7 |
| Met6-MenC **2c** | Oligo avDP 18 | 0.8 |
| Gap1-MenC **2d** | Oligo avDP 18 | 0.7 |
| Hpw1-MenC **2e** | Oligo avDP 18 | 0.7 |
| Eno1-MenC **2f** | Oligo avDP 18 | 0.8 |
| Fba-MenC **2g** | Oligo avDP 18 | 0.8 |

### PolyMenC Conjugates

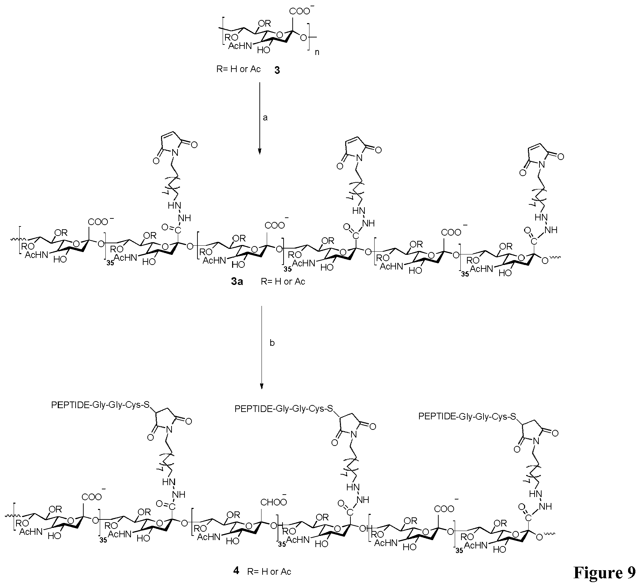

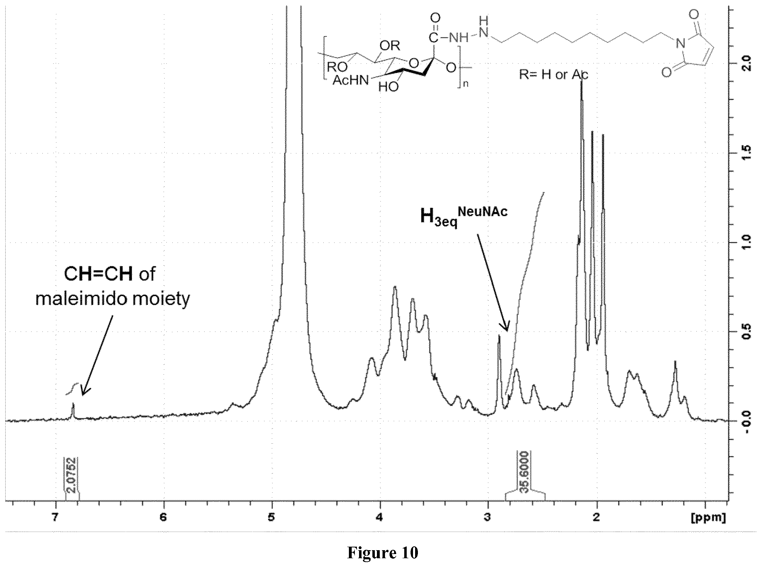

Meningococcal polysaccharide serogroup C was derivatized to introduce a maleimido moiety able to react with thiol group of each peptide. A KMUH (N-kappa-Maleimidoundecanoic acid hydrazide-TFA) linker was used as it is able to react with carboxyl groups of sialic acid using EDAC (N-(3-Dimethylaminopropyl)-N'-ethylcarbodiimide hydrochloride) chemistry (Figure 9 shows the reaction scheme). In this first set of experiments 1 mol of KMUH linker per about 35 mol of saccharide was introduced, as evidenced by ¹H NMR spectrum (see Figure 10).

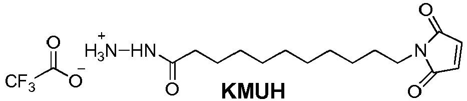

In this first set of experiments KMUH with 10 carbon atom was used at the linker to avoid problems of steric hindrance in the reaction of conjugation with peptides.

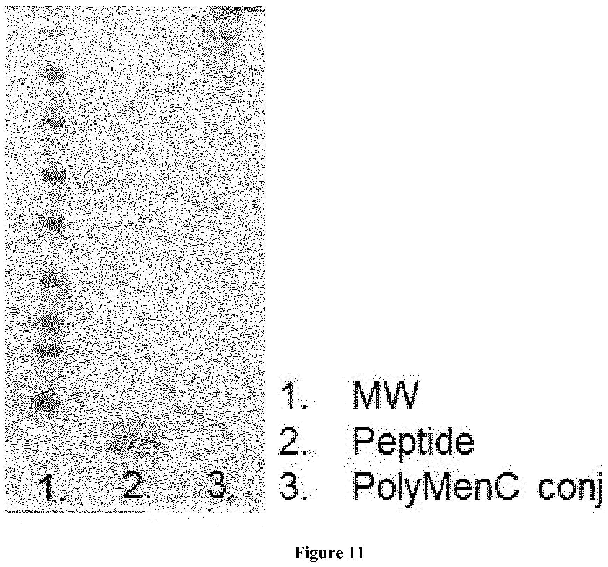

After introduction of maleimido moieties on saccharide chain, the conjugation reaction with each peptide has been performed properly in buffer phosphate, and after one night the glycopeptides were formed, as evidenced by SDS-Page 4-12% Bis-Tris. (Figure 11).

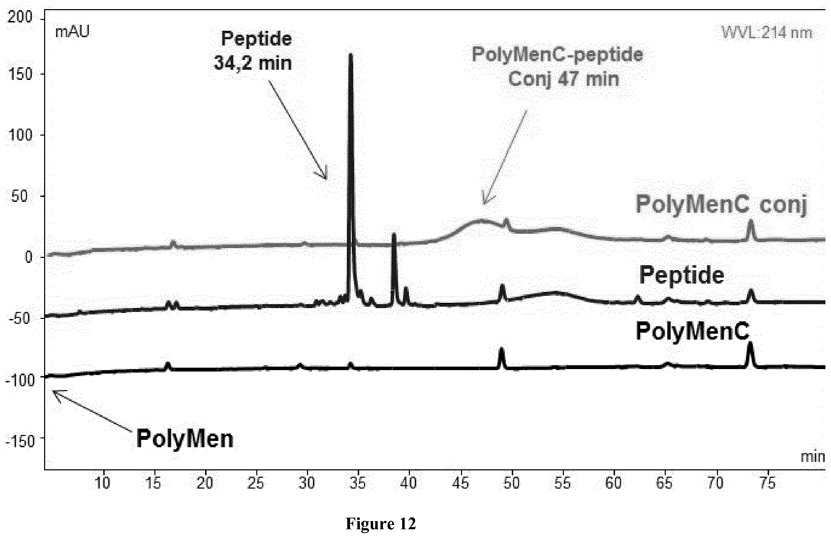

The seven glycopeptides were purified by vivaspin cut off 30kDa to remove free peptide and then they were characterized by RP-HPLC using a C18 column. Only one example of the HPLC profiles is provided for the polyMenC-Met6 conjugate; all conjugates showed a similar profile (Figure 12). These conjugates were retained on the RP-HPLC column longer than peptide alone due to increased hydrophobic interactions with column likely due to the presence of more peptides on saccharide chain. On the other hand, the polyMenC saccharide alone was not retained by the column and it was eluted in the void volume of the column.

After purification, the saccharide content and protein content of the conjugates were determined using colorimetric assays, in particular resorcinol and micro BCA assay respectively.

As reported in table 4 the seven glycopeptides prepared with polyMenC showed a nonlinear structure, with a multipresentation of the peptide epitopes on the saccharide chain.

| Sample | MenC saccharide | Saccharide/peptide (mol sialic acid res/mol peptide) |
|---|---|---|
| P32TT-MenC **4a** | polysaccharide | 25.8 |
| P30TT-MenC **4b** | polysaccharide | 25.6 |
| Met6-MenC **4c** | polysaccharide | 26.9 |
| Gap1-MenC **4d** | polysaccharide | 23.0 |
| Hpw1-MenC **4e** | polysaccharide | 26.3 |
| Eno1-MenC **4f** | polysaccharide | 24.7 |
| Fba-MenC **4g** | polysaccharide | 22.2 |

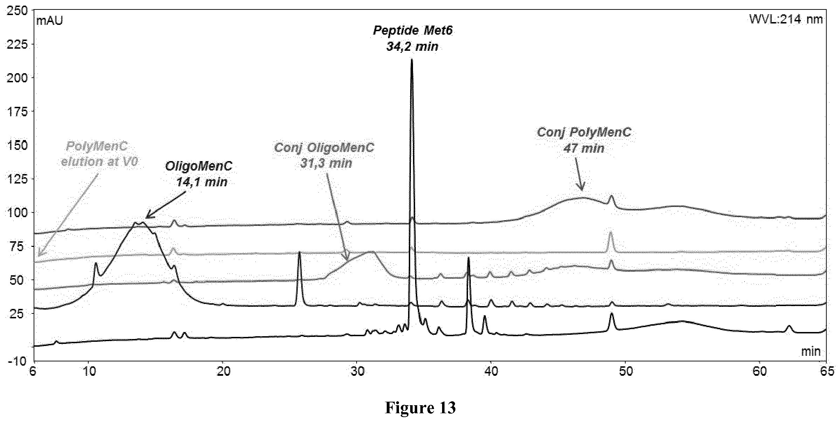

With this approach, seven linear conjugates were obtained with a ratio of approximately 1:1 oligoMenC:peptide and seven nonlinear conjugates were obtained with multiple copies of the same peptide epitope on each polyMenC saccharide chain. HPLC profiles confirmed their structure, evidencing a different interaction with C18 column between oligoMenC conjugates and polyMenC conjugates, due to different ratios of saccharide-peptide (Figure 13). Thus oligoMenC conjugates and polyMenC conjugates have different presentations of peptide T-cell epitopes.

### Immunological evaluation of the first set of conjugates

The prepared conjugates described above were tested in immunological studies in mice.

The immunogenicity was evaluated in Balb/c mice. Groups of eight mice were used. The conjugates were formulated with Aluminium Hydroxide as adjuvant. They were formulated alone or in mixture with a dosage of 1 µg or 0.2 µg in terms of saccharide (Table 5); a group of mice vaccinated with the mixture of polyMenC conjugates without adjuvant was also included. OligoMenC-CRM and oligoMenC-TT conjugates were used as positive controls; PBS plus adjuvant was used as a negative control. The conjugates were administered subcutaneously at day 1, 14 and 28. Vaccinated and control animals were bled 14 days after the second and the third conjugate injection.

| Group | Antigen name | Adjuvant | Antigen dose (based on saccharide content) |
|---|---|---|---|
| 1 | PBS | Alum | - |
| 2 | Peptides + polyMenC | Alum | 0.2 µ + 1 µg |
| 3 | Met6-oligoMenC | Alum | 1 µg |
| 4 | Met6-polyMenC | Alum | 1 µg |
| 5 | Gap1-oligoMenC | Alum | 1 µg |
| 6 | Gap1-polyMenC | Alum | 1 µg |
| 7 | Hpw1-oligoMenC | Alum | 1 µg |
| 8 | Hpw1-polyMenC | Alum | 1 µg |
| 9 | Eno1-oligoMenC | Alum | 1 µg |
| 10 | Eno1-polyMenC | Alum | 1 µg |
| 11 | Fba-oligoMenC | Alum | 1 µg |
| 12 | Fba-polyMenC | Alum | 1 µg |
| 13 | CRM 197-oligoMenC | Alum | 1 µg |
| 14 | TT-oligoMenC | Alum | 1 µg |
| 15 | Mix 5 oligoMenC | Alum | 1 µg each (5 µg tot) |
| 16 | Mix 5 polyMenC | Alum | 1 µg each (5 µg tot) |
| 17 | Mix 5 oligoMenC | Alum | 0.2 µg each (1 µg tot) |
| 18 | Mix 5 polyMenC | Alum | 0.2 µg each (1 µg tot) |
| 19 | Mix 5 polyMenC | None | 1 µg each (5 µg tot) |

First, the pool of sera post third immunization by ELISA assay was analysed to determine the content of IgG anti-polyMenC. None of the oligoMenC conjugates or polyMenC conjugates were able to induce IgG response to the saccharides.

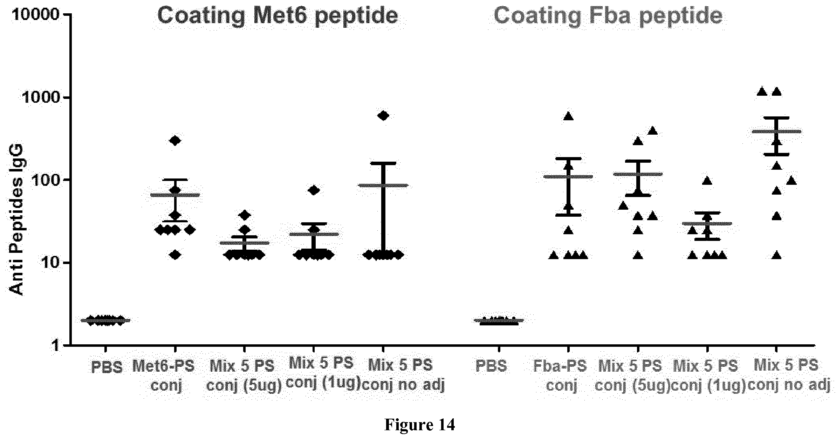

It has been reported that the administration of glycopeptides can also induce an IgG response against the peptide moiety, the IgG anti-peptide response was also analyzed. After sera analysis by ELISA assay, the only IgG responses against peptide have been induced by polyMenC conjugated to Met6 and Fba, alone or in mixture (Figure 14), with and without adjuvant.

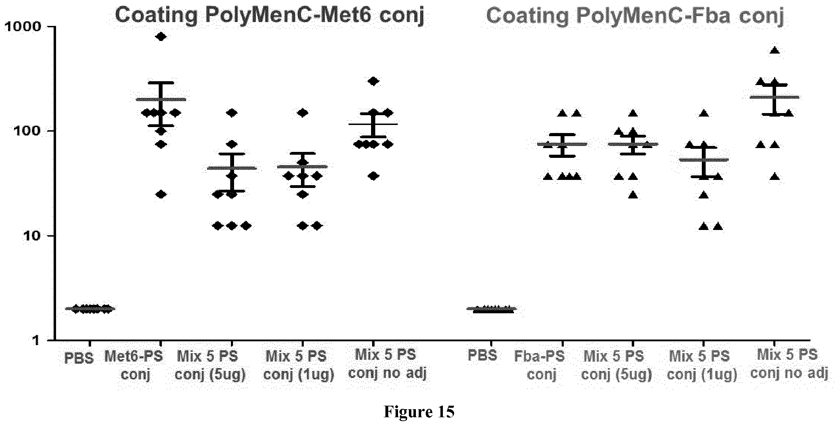

These two conjugates were also able to induce IgG titers against the respective glycopeptides as shown in Figure 15.

From the screening of these T-cell epitope with C-terminul orientation, IgG response to the conjugates were only observed against the Fba and Met6 peptides (epitopes of cell wall proteins of *Candida albicans*), indicating a shift of the immune response to recognizing only the peptide moiety. *Influence of peptide orientation, peptide loading on saccharide chain, linker length and multipresentation of different peptides: physico-chemically characterization and immunological evaluation in mouse model*

A further study was performed to evaluate additional parameters that may influence the immunogenicity of glycopeptides.

Starting from the previous examples, only meningococcal polysaccharide serogroup C in conjugates were tested.

Several features were tested: peptides linked at the *N-*terminus versus peptides linked at the *C-*terminus; a higher density of peptides on the saccharide chain; different linker length to conjugate peptides with polyMenC reducing the distance between B-cell and T-cell epitope; multipresentation of different peptides on the same saccharide chain. Furthermore, also synthetic T-cell epitope peptides were tested, including P2TT (another peptide epitope derived from tetanus toxin and used N19 protein) and the PV1 (a peptide T-cell epitope derived from the VP1 protein of poliovirus type 1) (table 6).

| Peptides | Source | Sequence [SEQ ID NO] | Linked at | Linker |
|---|---|---|---|---|
| Fba | *Candida albicans* | YGKDVKDLFDYAQE [6] | C-Terminus | -G-G-Cys-SH |
| Fba | *Candida albicans* | YGKDVKDLFDYAQE [6] | N-Terminus | HS-CH₂-CH₂-CO-NH- |
| P2TT | Tetanus toxin | QYIKANSKFIGITE [8] | C-Terminus | -G-G-Cys-SH |
| P2TT | Tetanus toxin | QYIKANSKFIGITE [8] | N-Terminus | HS-CH₂-CH₂-CO-NH- |
| PV1 | Poliovirus | KLFAVWKITYKDT [9] | C-Terminus | -G-G-Cys-SH |
| PV1 | Poliovirus | KLFAVWKITYKDT [9] | N-Terminus | HS-CH₂-CH₂-CO-NH- |

In this study, a linker equipped with a free thiol group was introduced at the *C*-terminus or *N-*terminus of selected peptides inducing a different spatial orientation of them.

### Glycopeptides with higher peptide density

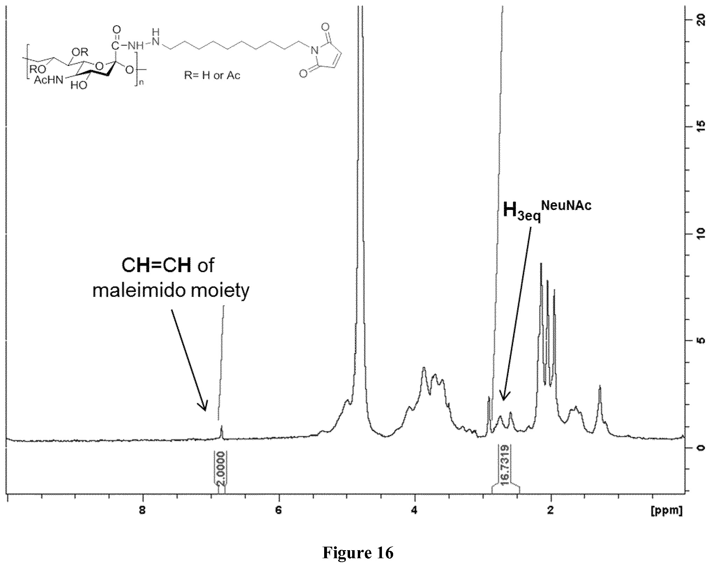

To enhance the density of peptides on saccharide chain, the derivatization degree of polysaccharide chain was increased using the same linker KMUH to introduce about 1 mol of linker per about 16 mol of saccharide as confirmed by ¹H NMR (Figure 16).

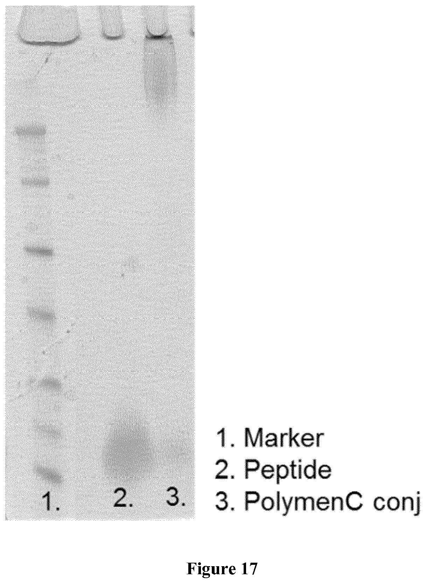

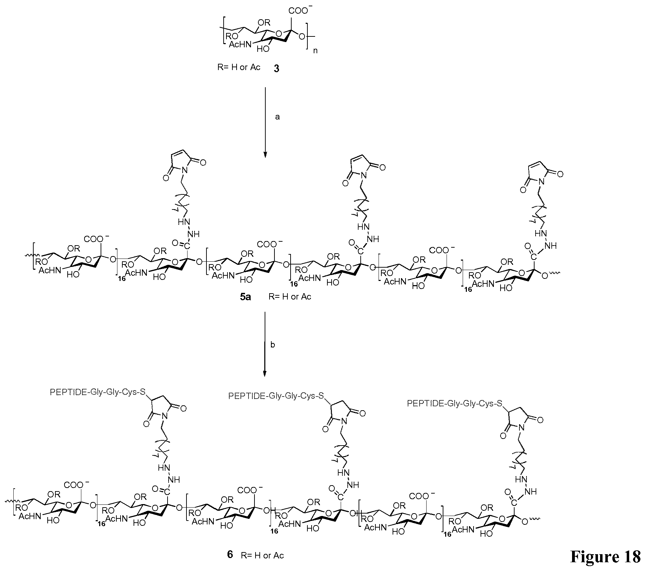

The subsequent conjugation reactions with each peptide (*N*- and *C*-terminus) were shown to have been complete using SDS-Page 4-12% Bis-Tris (see, e.g., Figure 17) after one night at r.t., obtaining nonlinear conjugates with a higher loading of epitope peptides on saccharide chain to test in immunological studies (see Figure 18).

The conjugates were purified by vivaspin 30 kDa to remove free peptide. Only the conjugate with PV1 linked at the *C*-terminus was not recovered. During purification, a gel was formed making it difficult to re-solubilize. This problem was likely due to the increased derivatization of saccharide with a longer lipophilic linker KMUH and subsequent introduction of a peptide with several hydrophobic amino acids.

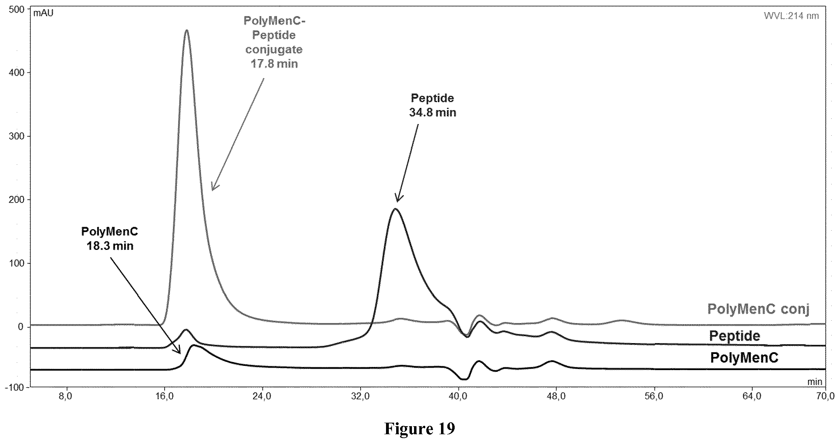

After purification, the glycopeptides were characterized by HPLC-SEC using a Superdex Peptide column. Only one representative example is provided (the PV1 peptide *N*-terminus); all conjugates showed similar profiles (Figure 19).

The C18 jupiter column was not suitable for these conjugates because the higher hydrophobic interactions with the column due to the density of peptides on saccharide chain being increased. Finally the saccharide content was determined by sialic acid assay and the protein content by micro BCA assay or by Lowry assay for PV1 *C*- and *N*-terminal linkage; these data confirmed the introduction of 1 mol of peptides per about 16 mol of saccharide (see Table 7).

| Sample | MenC Saccharide | Sacc/Peptide (mol sialic acid res/mol peptide) |
|---|---|---|
| Fba C-term polyMenC **6a** | Polysaccharide | 14.6 |
| Fba N-term polyMenC **6b** | Polysaccharide | 18.6 |
| P2TT C-term polyMenC **6c** | Polysaccharide | 12.5 |
| P2TT N-term polyMenC **6d** | Polysaccharide | 21.7 |
| PV1 N-term polyMenC **6e** | Polysaccharide | 11.2 |

### Conjugates with a shorter linker: BMPH

Another bifunctional linker was also used to derivatize polysaccharide that was shorter than KMUH in order to reduce the distance between saccharide antigen and T-cell epitope peptides. BMPH (N-β-Maleimidopropionic acid hydrazide-TFA) was used as the linker to introduce maleimido groups on carboxyl groups of saccharide chain using EDAC chemistry, reducing the distance between saccharide and T-cell epitope of only two carbon atoms.

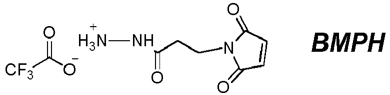

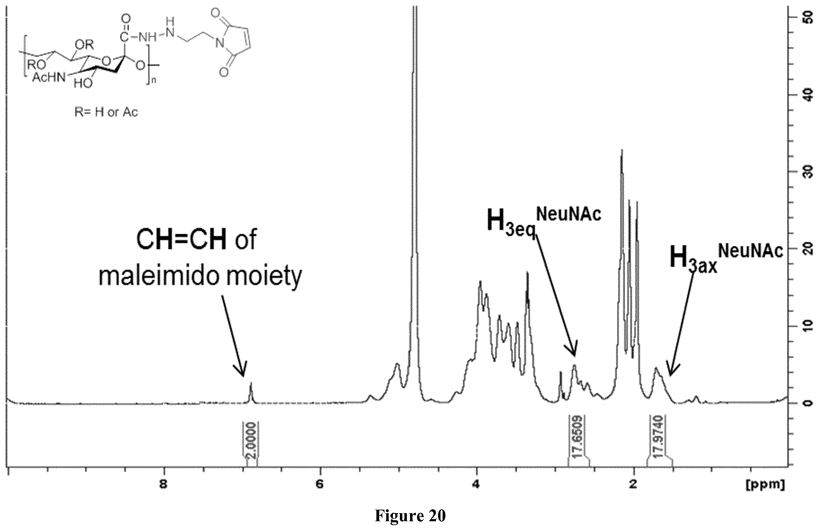

After the derivatization of polysaccharide MenC with BMPH linker, we introduced 1 mol of maleimido group per about 17 mol of saccharide, allowing the preparation of glycopeptides with a higher peptide density also using this linker (Figure 20).

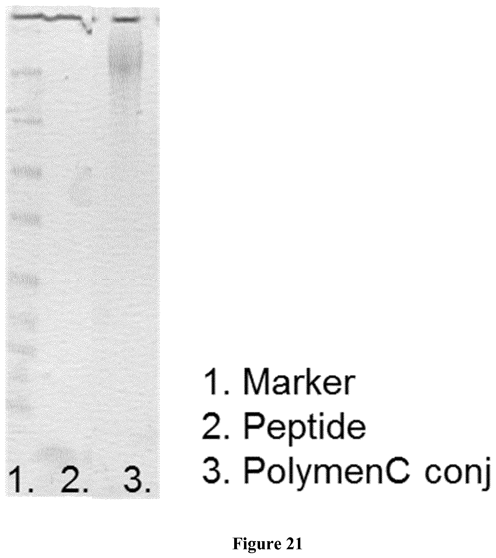

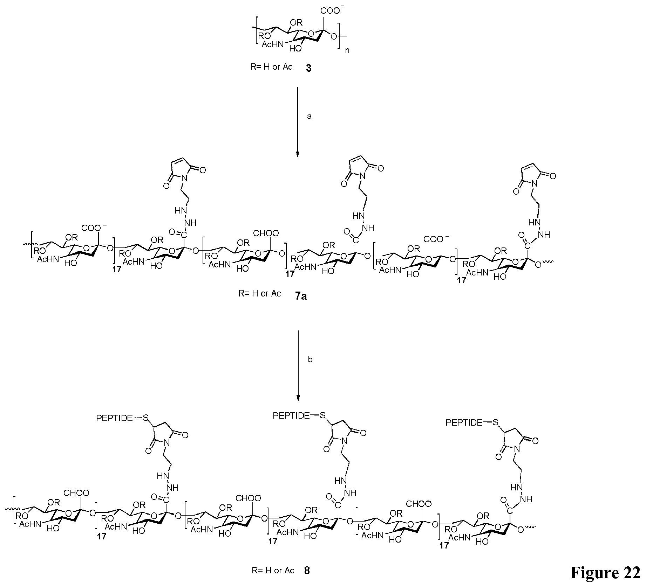

Once derivatized, the conjugation reactions with each peptide were shown to have been completed over one night at r.t., as evidenced by SDS-Page 4-12% Bis-Tris (Figure 21 and Figure 22).

The conjugates were purified by vivaspin 30 kDa to remove free peptide; except for conjugates with PV1 linked at the *C*-terminus and *N-*terminus which were purified by size exclusion chromatography using a pre-packed column Superdex Peptide. After purification the saccharide and protein content were determined respectively by sialic acid and micro BCA or Lowry colorimetric assays; these data confirmed the introduction of 1 mol of peptide per about 17 mol of saccharide (Table 8).

| Sample | MenC Saccharide | Sacc/Peptide (mol sialic acid res/mol peptide) |
|---|---|---|
| Fba C-term polyMenC **8a** | Polysaccharide | 14.6 |
| Fba N-term polyMenC **8b** | Polysaccharide | 15.8 |
| P2TT C-term polyMenC **8c** | Polysaccharide | 12 |
| P2TT N-term polyMenC **8d** | Polysaccharide | 9.8 |
| PV1 N-term polyMenC **8f** | Polysaccharide | 9 |
| PV1 N-term polyMenC **8g** | Polysaccharide | 10.9 |

Thus six glycopeptides combining different characteristics were obtained: higher density peptide, shorter distance between saccharide antigen and epitope peptides and use of peptides with different spatial orientation.

### Conjugates with multipresentation of different peptides on the same saccharide chain

Different peptides were conjugated to the same polyMenC saccharide to assess whether the simultaneous presence of different peptides on saccharide chain could induce an immune response against MenC. The following peptides were used: Fba, Met6, and Hpw1 (*C*-terminus) whose structures were reported in table 2. These peptides were selected on the basis of distinctive ¹H NMR signals, not common to each peptide, allowing after their conjugation an easy assessment by ¹H NMR experiments.

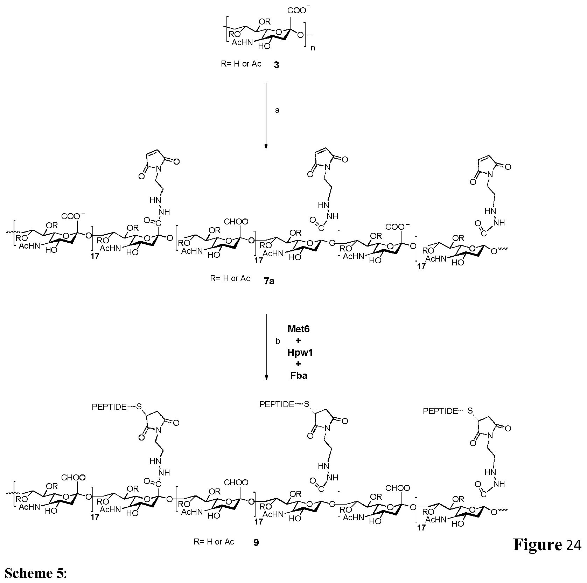

These peptides were conjugated to polyMenC after introduction of maleimido moiety with BMPH linker (compound **7a** in Figure 24).

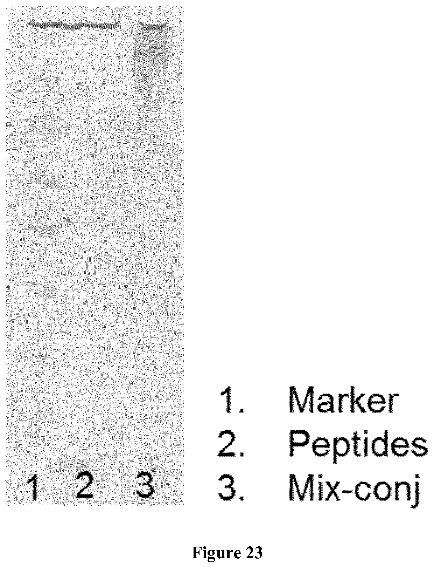

The reaction of conjugation was performed in one night at r.t. as evidenced by SDS-PAGE 4-12% Bis-Tris (Figure 23). The reaction has been carried out adding the mixture of peptides to solution of polysaccharide (Figure 24).

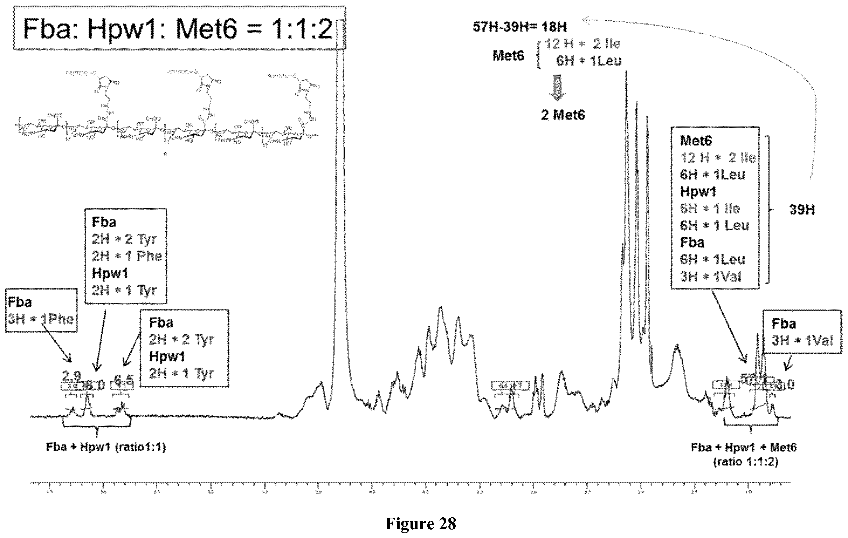

The conjugate was purified by vivaspin cut off 30 kDa to remove the free peptides and ¹H NMR spectrum of conjugate confirmed the introduction of three different peptides (Figure 28).

To differentiate between Met6, Hpw1 and Fba on saccharide chain we used ¹H NMR analysis analyzing the ¹H NMR spectra of each peptide and by assessing some representative signals.

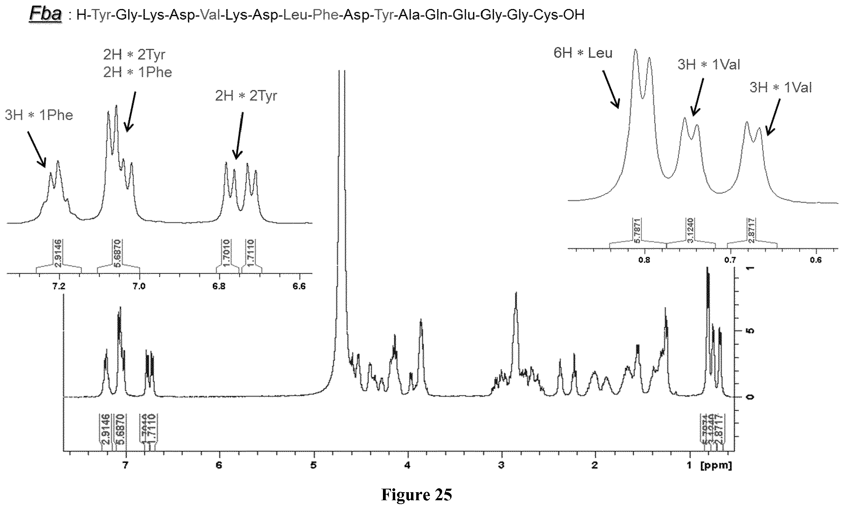

As shown in Figure 25 in the amino acid sequence of Fba there are 2 Tyr (8H) and 1 Phe (5H) whose protons showed signals between 6 and 7.5 ppm, 1 Leu (6H) and 1 Val (6H) with signals around 1 ppm.

In the structure of Met6 peptide, we identified 2 Ile (12H) and 1 Leu (6H) with signals at about 1 ppm (Fig22).

For Hpw1 peptide, there are 1 Tyr (4H) whose protons were at about 7 ppm, 1 Ile (6H) and 1 Leu (6H) with signals at about 1 ppm (Fig23).

From ¹H NMR spectrum of glycopeptide we determined that the ratio between the different peptides was Fba: Hpw1: Met6 = 1:1:2.

As shown in Figure 28 by integration of aromatic protons we identified a ratio 1:1 between Fba and Hpw1. The assignement of the protons of Val, Ile, Leu allowed to define the amount of Met6 respect to Fba and Hpw1. The integration of signals showed that we introduced 2 mol of Met6 peptide respect to Fba and Hpw1 peptides (Figure 28).

The saccharide content of pure conjugate was estimated by sialic acid colorimetric assay, while the content in protein was determined by micro BCA colorimetric assay.

The immunogenicity of this conjugates with different synthetic peptides conjugated to the same polyMenC chain was compared to the conjugates with just one type of peptide conjugated to saccharide chain.

PolyMenC-_{BMPH} **7a** was used to prepare conjugates respectively with Met6 and Hpw1; the conjugate Fba-MenC **8a** was already prepared (Table 8).

| Sample | MenC Saccharide | Sacc/Peptide (mol sialic acid res/mol peptide) |
|---|---|---|
| Fba C-term polyMenC **8a** | Polysaccharide | 14.6 |
| Fba C-term, Met6, Hpw1-C-term polyMenC **9** | Polysaccharide | 11.8 |
| Met6 C-term polyMenC **9a** | Polysaccharide | 11.3 |
| Hpw1 C-term polyMenC **9b** | Polysaccharide | 13 |

### Immunological evaluation of the second set of conjugates

The conjugates were tested in a second immunological study in mice. The immunogenicity was evaluated in Balb/c mice using groups of eight mice. The conjugates were formulated with Aluminium hydroxide as the adjuvant, with a dose of 1 µg in (based upon saccharide content). OligoMenC-CRM was used as positive control; PBS plus adjuvant was used as negative a control. The conjugates were subcutaneously administered at day 1, 14 and 28. Vaccinated and control animals were bled 14 days after the second and the third conjugate injection.

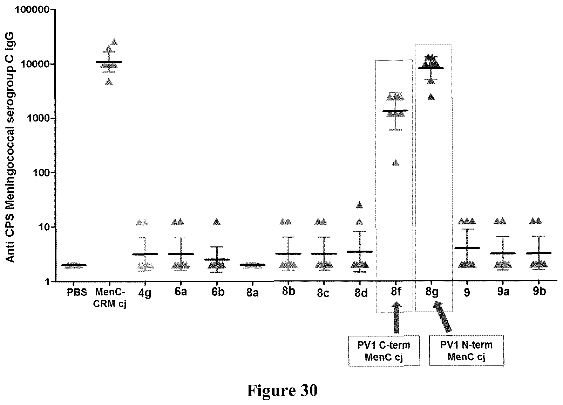

The sera on post 3 was analyzed by ELISA assay to determine the content of IgG anti-polyMenC. All the conjugates prepared with Fba, P2TT, Met6 and Hpw1 peptides only showed low immunogenicity in mice. Moreover, the evaluation of different parameters of these synthetic peptides (peptides with *N*-orientation, a higher density of peptides on the saccharide chain, linker with different length, multipresentation of different peptides on the same chain of saccharide) did not lead to significant differences owing to the low immunogenicity observed. These selected peptides were likely only poorly recognized as T-cell epitopes and therefore were good candidates by themselves for replacing the entire carrier protein (Figure 30).

One T-cell epitope peptide, the synthetic PV1 peptide derived from protein *polio virus* type 1, was found to be a good carrier for the Meningococcal polysaccharide serogroup C. As shown in the

Figure 30, conjugates **8f** and **8g** prepared with PV1 peptide were highly immunogenic, inducing significant IgG titers against polyMenC in comparison to the controls mice, immunized with PBS plus adjuvant (P value = 0.0007).

In particular, PV1 peptide with linked at the *N-*terminus induced IgG levels comparable with that induced by conventional oligoMenC-CRM conjugate (no significant differences between MenC-CRM conjugate *vs* MenC-PV1 *N-*term conjugate, P value = 0.5625). More importantly, as shown in the following table 14, the PV1 peptide conjugate was able to induce a serum bactericidal response that was as strong as the conventional oligoMenC-CRM conjugate.

| **Rabbit SBA titers (MenC11) >8192** | |
|---|---|
| | **post3** |
| PBS | <16 |
| MenC-CRM | >8192 |
| Fba C-term-MenC (KMUH) conj Lot1 **4g** | <16 |
| Fba C-term-MenC (KMUH) conj **6a** | <16 |
| Fba N-term-MenC (KMUH) conj **6b** | <16 |
| Fba C-term-MenC (BMPH) conj **8a** | <16 |
| Fba N-term-MenC (BMPH) conj **8b** | <16 |
| P2TT C-term-MenC (BMPH) conj **8c** | <16 |
| P2TT N-term-MenC (BMPH) conj **8d** | <16 |
| PV1 C-term-MenC (BMPH) conj **8f** | 2048 |
| PV1 N-term-MenC (BMPH) conj **8g** | 8192 |
| Fba, Met6, Hpw1-MenC (BMPH) conj **9** | <16 |
| Met6 C-term-MenC (BMPH) conj **9a** | <16 |
| Hpw1 C-term-MenC (BMPH) conj **9b** | <16 |

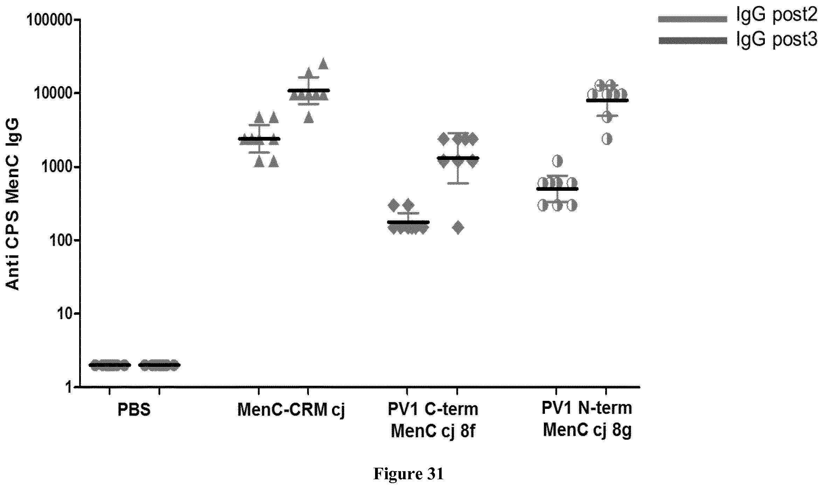

Analysis of sera after second and third immunization (Figure 31) showed a booster effect for both conjugates (significant differences between post2 and post3 for PV1 *C*-term conjugate ** P = 0.0036 and for PV1 *N-*term conjugate *** P = 0.0008).

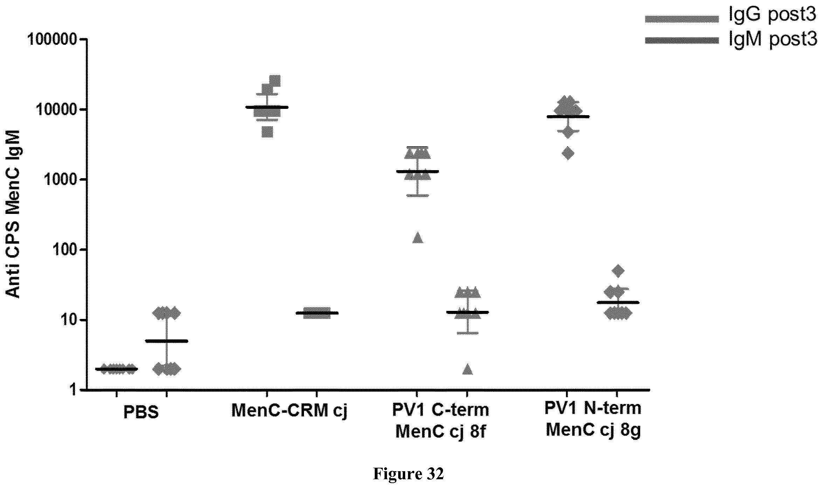

Moreover, further sera analysis on post3 (Figure 32) showed that the content of anti-MenC IgM induced by the conjugates was very low. This data evidenced that an IgM to IgG isotype shift occurred and the glycopeptides with PV1 peptides were able to induce a memory cell response.

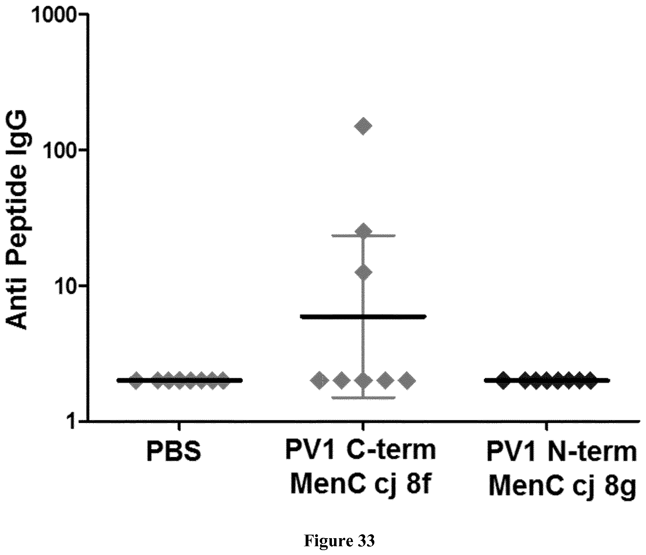

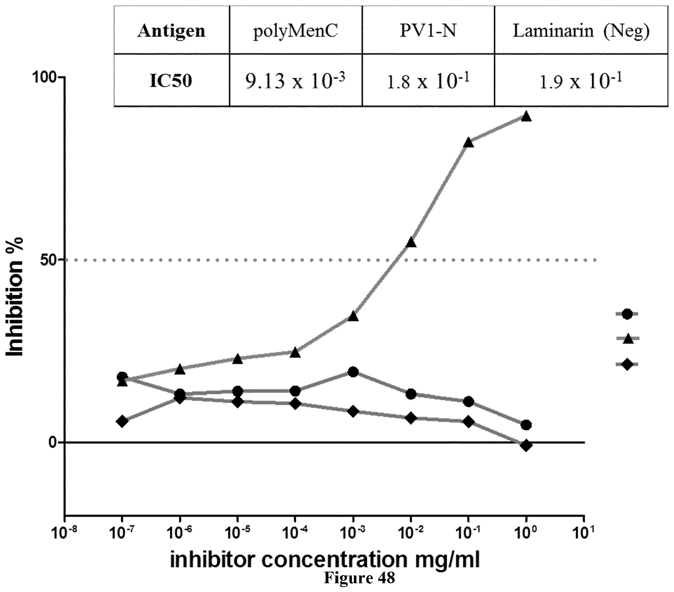

An additional important observation was that IgG titers against this peptide were found very low for PV1 *C*-terminus and almost non-existent for PV1 *N-*terminus (Figure 33), showing that this peptide could be a good candidate as a carrier in substitution of whole carrier protein. Figure 48 also shows the different IgG subtypes induced by the PV1 peptide nonlinear conjugates.

### Physico-chemically characterization and immunogenicity evaluation in mouse model of glycopeptides prepared with Ova peptide as carrier

### Screening of different B-cell epitope to test Ova peptide as carrier

An additional example involves the use of the synthetic peptide from Ovalbumin (OVA) as carrier protein. Starting from the literature where Ova peptide was shown to be a good carrier for the polysaccharide GBS type III, ability to substitute the whole carrier protein also tested in combination with other B-cell antigens such as Meningococcal polysaccharide serogroup W-135, Y and C.

In this example, the synthetic Ova peptide was linked at its C-terminus.

| Peptide | Source | Amino Acid Sequence | Linker |
|---|---|---|---|
| Ovap | Ovalbumin (OVA) | N-acteyl-ISQAVHAAHAEINEAGR- | -E-S-G-K-NH₂ |
| Ovap | Ovalbumin (OVA) | N-acteyl-ISQAVHAAHAEINEAGR- | -G-G-Cys-SH |

As reported in the table, a synthetic Ova peptide with 2 different linkers at *C*-terminal was used:
- linker E-S-G-K with free amino group to prepare conjugates with Meningococcal polysaccharide serogroups W-135 and Y (reductive ammination after oxidation of sialic acid), and with Meningococcal polysaccharide serogroup C (after derivatization of carboxyl groups).
- linker G-G-C with free thiol group to prepare conjugates using polyMenC derivatized with maleimido groups (compound **7a** and **5a** used in previous examples), to have a comparison with the previous examples.

### Synthesis of Ova peptide conjugates with Meningococcal Polysaccharide serogroup W-135 and Y.

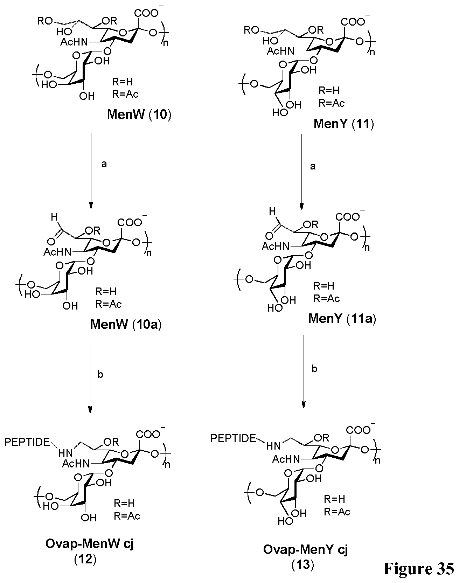

To prepare conjugates with polyMenW-135 and Y, an oxidation reaction of sialic acid (30% oxidation respect to repeating unit) was performed, followed by reductive ammination with Ova peptide in presence of sodiumcyanoborohydride (Figure 35).

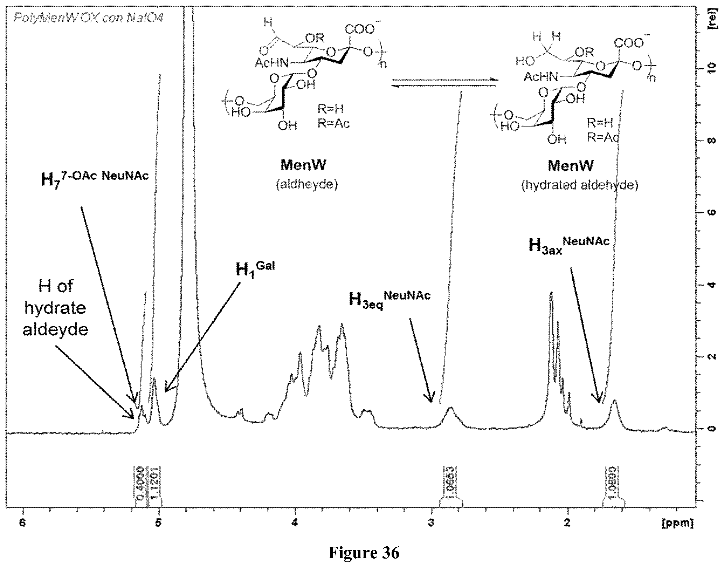

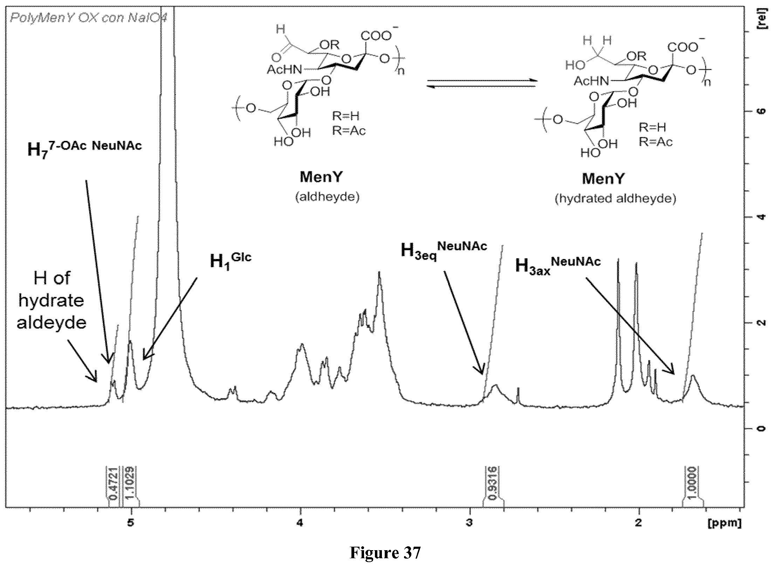

At the first we performed oxidation reaction of sialic acid with sodium *meta*-periodate, obtaining a 30% of oxidation degree in agreement with the desired target. The oxidized polysaccharides have been purified by vivaspin cut off 30 kDa and characterized by ¹H NMR to estimate the percentage of oxidation (Figures 36-37)

As shown in Figure 36-37, percentage of oxidation was estimated, considering the signal relative to the proton of aldehyde hydrate; in fact, proton NMR signals, expected both for the aldehyde and the hydrate aldehyde forms, were revealed only for the hydrate aldehyde form at 5.15 ppm, since the experimental condition used to collect the experiment moved the equilibrium toward it.

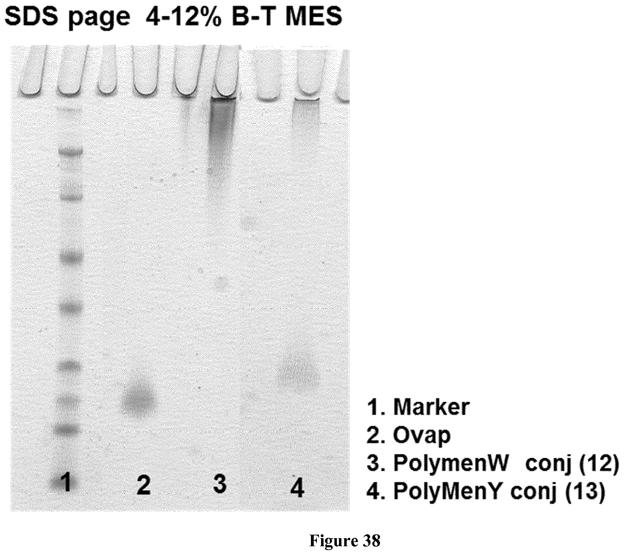

After oxidation of sialic acid, the following conjugation reaction with peptide were performed by reductive amination in presence of sodium cyanoborohydride. Both reactions were carried out at 37°C for 5 days and they worked properly as evidenced by SDS-Page 4-12% Bis-Tris (Figure 38).

Both conjugates were purified by vivaspin cut off 30 kDa to remove the excess of peptide. After purification the two conjugates were characterized in terms of saccharide and peptide content using respectively resorcinol and Lowry colorimetric assays.

These data showed that about 1 mol of peptide per 4 mol of sialic acid have been introduced (Table 12), obtaining glycopeptides with a high density of T-cell epitope on saccharide chain.

| Sample | Sacc/Peptide (mole saccharide/mol peptide) |
|---|---|
| Ovap-MenW conjugate **12** | 4 |
| Ovap-MenY conjugates **13** | 3.6 |

### Synthesis of Ova peptide conjugates with Meningococcal Polysaccharide serogroup C.

Conjugates of Ova peptide were prepared with Meningococcal polysaccharide serogroup C using two different synthetic approaches:
1- Ova peptide with G-G-C linker at *C*-terminus was conjugated to polyMenC derivatized with maleimido groups (compound 7a and 5a);
2- Ova peptide with E-S-G-K linker at *C*-terminus was conjugated to carboxyl groups of polyMenC using EDAC chemistry.

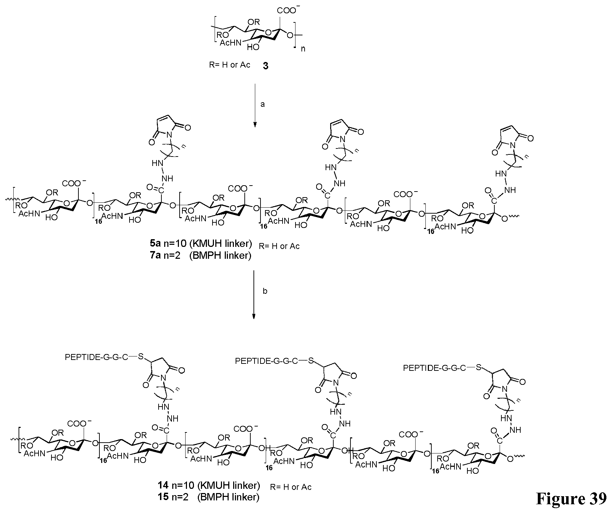

For the first approach, polyMenC derivatized with KMUH linker (5a) or with BMPH (7a) was used, with an introduction of 1 mol of maleimido group per about 16-17 mol of sialic acid. Thus free thiol group on peptide was able to react with maleimido moiety of saccharide chain and the reactions of conjugation have been performed as described previously (Figure 39).

The two reactions were conducted over night at room temperature, as evidenced by SDS-Page 4-12 % Bis-Tris (Figure 33), obtaining two conjugates with two different linker (KMUH and BMPH) between T-cell epitope and B-cell epitope (**14** and **15**).

The glycopeptides were purified by vivaspin cut off 30 kDa to remove free peptide.

The pure conjugates were characterized in terms of saccharide and peptide content using respectively resorcinol and Lowry colorimetric assays. These data showed the introduction of 1 mol of linkers per about 15-18 mol of sialic acid (table 13).

| Sample | Sacc/Peptide (mole saccharide/mol peptide) |
|---|---|
| Ovap GGC-MenC (KMUH) conjugate **14** | 15.2 |
| Ovap GGC-MenC (BMPH) conjugates **15** | 18.3 |

For the second approach, the conjugate polyMenC-Ovap using the peptide with linker E-S-G-K, the condensation reaction between free amino group of peptide and carboxyl groups of MenC was used to obtain an amide bond.

The reaction was performed in presence of EDAC and Sulfo-NHS to activate the carboxyl groups on saccharide for the reaction with amino group of peptide (Scheme 8).

As the first step, a mixture of EDAC and sulfo-NHS (one equivalent respect to saccharide mol) was added to a solution of polysaccharide in MES 0.03 M pH 6. The reaction was carried out under gently stirring in the dark for 15 minutes and then the solution of Ova peptide (1 eq) in water was added.

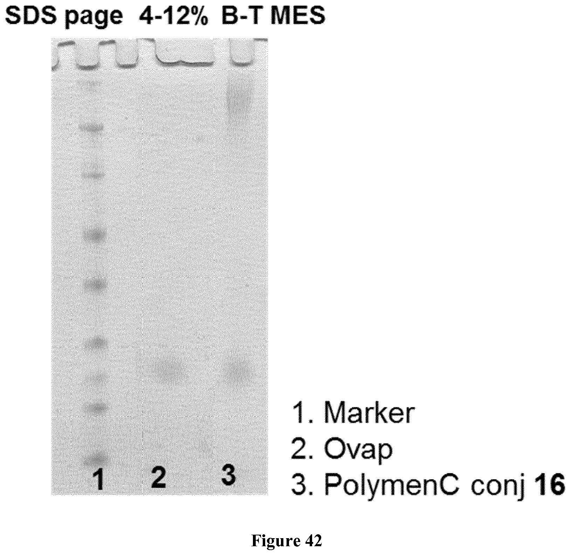

After one night at room temperature the SDS-Page showed that the reaction worked (Figure 42).

The conjugate **16** has been purified by vivaspin cut off 30 kDa and the pure conjugate has been characterized in terms of saccharide content and peptide content. The Ovap-MenC conjugate **16** had a low ratio of Sacc/Peptide (mol saccharide/mol peptide) of 80. Attempts to increase the peptide loading on MenC were unsuccessful, and in fact increasing the amount of EDAC caused the polysaccharide to form a sort of gel that was not re-solubilized due to side-reactions. Increasing the amount of peptide caused problems during the purification step.

Thus we used this conjugate in immunological studies in mice, also if the loading of peptide was low respect to **12, 13, 14** and **15** conjugates.

### Immunological evaluation of conjugates with Ova peptide as carrier

The conjugates with Ova peptide as carrier were tested in immunological studies in mice. The immunogenicity was evaluated in Balb/c mice using groups of eight mice. The conjugates were formulated with aluminium hydroxide as adjuvant, with a dose of 1 µg in (based upon saccharide content). oligoMenC-CRM, oligoMenW-CRM and oligoMenY-CRM were used as positive control; PBS plus adjuvant was used as a negative control. The poly conjugates were subcutaneously administered at day 1, 14 and 28. Vaccinated and control animals were bled 14 days after the second and the third conjugate injection.

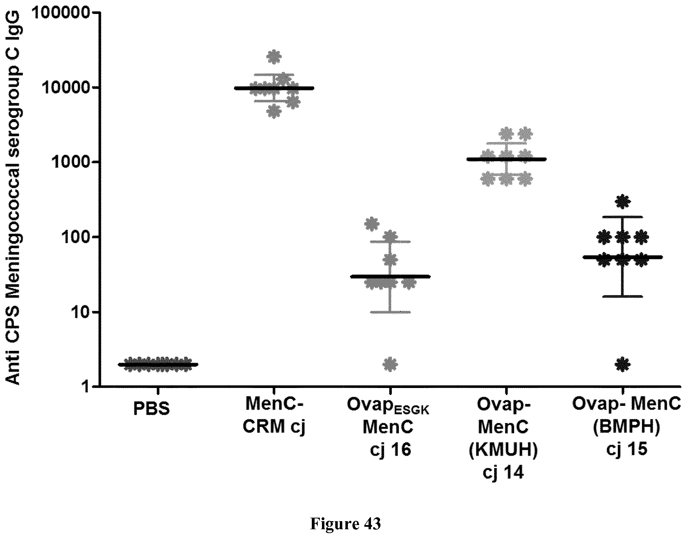

ELISA assay of sera post 3 showed that only glycopeptides with Ova peptide conjugated to Meningococcal polysaccharide serogroup C were found to be immunogenic in mice in comparison to the controls mice, immunized with PBS plus adjuvant (P value < 0.05), as reported in Figure 43. In particular the Ovap-MenC conjugate with Ova peptide coupled using KMUH linker was the most immunogenic (P value= 0.006 respect to the controls mice).

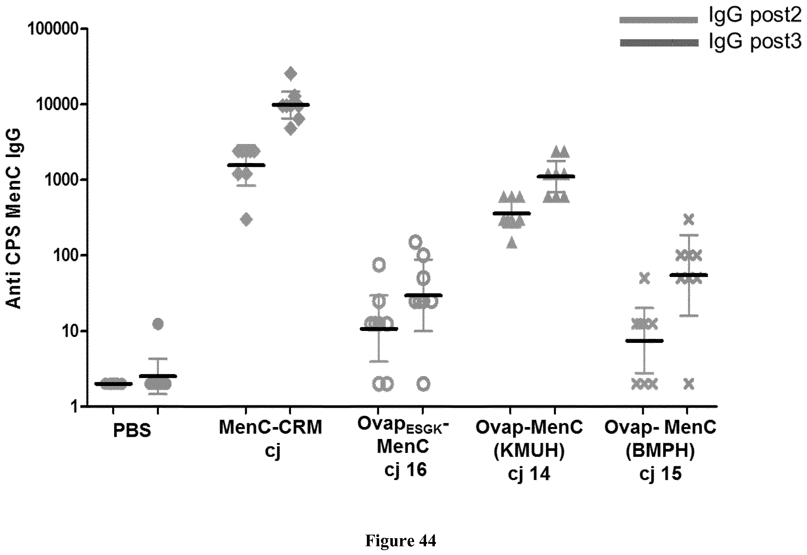

The analysis of anti-saccharide IgG on post2 in comparison with post3 evidenced that for the conjugates **14** and **15** there was a booster effect (P value < 0.05), in particular for Ovap-MenC (KMUH linker) with a P value of 0.0033. In contrast, the conjugate Ovap(ESGK)-MenC **(16)** did not produce a booster effect (P value 0.0594) (Figure 44).

However, all conjugates with Ova peptide induced a lower anti-MenC IgG response compared to MenC-CRM conjugate (significant differences between MenC-CRM vs Ovap-MenC conjugates ***P = 0.0009).

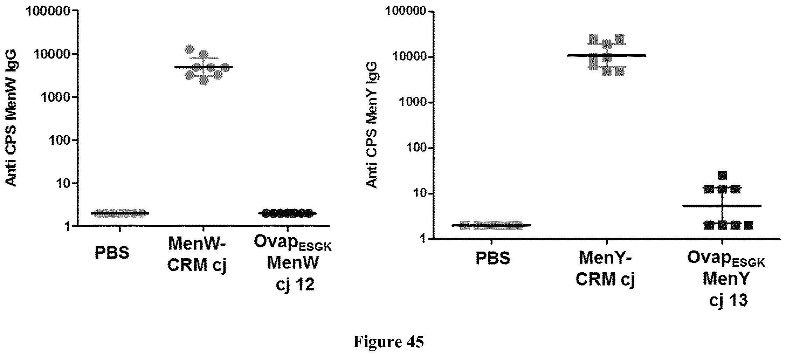

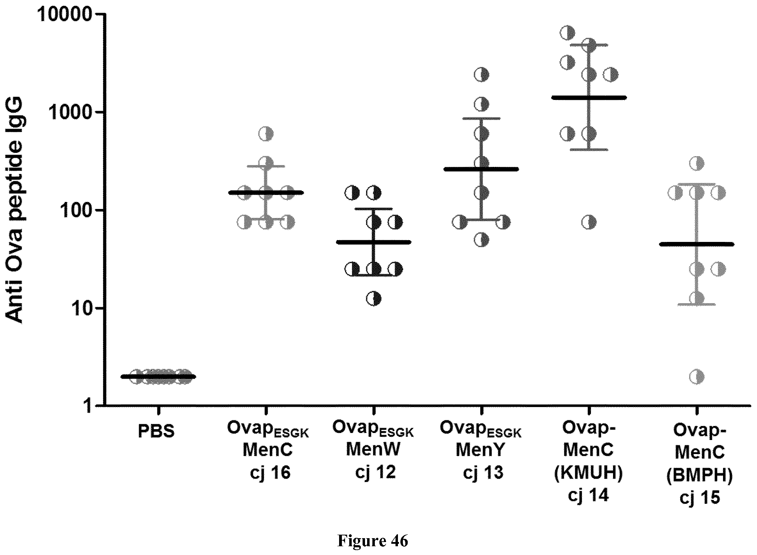

The other conjugates with Meningococcal serogroup W and Y polysaccharides (**12** and **13**), were only poorly immunogenic or not observably immunogenic (Figure 45). As reported in Figure 45, post3 sera did not induce anti-saccharide IgG titers; there were no difference in comparison to the controls mice, immunized with PBS plus adjuvant (P>0.05). Anti-Ovap IgG titers, after third immunization, showed that all glycopeptides induced antibody response against peptide even if the titers were not very high; in particular the glycopeptide with polyMenC conjugated with KMUH linker induced the highest anti-peptide IgG titers as reported in Figure 46.

### Material and Methods Used in the Examples

### Preparation of Meningococcal serogroup C, Wand Y Oligo and Polysaccharides

### Introduction of primary amino groups into the reducing end of meningococcal serogroup C oligosaccharides by reductive amination

The oligosaccharide **1** was treated by reductive amination to introduce an amino group at the reducing end of the saccharide. This reaction was performed in organic solvent, so the oligosaccharide was exchanged with tetrabutylammonium bromide (TAB) as counter-ion. To introduce this counter-ion, we performed an ionic exchange chromatography using CAPTO S resin, equilibrated with TAB 0.7M, and eluted the oligosaccharide from the column with TAB⁺ as a counter-ion. Then the dried product (20 mg) was dissolved in 10% DMSO, 90% methanol at a final concentration of 5mg/ml of sialic acid. Ammonium acetate (final concentration of 50 mM) and sodium cyanoborohydride (final concentration of 10 mM) was then added. The reaction mixture was incubated for 24-72 hours at 50°C. The excess methanol was then removed by a rotary evaporator under vacuum until the volume was less than or equal to twice the amount of DMSO added prior to reductive amination. After that, the reaction mix was diluted with NaCl at 0.5 M to obtain a solution with 20% of MeOH. The saccharide was then purified by size exclusion chromatography using a G10 resin to remove the reductive amination reagents from the aminated oligoMenC. Finally, the counter-ion TAB was again exchanged with CAPTO S resin equilibrated with NaCl 1 M, so the oligosaccharide **1a** was eluted from the column with Na⁺ as a counter-ion. The amount of introduced amino groups was estimated by colorimetric assay [Habeeb AF Anal Biochem 1966, 14, 328-38], which indicated that the amination was about 50% (as subsequently confirmed by its ¹H NMR spectrum).

### Derivatization of oligoMenC-NH₂ with N-ε-Maleimidocaproyl-oxysulfosuccinimide ester (sulfo-EMCS)

After reductive amination, the oligosaccharide **1a** was solubilized in a mixture of H₂O-DMSO 1:9, then 5 eq. of Et₃N were added followed by the addition of 10 eq. of sulfo-EMCS linker. The reaction was kept at r.t. under gently stirring for 2 hours. The derivatized oligosaccharide **1b** was then separated from the reagents by precipitation with 1:4 acetone, followed by washing of the precipitate with 1:4 acetone and drying under vacuum.

Introduction of maleimido groups on oligosaccharide chain was confirmed by ¹H NMR spectrum.

### Derivatization of polysaccharide MenC with N-k-Maleimidoundecanoic acid hydrazide-TFA (KMUH)

The carboxyl groups of sialic acid of Meningococcal polysaccharide serogroup C **3** were reacted with hydrazide groups on KMUH linker to introduce maleimido moieties on saccharide chain so that the epitope peptides could be linked in a nonlinear form. To perform the reaction, the polysaccharide **3** was solubilized in MES 0.1M pH 4.56 at a final concentration of 2 mg/ml; then EDAC (20 mol%) was added and the reaction was kept under gently stirring in the dark. After 10 minutes, KMUH (20 mol% or 46 mol% to introduce 1 mol of linker per about 35 mol of sialic acid or per about 17 mol of sialic acid, respectively) was added to the mixture and the reaction was carried out at r.t. for 2 hours under gently stirring. Finally the derivatized polysaccharide was purified by tangential flow filtration with 30 kDa cut-off membrane (Sartorius) and the derivatized polysaccharide was recovered in the retentate.

The amount of maleimido groups on saccharide chain was estimated by ¹H NMR spectrum: we introduced 1 mol of maleimido moiety per about 35 mol of sialic acid (**3a**) or 1 mol of maleimido moiety per about 16 mol of sialic acid (**5a**), depending on the amount of linker used in the reaction.

### Derivatization ofpolysaccharide MenC with N-β-Maleimidopropionic acid hydrazide-TFA (BMPH)

The carboxyl groups of sialic acid of Meningococcal polysaccharide serogroup C **3** was reacted with hydrazide groups on BMPH linker to introduce maleimido moieties on the saccharide chain. To perform the reaction, the polysaccharide **3** was solubilized in MES 0.1M pH 4.56 at a final concentration of 2 mg/ml; then EDAC (20 mol%) was added and the reaction was kept in the dark while gently stirring; after 10 minutes BMPH (46 mol%) was added to the mixture and the reaction was carried out at r.t. for 2 hours under gently stirring. Finally the derivatized polysaccharide was purified by tangential flow filtration with 30kDa cut-off membrane (Sartorius) and the derivatized polysaccharide has been recovered in the retentate.

The amount of maleimido groups on saccharide chain was estimated by ¹H NMR spectrum, establishing that we introduced 1 mol of maleimido moiety per about 17 mol of sialic acid (**7a**).

### Oxidation of Meningococcal polysaccharides serogroup W and Y

The sialic acid of polysaccharide MenW **10** and MenY **11** was oxidized to introduce an aldehyde group able to react with amino group of a peptide by reductive amination reaction. Both polysaccharides were solubilized in NaPi 10mM pH 7 at a concentration of 10mg/ml. NaIO₄ 0.1M was added in an amount equal to 30 mol% in order to obtain an oxidation of 30%. The reaction was kept in the dark while gently stirring at r.t. for 2 hours. After this time oxidized polysaccharides **10a** and **11a** were purified by ultrafiltration with Vivaspin system (Sartorius) using a 30kDa cut-off membrane. The oxidation degree of pure compounds was estimated by ¹H NMR spectra, showing 30% oxidation, in agreement with the desired target.

### Preparation of conjugates

### Synthesis of oligoMenC-peptide conjugates (2a, 2b, 2c, 2d, 2e, 2f, 2g)

The reactions of conjugation between oligosaccharide **1b** and synthetic peptides worked properly exploiting the reactivity between maleimido moiety on the saccharide and thiol group on peptides (Table 1). The conjugations were performed with a ratio peptide mol: maleimido mol on saccharide chain of 3:1, in PBS buffer pH 7 (3 mg/ml in terms of peptide) and incubated overnight at r.t. under gently stirring. All the conjugates were purified by size exclusion chromatography using a Superdex Peptide pre-packed column to remove free peptide followed by precipitation with ammonium sulphate to remove free saccharide.

### Synthesis of polyMenC_{KMUH}-peptide conjugates (4a, 4b, 4c, 4d, 4e, 4f, 4g)

The reactions of conjugation between polysaccharide **3a** and synthetic peptides worked properly exploiting the reactivity between maleimido moieties on the saccharide and thiol group on peptides (Table 1). The conjugations were performed with a ratio peptide mol: maleimido mol on saccharide chain of 3:1, in PBS buffer pH 7 (3 mg/ml in terms of peptide) and incubated overnight at r.t. under gently stirring. All the conjugates were purified by ultrafiltration with Vivaspin system (Sartorius) using a 30kDa cut-off membrane.

### Synthesis of poZyMenC_{KMUH}-peptide conjugates (6a, 6b, 6c, 6d, 6e)

The reactions of conjugation between polysaccharide **5a** and synthetic peptides (Table 5) were performed as reported above for polysaccharide **3a**. All conjugates were purified by ultrafiltration with Vivaspin system (Sartorius) using a 30kDa cut-off membrane.

### Synthesis of poZyMenC_{BMPH}-peptide conjugates (8a, 8b, 8c, 8d, 8e, 8f, 8g)

The reactions of conjugation between polysaccharide **7a** and synthetic peptides (Table 5) were performed as reported above for Polysaccharide **3a**. All conjugates were purified by ultrafiltration with Vivaspin system (Sartorius) using a 30kDa cut-off membrane, except that conjugates **8f** and **8g,** with VP1 *C*- and *N-*terminus peptides, were purified by size exclusion chromatography using a superdex peptide pre-packed column.

### Synthesis of polyMenC_{BMPH}-MIX Met6+Fba+Hpw1 peptides conjugate (9)

The reaction of conjugation was carried out using a prepared mixture of the peptides and adding this mixture to polysaccharide **7a**. We performed the reaction with a ratio mol Met6 peptide: mol Hpw1 peptide: mol Fba peptide: mol maleimido on saccharide chain of 1:1:1:1. The reaction was carried out in PBS buffer pH 7 (3 mg/ml in terms of peptide) and incubated overnight at r.t. under gently stirring. The conjugate **9** was purified by ultrafiltration with Vivaspin system (Sartorius) using a 30kDa cut-off membrane.

### Synthesis of conjugates polyMenW-Ovap and polyMenY-Ovap (12 and 13)

The conjugation reactions for the conjugates were performed by reductive amination introducing the amino group of Ovap-_{ESGK} on aldehyde moiety of both oxidized polysaccharides. To a solution of polysaccharide (**10** or **11**) 1 mg/ml in NaPi 10 mM pH7, sodium cyanoborohydride (30 eq.) was added followed by the addition of Ova peptide (3 eq), buffer conjugation NaPi 60Mm NaCl 250mM pH7. Both conjugation reaction were incubated at 37°C for 5 days. The conjugates **12** and **13** were purified by ultrafiltration with Vivaspin system (Sartorius) using a 30kDa cut-off membrane.

### Synthesis of conjugates poZyMenC_{KMUH}-Ovap and polyMenC_{BMPH}-Ovap (14 and 15)

The conjugation reactions were performed as reported above for polysaccharide **3a**. We used respectively the polysaccharide **5a** (derivatized with KMUH linker) to prepare the conjugate **14** and the polysaccharide **7a** (derivatized with BMPH linker) to prepare the conjugate **15.** Both conjugates were purified by ultrafiltration with Vivaspin system (Sartorius) using a 30kDa cut-off membrane.

### Synthesis of conjugate polyMenC-Ovap (16)

To prepare glycopeptide **16** where the peptide Ovap-_{ESGK} was conjugated directly to the saccharide chain, we exploited the condensation reaction between free amino group of peptide and carboxyl groups of polysaccharide **3** to insert an amide bond. To a solution of saccharide 3 mg/ml in MES 30mM pH 6, a mixture of EDAC (3 eq) and sulfo-NHS (3 eq) was added and the reaction was carried out at r.t. under gently stirring; after 15 minutes Ova peptide (3 eq.) was added to the mixture reaction. The mixture was kept at r.t. overnight under gently stirring. The conjugates **16** was purified by ultrafiltration with Vivaspin system (Sartorius) using a 30kDa cut-off membrane.

### Analytical methods

### Sodium Dodecyl Sulphate-Polyacrylamide gel electrophoresis (SDS-Page)

SDS-Page was performed use pre-cast polyacrylamide gels (NuPAGE®Invitrogen) 4-12% Bis-Tris. The electrophoretic runs were performed in MES SDS running buffer (NuPAGE®Invitrogen) loading 2.5-5µg of peptide each sample, using the electrophoretic chamber with a voltage of 150V for about 40 minutes. Samples were prepared by adding 3 µl of NuPAGE® LDS sample buffer. After electrophoresis, the gel was washed in H₂O 3 times, then the gel was fixed for 30 minutes in a solution of 50% MeOH and 10% acetic acid. After fixing, the gel was washed for 3 times in water and finally stained with coomassie dye.

### Colorimetric analyses

Peptide content on the conjugates was determined by Micro BCA (Thermo)[Smith, P.K. et al. Anal. Biochem. 1985, 150, 76-85] or by Lowry (Thermo)[Lowry, O.H. et al. J. Biol. Chem. 1951, 193, 265-275] colorimetric assay, depending on the type of peptide used.

Sialic acid content has been determined by resorcinol colorimetric assay[Svennerholm, L. Biochim. Biophys. Acta 1957, 24(3), 604-611].

Amino groups have been determined by colorimetric assay [Habeeb, A.F. Anal Biochem 1966, 14, 328-38].

### Size Exclusion High Performance Liquid Chromatography (SEC-HPLC)

SEC-HPLC was performed on ULTIMATE™ 3000 HPLC system (Dionex part of Thermo Fisher Scientific) equipped with a PDA, RF2000 Fluorescence Detector and RI-101 Shodex Detector. Chromatography was performed in 0.02M NaPi 0.250M NaCl pH 7.2 on Superdex Peptide analytical column at flow rate of 0.05 ml/min, with 50 µl of injection volume loading 15-25 µg of sample in protein content. The resulting chromatographic data was processed using CHROMELEON™ 6.7 software.

### Reversed Phase High Performance Liquid Chromatography (RP-HPLC)

RP-HPLC was performed on ULTIMATE™ 3000 HPLC system (Dionex part of Thermo Fisher Scientific) equipped with a PDA, RF2000 Fluorescence Detector. Chromatography was performed with a gradient of acetonitrile in H₂O, from 5% up to 45% + 0.1%TFA on Jupiter C18 analytical column at flow rate of 0.1 ml/min, with 50 µl of injection volume. The resulting chromatographic data was processed using CHROMELEON™ 6.7 software.

### NMR analyses

¹H NMR experiments were recorded at 25 ± 0.1 °C on Bruker Avance III 400 MHz spectrometer, equipped with a high precision temperature controller, and using 5-mm broadband probe (Bruker). All the samples were dissolved in 0.75 mL of deuterium oxide (D₂O, 99.9% atom D, Aldrich) and inserted in 5-mm NMR tube (Wilmad). For data acquisition and processing, TopSpin version 2.6 software (Bruker) was used.

¹H NMR spectra were collected at 400 MHz over a 10 ppm spectral width, accumulating approximately 128 scans. The transmitter was set at the HDO frequency which was used as the reference signal (4.79 ppm). All the NMR spectra were obtained in quantitative manner using a total recycle time to ensure a full recovery of each signal (5 x Longitudinal Relaxation Time T1).

### Vaccines formulation and immunological studies

### Preparation of glycoconjugates formulations

Antigens formulations were prepared under sterile hoods using sterile instrumentation and solutions. All formulations were made using PBS pH7.2 as buffer where the vaccines were diluted to obtain the require dosage of saccharide per mice in a total volume of 200µl.

Aluminium hydroxide (AlumOH) was used as adjuvant and was prepared as 2x solution to be mixed 1:1 to the total volume of PBS. Each dose contained 0.36 mg of aluminium hydroxide.

The conjugates and conjugate vaccines were administered to groups of eight female Balb/c mice in 1 µg per dose in saccharide content.

Mice were immunized subcutaneously at day 1, 14 and 28. Bleedings were performed at day 0 (pre immune), day 28 (post 2) and day 42 (post 3). Control groups received PBS plus adjuvant. Animal experimental guidelines set forth by the Novartis Animal Care Department were followed in the conduct of all animal studies.

### Immunochemical evaluation of response

The antibody response induced by the glycoconjugates against the homologous polysaccharide and peptides were measured by ELISA assay. Ninety-six-well Maxisorp plates (Nunc, Thermo Fisher Scientific) plates were coated with the different meningococcal polysaccharides or peptides by adding 100 µl/well of a 5 µg/ml polysaccharide solution in PBS buffer at pH 8.2 or 100 µl/well of a 10 µg/ml peptide solution in PBS buffer at pH 7.2, followed by incubation overnight (o.n.) at 4°C. After coating, the plates were washed three times with 300 µl per well of TPBS (PBS with 0.05% Tween 20, pH 7.4) and blocked with 100 µL/well of 3% BSA (Sigma-Aldrich) for 1 h at 37°C. Subsequently, each incubation step was followed by a triple TPBS wash. Sera, prediluted 1:25, 1:100, 1:200 in TPBS, were transferred into coated-plates (200 µL) and then serially two-fold diluted followed by 2 h incubation at 37°C. After three washes with TPBS, 100 µl TPBS solutions of secondary antibody alkaline phosphates conjugates (anti mouse IgG 1:10000) was added and the plates incubated 1h at 37°C. After three more washes with TPBS, 100 µl/well of a 1mg/ml of p-NPP (Sigma) in a 0.5 M di-ethanolammine buffer pH 9.8 was added. After 30min of incubation at room temperature, plates were read at 405nm using a Biorad plate reader. Raw data acquisition has been performed by Microplate Manager Software (Biorad). Sera titers were expressed as the reciprocal of sera dilution corresponding to a cut-off OD = 1 or to a cut-off OD=0.2. Each immunization group was represented as the geometrical mean (GMT) of the single mouse titers. The statistical and graphical analysis was performed using GraphPad Prism software.

It will be understood that the invention has been described by way of example only and modifications may be made whilst remaining within the scope and spirit of the invention.

### REFERENCES (the contents of which are hereby incorporated in full)

[1] Vaccine Design: The Subunit and Adjuvant Approach (eds. Powell & Newman) Plenum Press 1995 (ISBN 0-306-44867-X).
[2] Vaccine Adjuvants: Preparation Methods and Research Protocols (Volume 42 of Methods in Molecular Medicine series). ISBN: 1-59259-083-7. Ed. O'Hagan.
[3] US patent 6355271.
[4] WO00/23105.
[5] WO90/14837.
[6] WO90/14837.
[7] Podda (2001) Vaccine 19: 2673-2680.
[8] Vaccine Design: The Subunit and Adjuvant Approach (eds. Powell & Newman) Plenum Press 1995 (ISBN 0-306-44867-X).
[9] Vaccine Adjuvants: Preparation Methods and Research Protocols (Volume 42 of Methods in Molecular Medicine series). ISBN: 1-59259-083-7. Ed. O'Hagan.
[10] Allison & Byars (1992) Res Immunol 143:519-25.
[11] Hariharan et al. (1995) Cancer Res 55:3486-9.
[12] US-2007/014805.
[13] Suli et al. (2004) Vaccine 22(25-26):3464-9.
[14] WO95/11700.
[15] US patent 6,080,725.
[16] WO2005/097181.
[17] WO2006/113373.
[18] Han et al. (2005) Impact of Vitamin E on Immune Function and Infectious Diseases in the Aged at Nutrition, Immune functions and Health EuroConference, Paris, 9-10 June 2005.
[19] US- 6630161.
[20] US 5,057,540.
[21] WO96/33739.
[22] WO96/11711.
[23] WO00/07621.
[24] Barr et al. (1998) Advanced Drug Delivery Reviews 32:247-271.
[25] Sjolanderet et al. (1998) Advanced Drug Delivery Reviews 32:321-338.
[26] Niikura et al. (2002) Virology 293:273-280.
[27] WO03/024481.
[28] Gluck et al. (2002) Vaccine 20:B10-B16.
[29] EP-A-0689454.
[30] Johnson et al. (1999) Bioorg Med Chem Lett 9:2273-2278.
[31] Evans et al. (2003) Expert Rev Vaccines 2:219-229.
[32] Meraldi et al. (2003) Vaccine 21:2485-2491.
[33] Pajak et al. (2003) Vaccine 21:836-842.
[34] Kandimalla et al. (2003) Nucleic Acids Research 31:2393-2400.
[35] WO02/26757.
[36] WO99/62923.
[37] Krieg (2003) Nature Medicine 9:831-835.
[38] US 6,429,199.
[39] Kandimalla et al. (2003) Biochemical Society Transactions 31 (part 3):654-658.
[40] Blackwell et al. (2003) J Immunol 170:4061-4068.
[41] WO01/95935.
[42] Kandimalla et al. (2003) BBRC 306:948-953.
[43] WO03/035836.
[44] WO01/22972.
[45] Schellack et al. (2006) Vaccine 24:5461-72.
[46] WO95/17211.
[47] WO98/42375.
[48] Beignon et al. (2002) Infect Immun 70:3012-3019.
[49] Pine et al. (2002) J Control Release 85:263-270.
[50] Tebbey et al. (2000) Vaccine 18:2723-34.
[51] Domenighini et al. (1995) Mol Microbiol 15:1165-1167*.*
[52] WO99/40936.
[53] WO99/44636.
[54] Singh et al] (2001) J Cont Release 70:267-276.
[55] WO99/27960.
[56] US 6,090,406.
[57] EP-A-0626169.
[58] WO99/52549.
[59] WO01/21207.
[60] WO01/21152.
[61] Andrianov et al. (1998) Biomaterials 19:109-115.
[62] Payne et al. (1998) Adv Drug Delivery Review 31:185-196.
[63] US 4,680,338.
[64] US 4,988,815.
[65] WO92/15582.
[66] Stanley (2002) Clin Exp Dermatol 27:571-577.
[67] Jones (2003) Curr Opin Investig Drugs 4:214-218.
[68] WO03/011223.
[69] Johnson et al. (1999) Bioorg Med Chem Lett 9:2273-2278.
[70] Evans et al. (2003) Expert Rev Vaccines 2:219-229.
[71] WO2004/060308.
[72] WO2004/064759.
[73] US 6,924,271.
[74] US 5,658,731.
[75] US patent 5,011,828.
[76] WO2004/87153.
[77] US 6,605,617.
[78] WO02/18383.
[79] WO2004/018455.
[80] WO03/082272.
[81] Wong et al. (2003) J Clin Pharmacol 43(7):735-42.
[82] US2005/0215517.
[83] Dyakonova et al. (2004) Int Immunopharmacol 4(13):1615-23.
[84] FR-2859633.
[85] Signorelli & Hadden (2003) Int Immunopharmacol 3(8):1177-86.
[86] WO2004/064715.
[87] De Libero et al, Nature Reviews Immunology, 2005, 5: 485-496
[88] WO03/105769
[89] Cooper (1995) Pharm Biotechnol 6:559-80.
[90] WO99/11241.
[91] WO94/00153.
[92] WO98/57659.
[93] European patent applications 0835318, 0735898 and 0761231.
[94] Durant et al. (2007) Infect Immun 75:1916-25.
[95] WO02/081653.
[96] Gennaro (2000) Remington: The Science and Practice of Pharmacy. 20th edition, ISBN: 0683306472.
[97] PCR (Introduction to Biotechniques Series), 2nd ed. (Newton & Graham eds., 1997, Springer Verlag)
[98] Current Protocols in Molecular Biology (F.M. Ausubel et al., eds., 1987) Supplement 30
[99] Smith & Waterman (1981) Adv. Appl. Math. 2: 482-489.

## Claims

1. A composition comprising a nonlinear saccharide conjugate that comprises a saccharide selected from a polysaccharide and an oligosaccharide, wherein the saccharide linked to at least two carrier peptides, wherein:
the at least two carrier peptides comprise at least one T-cell epitope and have no conformational B-cell epitopes,
at least one of the at least two peptides is internally linked to the saccharide and
at least one of the at least two peptides comprise a PV1 (peptide derived from the VP1 protein of poliovirus type 1). T-cell epitope.

2. The composition of claim 1, wherein the at least two peptides comprise a linear B-cell epitope.

3. The composition of claim 1, wherein the at least two peptides do not comprise a linear B-cell epitope.

4. The composition of any one of claims 1-3, wherein the at least two peptides have the same amino acid sequence.

5. The composition of any one of claims 1-3, wherein the at least two peptides have different amino acid sequences.

6. The composition of any one of claims 1-5, wherein the at least two peptides are linked directly to the saccharide.

7. The composition of any one of claims 1-6, wherein the at least two peptides are linked to the saccharide via a linker.

8. The composition of claim 7, wherein the linkers for the at least two peptides are the same.

9. The composition of claim 7, wherein the linkers for the at least two peptides are different.

10. The composition of any one of claims 7-9, wherein the linker is linear.

11. The composition of any one of claims 7-10, wherein the linker is N-kappa-Maleimidoundecanoic acid hydrazide-TFA (KMUH) or N-β-Maleimidopropionic acid hydrazide-TFA (BMPH).

12. The composition of any one of claims 7-11, wherein the the saccharide is not linked to a carrier protein.

13. The composition of any one of claims 1-12, wherein the saccharide is linked to at least one peptide per five to thirty-five saccharides, at least one peptide per five to twenty-five saccharides, at least one peptide per five to twenty saccharides, or at least one peptide per seven to fifteen saccharides.

14. The composition of any one of claims 1-13, wherein the saccharide is linked to at least three peptides, at least four peptides, at least five peptides, at least six peptides, at least seven peptides, at least eight peptides, at least nine peptides, or at least ten peptides.

15. The composition of any one of claims 1-14, wherein the saccharide is a capsular saccharide.

16. The composition of any one of claims 1-15, wherein the capsular saccharide is from N. meningitidis, S. pneumoniae, S. pyogenes, S. agalactiae, H. influenzae, P. aeruginosa, S. aureus, E. faecalis, E. faecium, Y. enteracolitica, V. cholerae or S. typhi.

17. The composition of any one of claims 1-14, wherein the saccharide is a glucan.

18. The composition of claim 17, wherein the glucan is from C. albicans, Coccidioides immitis, Trichophyton verrucosum, Blastomyces dermatidis, Cryptococcus neoformans, Histoplasma capsulatum, Saccharomyces cerevisiae, Paracoccidioides brasiliensis, or Pythiumn insidiosum.

19. The composition of any one of claims 1-18, wherein the saccharide comprises at least ten saccharides, at least fifteen saccharides, at least twenty saccharides, at least twenty-five saccharides, at least thirty saccharides, at least thirty-five saccharides, or at least forty saccharides.

20. The composition of any one of claims 1-19, further comprising a pharmaceutically acceptable carrier.

21. The composition of any one of claims 1-20, further comprising an adjuvant.

22. The composition of any one of claims 1-21, further comprising an additional component selected from: a Neisseria meningitidis antigen, a Streptococcus pneumoniae antigen, a Streptococcus pyogenes antigen, a Moraxella catarrhalis antigen, a Bordetella pertussis antigen, a Staphylococcus aureus antigen, a Staphylococcus epidermis antigen, a Clostridium tetani antigen, a Cornynebacterium diphtheriae antigen, a Haemophilus influenzae type B (Hib) antigen, a Pseudomonas aeruginosa antigen, a Legionella pneumophila antigen, a Streptococcus agalactiae antigen, a Neiserria gonorrhoeae antigen, a Chlamydia trachomatis antigen, a Treponema pallidum antigen, a Haemophilus ducreyi antigen, an Enterococcus faecalis antigen, an Enterococcus faecium antigen, a Helicobacter pylori antigen, a Staphylococcus saprophyticus antigen, a Yersinia enterocolitica antigen, an E. coli antigen, a Bacillus anthracis antigen, a Yersinia pestis antigen, a Mycobacterium tuberculosis antigen, a Rickettsia antigen, a Listeria monocytogenes antigen, a Chlamydia pneumoniae antigen, a Vibrio cholerae antigen, a Salmonella typhi antigen, a Borrelia burgdorferi antigen, a Porphyromonas gingivalis antigen, a Shigella antigen and a Klebsiella antigen.

23. The composition of any one of claims 1-22 for use in a method of inducing an immune response in a subject, wherein optionally:
(i) the subject is human; and/or
(ii) the immune response recognizes the polysaccharide, which immune response is optionally more T-cell dependent that an immune response induced by the polysaccharide unlinked to carrier proteins or other T-cell epitopes.

24. The composition of any one of claims 1-23 for use in a method of inducing an enhanced immune response to the saccharide in a mammalian subject.

## Patentansprüche

1. Zusammensetzung, umfassend ein nicht-lineares Saccharid-Konjugat, welches ein Saccharid, ausgewählt aus einem Polysaccharid und einem Oligosaccharid, umfasst, wobei das Saccharid an wenigstens zwei Trägerpeptide gebunden ist, wobei:
die wenigstens zwei Trägerpeptide wenigstens ein T-Zell-Epitop umfassen und keine konformativen B-Zell-Epitope aufweisen,
wenigstens eines der wenigstens zwei Peptide intern an das Saccharid gebunden ist und
wenigstens eines der wenigstens zwei Peptide ein PV1-(vom VP1-Protein von Poliovirus Typ 1 abgeleitetes Peptid) T-Zell-Epitop umfasst.

2. Zusammensetzung gemäß Anspruch 1, wobei die wenigstens zwei Peptide ein lineares B-Zell-Epitop umfassen.

3. Zusammensetzung gemäß Anspruch 1, wobei die wenigstens zwei Peptide kein lineares B-Zell-Epitop umfassen.

4. Zusammensetzung gemäß einem der Ansprüche 1-3, wobei die wenigstens zwei Peptide die gleiche Aminosäuresequenz aufweisen.

5. Zusammensetzung gemäß einem der Ansprüche 1-3, wobei die wenigstens zwei Peptide unterschiedliche Aminosäuresequenzen aufweisen.

6. Zusammensetzung gemäß einem der Ansprüche 1-5, wobei die wenigstens zwei Peptide direkt an das Saccharid gebunden sind.

7. Zusammensetzung gemäß einem der Ansprüche 1-6, wobei die wenigstens zwei Peptide über einen Linker an das Saccharid gebunden sind.

8. Zusammensetzung gemäß Anspruch 7, wobei die Linker für die wenigstens zwei Peptide gleich sind.

9. Zusammensetzung gemäß Anspruch 7, wobei die Linker für die wenigstens zwei Peptide unterschiedlich sind.

10. Zusammensetzung gemäß einem der Ansprüche 7-9, wobei der Linker linear ist.

11. Zusammensetzung gemäß einem der Ansprüche 7-10, wobei der Linker N-kappa-Maleimidoundecansäure-Hydrazid-TFA (KMUH) oder N-β-Maleimidopropionsäure-Hydrazid-TFA (BMPH) ist.

12. Zusammensetzung gemäß einem der Ansprüche 7-11, wobei das Saccharid nicht an ein Trägerprotein gebunden ist.

13. Zusammensetzung gemäß einem der Ansprüche 1-12, wobei das Saccharid an wenigstens ein Peptid pro fünf bis fünfunddreißig Saccharide, wenigstens ein Peptid pro fünf bis fünfundzwanzig Saccharide, wenigstens ein Peptid pro fünf bis zwanzig Saccharide oder wenigstens ein Peptid pro sieben bis fünfzehn Saccharide gebunden ist.

14. Zusammensetzung gemäß einem der Ansprüche 1-13, wobei das Saccharid an wenigstens drei Peptide, wenigstens vier Peptide, wenigstens fünf Peptide, wenigstens sechs Peptide, wenigstens sieben Peptide, wenigstens acht Peptide, wenigstens neun Peptide oder wenigstens zehn Peptide gebunden ist.

15. Zusammensetzung gemäß einem der Ansprüche 1-14, wobei das Saccharid ein Kapsel-Saccharid ist.

16. Zusammensetzung gemäß einem der Ansprüche 1-15, wobei das Kapsel-Saccharid aus N. meningitidis, S. pneumoniae, S. pyogenes, S. agalactiae, H. influenzae, P. aeruginosa, S. aureus, E. faecalis, E. faecium, Y. enteracolitica, V. cholerae oder S. typhi stammt.

17. Zusammensetzung gemäß einem der Ansprüche 1-14, wobei das Saccharid ein Glucan ist.

18. Zusammensetzung gemäß Anspruch 17, wobei das Glucan aus C. albicans, Coccidioides immitis, Trichophyton verrucosum, Blastomyces dermatidis, Cryptococcus neoformans, Histoplasma capsulatum, Saccharomyces cerevisiae, Paracoccidioides brasiliensis oder Pythiumn insidiosum stammt.

19. Zusammensetzung gemäß einem der Ansprüche 1-18, wobei das Saccharid wenigstens zehn Saccharide, wenigstens fünfzehn Saccharide, wenigstens zwanzig Saccharide, wenigstens fünfundzwanzig Saccharide, wenigstens dreißig Saccharide, wenigstens fünfunddreißig Saccharide oder wenigstens vierzig Saccharide umfasst.

20. Zusammensetzung gemäß einem der Ansprüche 1-19, weiterhin umfassend einen pharmazeutisch geeigneten Träger.

21. Zusammensetzung gemäß einem der Ansprüche 1-20, weiterhin umfassend ein Adjuvans.

22. Zusammensetzung gemäß einem der Ansprüche 1-21, weiterhin umfassend eine zusätzliche Komponente ausgewählt aus: einem Neisseria meningitidis-Antigen, einem Streptococcus pneumoniae-Antigen, einem Streptococcus pyogenes-Antigen, einem Moraxella catarrhalis-Antigen, einem Bordetella pertussis-Antigen, einem Staphylococcus aureus-Antigen, einem Staphylococcus epidermis-Antigen, einem Clostridium tetani-Antigen, einem Cornynebacterium diphtheriae-Antigen, einem Haemophilus influenzae type B- (Hib-) Antigen, einem Pseudomonas aeruginosa-Antigen, einem Legionella pneumophila-Antigen, einem Streptococcus agalactiae-Antigen, einem Neiserria gonorrhoeae-Antigen, einem Chlamydia trachomatis-Antigen, einem Treponema pallidum-Antigen, einem Haemophilus ducreyi-Antigen, einem Enterococcus faecalis-Antigen, einem Enterococcus faecium-Antigen, einem Helicobacter pylori-Antigen, einem Staphylococcus saprophyticus-Antigen, einem Yersinia enterocolitica-Antigen, einem E. coli-Antigen, einem Bacillus anthracis-Antigen, einem Yersinia pestis-Antigen, einem Mycobacterium tuberculosis-Antigen, einem Rickettsien-Antigen, einem Listeria monocytogenes-Antigen, einem Chlamydia pneumoniae-Antigen, einem Vibrio cholerae-Antigen, einem Salmonella typhi-Antigen, einem Borrelia burgdorferi-Antigen, einem Porphyromonas gingivalis-Antigen, einem Shigella-Antigen und einem Klebsiella-Antigen.

23. Zusammensetzung gemäß einem der Ansprüche 1-22 zur Verwendung in einem Verfahren zum Induzieren einer Immunantwort in einem Subjekt, wobei optional:
(i) das Subjekt ein Mensch ist; und/oder
(ii) die Immunantwort das Polysaccharid erkennt, welche Immunantwort optional stärker T-Zell-abhängig ist als eine Immunantwort, induziert durch das Polysaccharid, welches nicht an Trägerproteine oder andere T-Zell-Epitope gebunden ist.

24. Zusammensetzung gemäß einem der Ansprüche 1-23 zur Verwendung in einem Verfahren zum Induzieren einer verbesserten Immunantwort gegen das Saccharid in einem Säugetier.

## Revendications

1. Composition comprenant un conjugué saccharidique non linéaire qui comprend un saccharide choisi parmi un polysaccharide et un oligosaccharide, dans laquelle le saccharide s'est lié à au moins deux peptides de support, dans laquelle :
les au moins deux peptides de support comprennent au moins un épitope de lymphocyte T et n'ont aucun épitope conformationnel de lymphocyte B,
au moins l'un des au moins deux peptides est lié en interne au saccharide et
au moins l'un des au moins deux peptides comprend l'épitope de lymphocyte T PV1 (peptide dérivé de la protéine VP1 de poliovirus de type 1).

2. Composition selon la revendication 1, dans laquelle les au moins deux peptides comprennent un épitope de lymphocyte B linéaire.

3. Composition selon la revendication 1, dans laquelle les au moins deux peptides ne comprennent pas un épitope de lymphocyte B linéaire.

4. Composition selon l'une quelconque des revendications 1 à 3, dans laquelle les au moins deux peptides ont la même séquence d'acides aminés.

5. Composition selon l'une quelconque des revendications 1 à 3, dans laquelle les au moins deux peptides ont des séquences d'acides aminés différentes.

6. Composition selon l'une quelconque des revendications 1 à 5, dans laquelle les au moins deux peptides sont liés directement au saccharide.

7. Composition selon l'une quelconque des revendications 1 à 6, dans laquelle les au moins deux peptides sont liés au saccharide par l'intermédiaire d'un lieur.

8. Composition selon la revendication 7, dans laquelle les lieurs pour les au moins deux peptides sont identiques.

9. Composition selon la revendication 7, dans laquelle les lieurs pour les au moins deux peptides sont différents.

10. Composition selon l'une quelconque des revendications 7 à 9, dans laquelle le lieur est linéaire.

11. Composition selon l'une quelconque des revendications 7 à 10, dans laquelle le lieur est l'hydrazide de l'acide N-kappa-maléimido-undécanoïque-TFA (KMUH) ou l'hydrazide de l'acide N-β-maléimido-propionique-TFA (BMPH).

12. Composition selon l'une quelconque des revendications 7 à 11, dans laquelle le saccharide n'est pas lié à une protéine de support.

13. Composition selon l'une quelconque des revendications 1 à 12, dans laquelle le saccharide est lié à au moins un peptide pour cinq à trente-cinq saccharides, au moins un peptide pour cinq à vingt-cinq saccharides, au moins un peptide pour cinq à vingt saccharides, ou au moins un peptide pour sept à quinze saccharides.

14. Composition selon l'une quelconque des revendications 1 à 13, dans laquelle le saccharide est lié à au moins trois peptides, au moins quatre peptides, au moins cinq peptides, au moins six peptides, au moins sept peptides, au moins huit peptides, au moins neuf peptides, ou au moins dix peptides.

15. Composition selon l'une quelconque des revendications 1 à 14, dans laquelle le saccharide est un saccharide capsulaire.

16. Composition selon l'une quelconque des revendications 1 à 15, dans laquelle le saccharide capsulaire provient de N. meningitidis, S. pneumoniae, S. pyogenes, S. agalactiae, H. influenzae, P. aeruginosa, S. aureus, E. faecalis, E. faecium, Y. enteracolitica, V. cholerae ou S. typhi.

17. Composition selon l'une quelconque des revendications 1 à 14, dans laquelle le saccharide est un glucane.

18. Composition selon la revendication 17, dans laquelle le glucane provient de C. albicans, Coccidioides immitis, Trichophyton verrucosum, Blastomyces dermatidis, Cryptococcus neoformans, Histoplasma capsulatum, Saccharomyces cerevisiae, Paracoccidioides brasiliensis, ou Pythiumn insidiosum.

19. Composition selon l'une quelconque des revendications 1 à 18, dans laquelle le saccharide comprend au moins dix saccharides, au moins quinze saccharides, au moins vingt saccharides, au moins vingt-cinq saccharides, au moins trente saccharides, au moins trente-cinq saccharides, ou au moins quarante saccharides.

20. Composition selon l'une quelconque des revendications 1 à 19, comprenant en outre un support pharmaceutiquement acceptable.

21. Composition selon l'une quelconque des revendications 1 à 20, comprenant en outre un adjuvant.

22. Composition selon l'une quelconque des revendications 1 à 21, comprenant en outre un composant supplémentaire choisi parmi : un antigène de Neisseria meningitidis, un antigène de Streptococcus pneumoniae, un antigène de Streptococcus pyogenes, un antigène de Moraxella catarrhalis, un antigène de Bordetella pertussis, un antigène de Staphylococcus aureus, un antigène de Staphylococcus epidermis, un antigène de Clostridium tetani, un antigène de Cornynebacterium diphtheriae, un antigène de Haemophilus influenzae de type B (Hib), un antigène de Pseudomonas aeruginosa, un antigène de Legionella pneumophila, un antigène de Streptococcus agalactiae, un antigène de Neiserria gonorrhoeae, un antigène de Chlamydia trachomatis, un antigène de Treponema pallidum, un antigène de Haemophilus ducreyi, un antigène d'Enterococcus faecalis, un antigène d'Enterococcus faecium, un antigène d'Helicobacter pylori, un antigène de Staphylococcus saprophyticus, un antigène de Yersinia enterocolitica, un antigène d'E. coli, un antigène de Bacillus anthracis, un antigène de Yersinia pestis, un antigène de Mycobacterium tuberculosis, un antigène de Rickettsia, un antigène de Listeria monocytogenes, un antigène de Chlamydia pneumoniae, un antigène de Vibrio cholerae, un antigène de Salmonella typhi, un antigène de Borrelia burgdorferi, un antigène de Porphyromonas gingivalis, un antigène de Shigella et un antigène de Klebsiella.

23. Composition selon l'une quelconque des revendications 1 à 22 pour une utilisation dans un procédé d'induction d'une réponse immunitaire chez un sujet, où éventuellement :
(i) le sujet est humain ; et/ou
(ii) la réponse immunitaire reconnaît le polysaccharide, laquelle réponse immunitaire est éventuellement plus dépendante des lymphocytes T qu'une réponse immunitaire induite par le polysaccharide non lié à des protéines de support ou d'autres épitopes de lymphocytes T.

24. Composition selon l'une quelconque des revendications 1 à 23 pour une utilisation dans un procédé d'induction d'une réponse immunitaire amplifiée au saccharide chez un sujet mammifère.
